# EUROPEAN PATENT APPLICATION

(11) **EP 3 904 354 A1**
(43) Date of publication of application: **03.11.2021**
(21) Application number: 20305421.8
(22) Date of filing: 30.04.2020
(51) Int. Cl.: C07D 417/06, A61K 31/428, A61P 25/00

(54) **NEW IMIDAZOLONE DERIVATIVES AS INHIBITORS OF PROTEIN KINASES IN PARTICULAR DYRK1A, CLK1 AND/OR CLK4**

(71) Applicant: Perha Pharmaceuticals, 29680 Roscoff (FR)
(72) Inventor: The designation of the inventor has not yet been filed
(74) Representative: Nony

(57) **Abstract**

The present invention relates to a compound of formula (I) 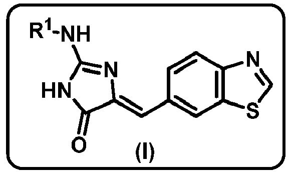 wherein R¹ represents a (C₁-C₆)alkyl group, a spiro(C₅-C₁₁)bicyclic ring, a fused phenyl group, a substituted phenyl group, a R'-L- group, wherein L is either a single bond or a (Ci-C3)alkanediyl group, and R' represents a (C₃-C₈)cycloalkyl group, a bridged (C6-C₁₀)cycloalkyl group, a (C₃-C₈)heterocycloalkyl group, or a (C₃-C₈)heteroaryl group, or a R'-L- group wherein L is a (Ci-C3)alkanediyl group, and R' is a an optionally substituted phenyl group or any of its pharmaceutically acceptable salt.

The present invention further relates to a composition comprising a compound of formula (I) and a process for manufacturing said compound as well as its synthesis intermediates.

It also relates to said compound for use as a medicament, in particular in the treatment and/or prevention of cognitive deficits associated with Down syndrome; Alzheimer's disease; dementia; tauopathies; Parkinson's disease; CDKL5 Deficiency Disorder; Phelan-McDermid syndrome; autism; diabetes; regulation of folate and methionine metabolism; osteoarthritis; Duchenne muscular dystrophy; several cancers; neuroinflammation, anemia and infections and for regulating body temperature.

## Description

### FIELD OF THE INVENTION

The present invention relates to Leucettinibs, a class of new compounds useful as a medicament. Said new compounds are in particular useful as kinase inhibitors, and even more particularly as inhibitors of DYRK1A and/or CLK1. They are efficient for treating and/or preventing cognitive deficits associated with Down syndrome; Alzheimer's disease and related diseases; dementia; tauopathies; Parkinson's disease; other neurodegenerative diseases; CDKL5 Deficiency Disorder; diabetes; regulation of folate and methionine metabolism; osteoarthritis; Duchenne muscular dystrophy; several cancers and viral infections and for regulating body temperature.

Some of said compounds are further inhibitors of other kinases and namely other DYRKs (DYR1B, 2, 3, 4) and the closely related further cdc2-like kinases (CLKs) (CLK 2, 3, 4). Said compounds may then further be efficient for treating and/or preventing of Phelan-McDermid syndrome; autism; further viral infections, further cancers, neuroinflammation, anemia and infections caused by unicellular parasites.

It further relates to the pharmaceutical compositions containing said new compounds and to the chemical synthesis processes for obtaining them.

### BACKGROUND

The DYRK and CLK kinase families belong to the CMGC group of kinases which also includes the mitogen-activated protein kinases (MAPK), cyclin-dependent kinases (CDKs) and glycogen synthase kinase-3 (GSK-3). They phosphorylate many substrates involved in signaling pathways. DYRKs and CLKs play key roles in mRNA splicing, chromatin transcription, DNA damage repair, cell survival, cell cycle, differentiation, homocysteine/methionine/folate regulation, endocytosis, neuronal development and functions, synaptic plasticity.

### DYRK1A and Down syndrome (DS)

The gene encoding **DYRK1A** is located on chromosome 21, in particular in the "**Down syndrome critical region**" (DSCR), the triploidy of which is responsible for most DS-associated deficiencies. There is considerable genetic and pharmacological evidence showing that the mere 1.5-fold overexpression of **DYRK1A** is responsible for most cognitive deficits observed in DS patients (Rueda N et al., 2020. Translational validity and implications of pharmacotherapies in preclinical models of Down syndrome. Prog Brain Res 251, 245). Pharmacological or genetical normalization of DYRK1A levels restores cognitive functions.

### DYRK1A and Alzheimer's disease (AD), Tauopathies

There is mounting evidence for a role of **DYRK1A** in the onset of AD. **DYRK1A** phosphorylates key substrates involved in AD and dementia: Tau, septin 4, amyloid precursor protein (APP), presenilin 1, neprilysin, Munc18-1, α-synuclein, RCAN1, β-Tubulin. By modulating alternative splicing of exon 10, **DYRK1A** favors the production of the 3R-Tau splice isoform (characteristic for DS/AD/tauopathy) over the normal 4R-Tau isoform. **DYRK1A** inhibition promotes autophagy which could counterbalance the autophagy deficit seen in AD.

### DYRK1A and Parkinson's disease (PD) and Pick disease

GWAS studies have revealed that **DYRK1A** is a risk factor for PD (Nalls MA et al., 2019. Identification of novel risk loci, causal insights, and heritable risk for Parkinson's disease: a meta-analysis of genome-wide association studies. Lancet Neurol 18, 1091). DYRK1A phosphorylates key factors for PD such as Parkin, septin 4, α-synuclein. Upregulation of micro-RNA specific for PD target DYRK1A expression (Chiu CC et al., 2019. Upregulated expression of microRNA-204-5p leads to the death of dopaminergic cells by targeting DYRK1A-mediated apoptotic signaling cascade. Front Cell Neurosci 13, 399). There is further evidence that **DYRK1A** expression is increased in PD. DYRK1A is overexpressed in Pick disease.

### DYRK1A and other diseases (viral infections, diabetes, cancers)

**DYRK1A** and **DYRK1B** are utilized during HCMV placental replication. Inhibition of **DYRKs** prevent replication of various viruses including Herpes virus, cytomegalovirus and HIV-1. **DYRK1A** inhibitors stimulate the proliferation of pancreatic, insulin-producing β-cells, a promising approach to diabetes (Ackeifi C et al., 2020. Pharmacologic and genetic approaches define human pancreatic β cell mitogenic targets of DYRK1A inhibitors. JCI Insight 5, e132594). There is abundant literature linking **DYRK1A** with cancer. The most prominent examples are megakaryoblastic leukemia, pancreatic cancer and brain tumor.

Accordingly, abnormalities in **DYRK1A** dosage are associated with cognitive disorders observed in Down syndrome, and Alzheimer's disease. DYRK1A is a risk factor for Parkinson's disease. Inhibition of DYRK1A additionally triggers the proliferation of pancreatic, insulin-producing β-cells). DYRK1A inhibitors may thus find applications in preventing and/or treating DS, AD, and other Tauopathies, dementia, PD, Niemann-Pick Type C Disease, CDKL5 deficiency disorder, diabetes, viral infections, several cancers (leukemia, pancreatic cancer, glioblastoma), osteoarthritis, infections caused by unicellular parasites and for regulating body temperature.

### Other DYRKs and human disease

**DYRK1B** is involved in the replication of various viruses including hepatitis C virus, Chikungunya virus, Dengue virus and SARS coronavirus, cytomegalovirus and human papillomavirus. Like **DYRK1A**, **DYRK1B** inhibition leads to the proliferation of pancreatic, insulin-producing β-cells. **DYRK1B** is involved in neuroinflammation. Targeting **DYRK1B** provides a new rationale for treatment of various cancers such as liposarcoma or breast cancers.

**DYRK2**, in association with GSK-3β, regulates neuronal morphogenesis. **DYRK2** is involved in various ways in cancer development.

**DYRK3** promotes hepatocellular carcinoma. **DYRK3** couples stress granule condensation/dissolution to mTORC1 signaling. **DYRK3** regulates phase transition of membraneless organelles in mitosis. **DYRK3** and **DYRK4** are involved in the regulation of cytoskeletal organization and process outgrowth in neurons.

**DYRK1A** decreases axon growth, **DYRK3** and **DYRK4** increase dendritic branching and **DYRK2** decreases both axon and dendrite growth and branching.

### CLKs and human disease

Note that **CLK** is a confusing abbreviation as it has the following meanings: (**a**) monooxygenase CLK-1 (human homologue COQ7); (**b**) Collectin-K1 (CL-K1, or CL-11), a multifunctional Ca(2+)-dependent lectin; (**c**) MAPK gene of the maize pathogen Curvularia lunata, Clk1; (**d**) mitochondrial membrane-bound enzyme Clock-1 (CLK-1); (**e**) Colletotrichum lindemuthianum kinase 1 (clk1).

**CLKs** play essential functions in alternative splicing. **CLKs** act as a body-temperature sensor which globally controls alternative splicing and gene expression. The activity of CLKs is indeed highly responsive to physiological temperature changes, which is conferred by structural rearrangements within the kinase activation segment (Haltenhof T et al., 2020. A conserved kinase-based body-temperature sensor globally controls alternative splicing and gene expression. Mol Cell 78, 57).

### CLK1 and human disease

**CLK1** triggers periodic alternative splicing during the cell division cycle. **CLK1** regulates influenza A virus mRNA splicing and its inhibition prevents viral replication. **CLK1** and **CLK2** also regulates HIV-1 gene expression. **CLK1** is an autophagy inducer. **CLK1** inhibition may prevent chemoresistance in glioma and **CLK1** inhibition by TG693 allows the skipping of mutated exon 31 of the dystrophin gene in Duchenne Muscular Dystrophy.

### Other CLKs and human disease

Inhibition of **CLK2** has been proposed as a way to improve neuronal functions and combat intellectual disability and autism in Phelan-McDermid syndrome (PMDS). Dual inhibition of **CLK2** and DYRK1A by Lorecivivint is a potential disease-modifying approach for knee osteoarthritis. **CLK2** inhibition compromises MYC-driven breast tumors, triple negative breast cancer and glioblastoma. Inhibition of **CLK2** improves autistic features in Phelan-McDermid syndrome (PMDS). Alternative splicing of Tau exon 10 is regulated by **CLK2** and **other CLKs**, leading to changes in the 3R/4R isoforms ratio and neurodegeneration in sporadic AD. Inhibition of **CLK2, CLK3, CLK4** blocks HIV-1 production.

**CLK3** contributes to hepatocellular carcinoma and prostate cancer.

The following Table 1 gathers the implication of the DYRKs and CLKs kinase target in associated diseases.

**Table 1**

| **Kinase target** | **Disease** |
|---|---|
| DYRK1A | Down syndrome (DS) |
| DYRK1A | Alzheimer's disease (AD) and other Tauopathies |
| DYRK1A | Parkinson's disease |
| DYRK1A | Pick disease |
| DYRK1A | CDKL5 Deficiency Disorder |
| DYRK1A | Diabetes |
| DYRK1A | Regulation of folate and methionine metabolism |
| DYRK1A | Glioblastoma |
| DYRK1A | Head and neck squamous cell carcinoma |
| DYRK1A | Pancreatic ductal adenocarcinoma |
| DYRK1A | Megakaryoblastic leukemia |
| DYRK1A | Knee osteoarthritis |
| DYRK1A | Human immunodeficiency virus type 1 (HIV-1) |
| DYRK1A, DYRK1B | Human cytomegalovirus (HCMV) |
| DYRK1B | Hepatitis C virus, Chikungunya virus, Dengue virus and Severe acute respiratory syndrome coronavirus, Cytomegalovirus, Human papillomavirus |
| DYRK1B | Diabetes |
| DYRK1B | Neuroinflammation |
| DYRK1B | Liposarcoma, Breast cancer, Hedgehog/GLI-dependent cancer |
| DYRK2 | Triple-negative breast cancer (TNBC) and multiple myeloma (MM) |
| DYRK2 | Glioblastoma |
| DYRK3 | Hepatocellular carcinoma |
| DYRK3 | Influenza virus replication |
| DYRK3 | Anemia |
| DYRKs | Glioblastoma |
| DYRKs | Herpes simplex virus, cytomegalovirus, varicella-zoster virus |
| LinDYRK1 | Leishmaniasis |
| TbDYRK | *Trypanosoma brucei* |
| | |
| CLK1 | Glioblastoma |
| CLK1 | Duchenne muscular dystrophy |
| CLK1 | Influenza A |
| CLK2 | HIV-1 |
| CLK1/CLK2 | Triple-negative breast cancer |
| CLK2 | Autism, Phelan-McDermid syndrome (PMDS) |
| CLK2 | Knee osteoarthritis |
| CLK2 | Breast cancer, Triple negative breast cancer, Glioblastoma |
| CLK2 | Alzheimer's disease (alternative splicing of Tau exon 10) |
| CLK3 | Hepatocellular carcinoma, Prostate cancer |
| CLKs | Body temperature |
| CLKs | Prostate cancer, Gastrointestinal cancer |
| PfCLKs | Malaria |
| | |
| DYRKs/CLKs | Glioblastoma |

### DYRKs and CLK inhibitors

Several DYRK1A inhibitors have been reported in recent years. Most DYRK1A inhibitors also inhibit DYRK1B, 2, 3, 4, as well as the closely related CLK1, 2, 3, 4, with several possible inhibition profiles.

Some imidazolone derivatives, afterwards named **Leucettines**, were disclosed in WO2009/050352 as kinase inhibitors and more particularly as inhibitors of the DYRK1A kinase.

There is still a need to identify new compounds for treating and/or preventing the diseases as recited above, and particularly through the inhibition, and in particular selective inhibition, of DYRK1A, other DYRKs and the related CLKs kinases.

### SUMMARY OF THE INVENTION

It has now been found that the compounds as defined in formula (I) hereinafter are useful in the treatment and/or prevention of cognitive deficits associated with Down syndrome; Alzheimer's disease and related diseases; dementia; tauopathies; Parkinson's disease; other neurodegenerative diseases; CDKL5 Deficiency Disorder; Phelan-McDermid syndrome; autism; diabetes; regulation of folate and methionine metabolism; osteoarthritis; several cancers; neuroinflammation, anemia and infections caused by unicellular parasites and viral infections and for regulating body temperature.

The present invention therefore relates to a compound of formula (I), as defined below.

The present invention further relates to a compound of formula (I) as defined below for use as a medicament.

The present invention further relates to a compound of formula (I) as defined below for use in the treatment and/or prevention of cognitive deficits associated with Down syndrome; Alzheimer's disease and related diseases; dementia; tauopathies; Parkinson's disease; other neurodegenerative diseases; CDKL5 Deficiency Disorder; Phelan-McDermid syndrome; autism; diabetes; regulation of folate and methionine metabolism; osteoarthritis; Duchenne muscular dystrophy; several cancers; neuroinflammation, anemia and infections caused by unicellular parasites and viral infections and for regulating body temperature.

The present invention further relates to a pharmaceutical compositon comprising it and to a process for manufacturing it.

The present invention at last relates to synthetic intermediates of formula (II) as defined below.

### DEFINITIONS

As used herein, the term "patient" refers to either an animal, such as a valuable animal for breeding, company or preservation purposes, or preferably a human or a human child, which is afflicted with, or has the potential to be afflicted with one or more diseases and conditions described herein.

In particular, as used in the present application, the term "patient" refers to a mammal such as a rodent, cat, dog, primate or human, preferably said subject is a human and also extends to birds.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "treating" or "treatment", as used herein, means preventing, reversing, alleviating, inhibiting the progress of, or preventing the disease and its cognitive, motor or metabolic changes resulting from high DYRK1A kinase and/or CLK1 expression and activity, and optionally associated with the abnormalities in other DYRKs (DYRK1B, 2, 3, 4) and the closely related further cdc2-like kinases (CLKs) (CLK 2, 3, 4) and more particularly in connection to the diseases as decribed herein after in the paragraph "PATHOLOGIES".

Therefore, the term "treating" or "treatment" encompasses within the framework of the present invention the improvement of medical conditions of patients suffering from the diseases as decribed herein after in the paragraph "PATHOLOGIES", related to high expression and activity of any of the DYRK1A and CLK1 kinases, and optionally associated with the abnormalities in other DYRKs (DYRK1B, 2, 3, 4) and the closely related further cdc2-like kinases (CLKs) (CLK 2, 3, 4).

As used herein, an "effective amount" refers to an amount of a compound of the present invention which is effective in preventing, reducing, eliminating, treating or controlling the symptoms of the herein-described diseases and conditions.

The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

The term "effective amount" includes "prophylaxis-effective amount" as well as "treatment-effective amount".

The term "preventing", as used herein, means reducing the risk of onset or slowing the occurrence of a given phenomenon, namely in the present invention, a disease resulting from abnormal DYRKs/CLKs kinase activity, in particular DYRK1A kinase activity.

As used herein, « *preventing* » also encompasses « *reducing the likelihood of occurrence* » or « *reducing the likelihood of reoccurrence* ».

The term "*prophylaxis-effective amount*" refers to a concentration of compound of this invention that is effective in inhibiting, preventing, decreasing the likelihood of anyone of the hereabove described diseases.

Likewise, the term "treatment-effective amount" refers to a concentration of compound that is effective in treating the hereabove described diseases, *e*.*g*. leads to a reduction or normalization DYRK1A and/or CLK1 kinase activity, and optionally additionally of DYRKs/CLKs kinase activity in general, following examination when administered after disease has occurred.

As used herein, the term "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

### DETAILED DESCRIPTION OF THE INVENTION

The inventors have surprisingly found that the compounds of formula (I) as disclosed herein after inhibit the DYRK1A, other DYRKs (DYRK1B, DYRK2, DYRK3, DYRK4) and CLKs (CLK1, CLK2, CLK3, CLK4). This assertion is based on data as illustrated in the following examples and more detailed herein after.

According to a first aspect, a subject-matter of the present invention relates to a compound of formula (I) 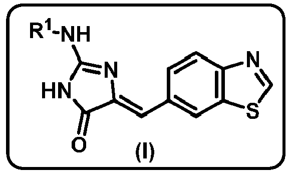 wherein R¹ represents:
(i). a (C₁-C₆)alkyl group substituted by one or two groups selected from a -COOR^{a} group, a hydroxy group, a halogen, a (C₁-C₃)alkoxy group and a benzyloxy group,
(ii). a spiro(C₅-C₁₁)bicyclic ring,
(iii). a fused phenyl group, selected from phenyl groups fused with a (C₅-C₆)cycloalkyl or (C₅-C₆)heterocycloalkyl, which (C₅-C₆)cycloalkyl and (C₅-C₆)heterocycloalkyl ring optionally comprise an insaturation and is optionally substituted by a (C₁-C₄) alkyl group, a hydroxy group, a halogen atom, a (C₁-C₃)alkoxy group or a -COR^{a} group,
(iv). a phenyl group, substituted by one or two groups selected from a (C₁-C₈)alkyl, a (C₁-C₃)fluoroalkyl, a fluoro(C₁-C₄)alkoxy group, a halogen atom, and a (C₄-C₇)heterocycloalkyl group, said (C₄-C₇)heterocycloalkyl group being itself optionally substituted by a (C₁-C₄) alkyl group, or
(v). a R'-L- group, wherein L is either a single bond or a (C₁-C₃)alkanediyl group, optionnally susbstituted by a group selected from a hydroxy group and a (C₁-C₃)alkoxy group, and
   R' represents:
   (v.1) a (C₃-C₈)cycloalkyl group, optionally substituted by one, two or three groups selected from a (C₁-C₄)alkyl group, a hydroxy group, a halogen atom and a (C₁-C₃) alkoxy group,
   (v.2) a bridged (C₆-C₁₀)cycloalkyl group, optionally substituted by one to three groups selected from a (C₁-C₄)alkyl group, a (C₁-C₄)alkoxy group, a halogen atom and a hydroxy group,
   (v.3) a (C₃-C₈)heterocycloalkyl group, optionally substituted by one or two groups selected from a -COOR^{a} group, a hydroxy group, a halogen atom, a (C₁-C₄)alkyl group and an oxo group, or
   (v.4) a (C₃-C₈)heteroaryl group, optionally substituted by one to three groups selected from a halogen atom, a (C₁-C₄)alkyl group, a (C₁-C₄)alkoxy group and a N-methylpiperazinyl group, or
(vi). a R'-L- group wherein L is a (C₁-C₃)alkanediyl group, optionally substituted by a group selected from a -NR^{b}R^{c} group, a (C₁-C₃)alkoxy group, a hydroxy group, a -COOR^{a} group and a halogen atom, and
   R' is a phenyl group, optionally substituted by one to three groups selected from the group consisting of (C₁-C₆)alkyl group, a fluoro(C₁-C₄)alkyl group and a fluoro(C₁-C₄)alkoxy group, a halogen atom and a hydroxy group,
R^{a} represents a (C₁-C₄)alkyl group or a hydrogen atom, and
R^{b} and R^{c} independently represent a (C₁-C₆)alkyl group or a hydrogen atom, or any of its pharmaceutically acceptable salt.

According to a particular embodiment, the present invention relates to a compound of formula (I) as defined herein above wherein R¹ represents:
(i). a (C₂-C₆)alkyl group substituted by one or two groups selected from a -COOR^{a} group, a hydroxy group, a halogen, a (C₁-C₃) alcoxy group and a benzyloxy group,
(ii). a spiro(C₇-C₉)bicyclic ring,
(iii). a fused phenyl group, selected from phenyl groups fused with a cyclopentyl or a heterocyclopentyl, which cyclopentyl and heterocyclopentyl group optionally comprise an insaturation and is optionally substituted by a (C₁-C₄) alkyl group, a hydroxy group, a halogen atom, a (C₁-C₃)alkoxy group or a -COR^{a} group
(iv). a phenyl group, substituted by one or two groups selected from (C₁-C₈)alkyl, a (C₁-C₃)fluoroalkyl, a fluoro(C₁-C₄)alkoxy group a halogen atom, and a (C₄-C₇)heterocycloalkyl group, said (C₄-C₇)heterocycloalkyl group being itself optionally substituted by a (C₁-C₄) alkyl group, or
(v). a R'-L- group wherein
   - L is either a single bond or a (C₁-C₃)alkanediyl group, optionnally susbstituted by a group selected from a hydroxy group and a (C₁-C₃)alkoxy group, and
   - R' represents:
      (v.1) a (C₃-C₈)cycloalkyl group, optionally substituted by one, two or three groups selected from a (C₁-C₄)alkyl group, a hydroxy group and a (C₁-C₃) alkoxy group,
      (v.2) a bridged (C₇-C₁₀)cycloalkyl group, optionally substituted by one to three groups selected from a (C₁-C₄)alkyl group, a (C₁-C₄)alkoxy group, a hydroxy group and a halogen atom,
      (v.3) a (C₄-C₇)heterocycloalkyl group, optionally substituted by one or two groups selected from a -COOR^{a} group, a hydroxy group, a halogen atom, a (C₁-C₄)alkyl group and a oxo group, or
      (v.4) a heteroaryl group, optionally substituted by one to three groups selected from a halogen atom, (C₁-C₄)alkyl group, a (C₁-C₄)alkoxy group, a N-methylpiperazinyl group,
(vi). a R'-L- group wherein
   - L is a (C₁-C₃)alkanediyl group, optionally substituted by a group selected from a -NR^{b}R^{c} group, a (C₁-C₃)alkoxy group, a hydroxy group, a -COOR^{a} group and a halogen atom, and
   - R' is a phenyl group, optionally substituted by one to three groups selected from the group consisting of (C₁-C₆)alkyl group, a fluoro(C₁-C₄)alkyl group and a fluoro(C₁-C₄)alkoxy group, a hydroxy group and a halogen atom,
R^{a} representing a (C₁-C₄)alkyl group or a hydrogen atom, and
R^{b} and R^{c} independently represent a (C₁-C₆)alkyl group or a hydrogen atom, or any of its pharmaceutically acceptable salt.

According to another particular embodiment, the present invention relates to a compound of formula (I) as defined herein above wherein R¹ represents:
(i). a (C₂-C₆)alkyl group substituted by one or two groups selected from a -COOCH3 group, a hydroxy group, a fluorine atom, a methoxy group and a benzyloxy group,
(ii). a spiro(C₇-C₈)bicyclic ring, in particular a spiro[3.3]heptyl, a spiro[2.5]octanyl or a 7-azaspiro[3.5]nonyl
(iii). a fused phenyl group, chosen from phenyl groups fused with a cyclopentyl or a heterocyclopentyl, which cyclopentyl and heterocyclopentyl group optionally comprise an insaturation and is optionally substituted by a methyl, a hydroxy group, a methoxy group and a -COCH₃ group
(iv). a phenyl group, substituted by one or two groups selected from a methyl, a hexyl, a trifluoromethyl, a difluoromethoxy group, a halogen atom, in particular a fluor atom, a morpholino group and a *N-*methylpiperazinyl group, or
(v). a R'-L- group wherein L is either a single bond or a (C₁-C₃)alkanediyl group, optionnally susbstituted by a group chosen from a hydroxy group and a (C₁-C₃)alkoxy group,
   and R' is selected from the group consisting of:
   (v.1). a (C₃-C₈)cycloalkyl group in particular a cyclopropyl, a cyclobutyl, a cyclopentyl, a cyclohexyl, a cycloheptyl or a cyclooctyl, optionally substituted by one, two or three groups selected from a methyl, an isopropyl, a hydroxy group and a methoxy group,
   (v.2). a bridged (C₇-C₁₀)cycloalkyl group, in particular an adamantyl or a bicyclo[3.1.1]heptyl, optionally substituted by one to three groups selected from methyl, methoxy and hydroxy group,
   (v.3). a (C₅-C₈)heterocycloalkyl group, in particular a tetrahydropyranyl, a piperidinyl, an oxetanyl, a tetrahydrofuranyl or an oxepanyl, optionally substituted by a group selected from a -COOR^{d} group, a hydroxy group, a methyl group, wherein R^{d} represents either an ethyl or an isopropyl group
   (v.4). a heteroaryl group, in particular a pyrimidinyl, a pyridinyl, a thiazolyl, a imidazolyl, a pyrazolyl, a thiadiazolyl, a pyridazinyl, a pyrazinyl, a furyl, optionally substituted by one to three groups selected from methyl group, a methoxy group and a N-methylpiperazinyl group, or
(vi). a R'-L- group wherein L is a (C₁-C₃)alkanediyl group, optionally substituted by a group selected from the group consisting of a -NR^{b}R^{c} group, a (C₁-C₃)alkoxy group, a hydroxy group, a -COOR^{a} group and a halogen atom, in particular a fluor atom, and
   R' is a phenyl group, optionally substituted by one or two groups selected from the group consisting of methyl group, a trifluoromethyl group and a trifluoromethoxy group,
R^{a} representing a (C₁-C₃)alkyl group, and
R^{b} and R^{c} are independently chosen from a methyl group pr a hydrogen atom, or any of its pharmaceutically acceptable salts.

According to another particular embodiment, the present invention relates to a compound of formula (I) as defined herein above wherein R¹ is a R'-L- group wherein L is selected from a group consisting of a -CH₂- group, a -CH(CH₃)- group, a -CH(CH₂OH)-CH₂- group, a -CH(CH₂OH)- group, a -CH(CH₂OCH₃)- group, a -CH(OH)-CH₂- group, a -CH₂-CH(CH₂OCH₃)- group, a -CH(OCH₃)-CH₂- group, a -CH₂-CH(COOCH₃)- group, a -CH(CH₂F)- group, a -CH(CH₂NH₂)- group, a -CH(CH₂NHCH₃)- group, a -CH(CH₂N(CH₃)₂)- group, a -CH₂-CH(CH₂OH)- group, a -CH(OCH₃)-CH₂- group, a -CH₂-CH(OCH₃)- group, a -CH₂-CH(OH)-CH₂- group, a -CH₂-CH(OCH₃)-CH₂ group, a -(CH₂)₃- group and a -(CH₂)₂- group, or any of its pharmaceutically acceptable salts.

According to another particular embodiment, the present invention relates to a compound of formula (I) as defined herein above wherein R¹ is a R'-L- group wherein:
(v.1). when R' is a (C₃-C₈)cycloalkyl group, L is selected from the group consisting of a -CH₂- group, a -CH(CH₃)- group, a -CH(CH₂OH)-CH₂- group, a -CH(CH₂OH)- group, a -CH(CH₂OCH₃)-group and a -CH(OH)-CH₂- group and a -CH(OCH₃)-CH₂- group,
(v.2). when R' is a bridged (C₇-C₁₀)cycloalkyl group, L is a -CH₂-group or a -CH(CH₃)- group,
(v.3). when R' is a (C₅-C₈)heterocycloalkyl group including spiro(C₃-C₈)heterocycloalkyls, L is a -CH₂- group,
(v.4). when R' is a phenyl, L is selected from the group consisting of a -CH₂- group, a -CH₂-CH(COOCH₃)- group, a -CH(CH₂F)- group, a -CH(CH₂NH₂)- group, a -CH(CH₂NHCH₃)- group, a -CH(CH₂N(CH₃)₂)-group, a -CH₂-CH(CH₂OH)- group, a -CH(CH₂OH)- group, a -CH(CH₂OCH₃)- group, a -CH(OH)-CH₂- group, a -CH₂-CH(CH₂OCH₃)-group, a -CH₂-CH(OH)-CH₂- group and a -CH₂-CH(OCH₃)-CH₂ group,
(v.5). when R' is a heteroaryl group, L is selected from the group comprising a -CH₂- group, a -(CH₂)₃- group and a -(CH₂)₂- group.

According to another particular embodiment, the present invention relates to a compound of formula (I) as defined herein above wherein R¹ represents:
- a (C₁-C₆)alkyl group substituted by one or two groups selected from a -COOR^{a} group, a hydroxy group, a fluorine atom, a (C₁-C₃)alkoxy group and a benzyloxy group,
- a spiro(C₅-C₁₁)bicyclic ring, or
- a R'-L- group, wherein
   ∘ L is either a single bond or a (C₁-C₃)alkanediyl group, optionnally susbstituted by a group chosen from a hydroxy group and a (C₁-C₃)alkoxy group, and
   ∘ R' is selected from the group consisting of:
      ▪ a (C₃-C₈)cycloalkyl group, optionally substituted by one, two or three groups selected from a halogen atom, a (C₁-C₄)alkyl group, a hydroxy group and a (C₁-C₃) alkoxy group, and
      ▪ a bridged (C₆-C₁₀)cycloalkyl group, optionally substituted by one to three groups selected from a (C₁-C₄)alkyl group, a (C₁-C₄)alkoxy group, a halogen atom and a hydroxy group.
R^{a} representing a (C₁-C₄)alkyl group, or any of its pharmaceutically acceptable salt.

Said sub-group of compounds are gathered under the "A1" type of compounds within the herein after table 1.

Still according to said embodiment, R¹ may more particularly represent a cyclopropylmethyl, a cyclopropyl, a cyclobutyl, a cyclopentyl, a cyclohexylmethyl, a cyclohexyl, a cycloheptylmethyl, a cycloheptyl, a cyclooctyl, a 3-hydroxy-2,2-dimethylpropyl, a 2-benzyloxyethyl, a 2-methylcyclohexyl, a 1-cyclohexylethyl, a 1-adamantylmethyl, a 1-(1-adamantyl)ethyl, a 1-adamantyl, a 2-adamantyl, a 3,5-dimethyl-1-adamantyl, a 5-hydroxy-2-adamantyl, a 3-hydroxy-1-adamantyl, a 3-methoxy-1-adamantyl, a 2,6,6-trimethylnorpinan-3-yl, a 6,6-dimethylnorpinan-2-yl, a spiro[2.5]octan-2-yl, a spiro[3.3]heptan-2-yl, a 1,7,7-trimethylnorbornan-2-yl, norbornan-2-yl, a 2-isopropyl-5-methyl-cyclohexyl, a 1-(cyclohexylmethyl)-2-hydroxy-ethyl, a 1-(cyclopentylmethyl)-2-hydroxy-ethyl, a 1-(cyclobutylmethyl)-2-hydroxy-ethyl, a 1-(cyclopropylmethyl)-2-hydroxy-ethyl, a 1-(hydroxymethyl)-3-methyl-butyl, a 1-(methoxymethyl)-3-methyl-butyl, a 1-(hydroxymethyl)propyl, a 1-(fluoromethyl)-3-methyl-butyl, a 1-cyclohexyl-2-hydroxyethyl, a 1-cyclohexyl-2-methoxy-ethyl, a 2-cyclohexyl-2-hydroxy-ethyl, a 2-cyclohexyl-2-methoxy-ethyl, a 2-hydroxycyclopentyl, a 2-methoxycyclopentyl, a 2-hydroxycyclohexyl, a 3-hydroxycyclohexyl, a 4-hydroxycyclohexyl, a 2-methoxycyclohexyl, a 4-methoxycyclohexyl, a 2-hydroxycycloheptyl, a 3-hydroxycycloheptyl, a 2-methoxycycloheptyl, a -CH(COOCH3)-CH(CH3)2, a -CH(COOCH3)-CH3, a - CH(COOCH₃)-CH₂-CH(CH₃)₂, a -CH(COOCH₃)-CHOH-CH₃.

According to another particular embodiment, the present invention relates to a compound of formula (I) as defined herein above wherein R¹ represents:
- a fused phenyl group, chosen from phenyl groups fused with a (C₅-C₆)cycloalkyl or (C₅-C₆)heterocycloalkyl, which (C₅-C₆)cycloalkyl and (C₅-C₆)heterocycloalkyl group optionally comprise an insaturation and is optionally substituted by a (C₁-C₄) alkyl group, a hydroxy group, a halogen atom, a (C₁-C₃)alkoxy group and a - COR^{a} group,
- a phenyl group, substituted by one or two groups selected from (C₁-C₈)alkyl, a (C₁-C₃)fluoroalkyl, a fluoro(C₁-C₄)alkoxy group a halogen atom, and a (C₄-C₇)heterocycloalkyl group said (C₄-C₇)heterocycloalkyl group being itself optionally substituted by a (C₁-C₄) alkyl group, or
- a R'-L- group, wherein
   ∘ L is a (C₁-C₃)alkanediyl group, optionnally susbstituted by a group chosen from a hydroxy group, a (C₁-C₃)alkoxy group, a -NR^{b}R^{c} group, a -COOR^{a} group and a halogen atom, and
   ∘ R' is a phenyl group, optionally substituted by one to three groups selected from the group consisting of (C₁-C₆)alkyl group, a fluoro(C₁-C₄)alkyl group and a fluoro(C₁-C₄)alkoxy group, a halogen atom and a hydroxy group,
      wherein R^{a} is a (C₁-C₄)alkyl or a hydrogen atom and R^{b} and R^{c} are independently chosen from (C₁-C₆)alkyl and a hydrogen atom
      or any pharmaceutically acceptable salt thereof.

Said sub-group of compounds are gathered under the "A2" and "A5" type of compounds within the herein after table 1.

Still according to said embodiment, R¹ may more particularly represent a benzyl, an indan-2-yl, a (3,4-dimethylphenyl)methyl, a (2,4-dimethylphenyl)methyl, a [2-(trifluoromethyl)phenyl]methyl, a [2-(trifluoromethoxy)phenyl]methyl, a 2-hydroxyindan-1-yl, a 2-methoxyindan-1-yl, a -CH(COOCH3)-CH2-Ph, a -CH(CH2F)Ph, a 2-amino-1-phenyl-ethyl, a 2-(methylamino)-1-phenyl-ethyl, a 2-(dimethylamino)-1-phenyl-ethyl, a 1-benzyl-2-hydroxy-ethyl, a 1-benzyl-2-methoxy-ethyl, a 2-hydroxy-1-phenyl-ethyl, a 2-methoxy-1-phenyl-ethyl, a 2-hydroxy-2-phenyl-ethyl, a 2-methoxy-2-phenyl-ethyl, a 2-hydroxy-3-phenyl-propyl, a 2-methoxy-3-phenyl-propyl, a 3-fluoro-4-methyl-phenyl, a 4-fluorophenyl, a 4-*n*-hexylphenyl, a 4-(4-methylpiperazin-1-yl)phenyl, a 3-(difluoromethoxy)phenyl, a 1-acetylindolin-6-yl, a 3-(trifluoromethyl)phenyl, an indan-5-yl, a 4-morpholinophenyl, a 1-methylindazol-7-yl.

According to another particular embodiment, the present invention relates to a compound of formula (I) as defined herein above wherein R¹ represents a R'-L- group wherein:
- R' is a (C₃-C₈)heteroaryl group, optionally substituted by one to three groups selected from a halogen atom, a (C₁-C₄)alkyl group, a (C₁-C₄)alkoxy group and a N-methylpiperazinyl group, and
- L is a (C₁-C₃)alkanediyl or a single bond
   or any pharmaceutically acceptable salt thereof.:

Said sub-group of compounds are gathered under the "A3" and "A6" type of compounds within the herein after table 1.

Still according to said embodiment, R¹ may more particularly represent a (5-methylpyrazin-2-yl)methyl, a 2-pyridylmethyl, a 3-pyridylmethyl, a 4-pyridylmethyl, a (5-methyl-2-furyl)methyl, a (4-methylthiazol-2-yl)methyl, a 3-imidazol-1-ylpropyl, a 2-(2-pyridyl)ethyl, a 1,3-benzothiazol-2-ylmethyl, a 2-pyrimidinyl, a 2-pyridyl, a 1-methylpyrazol-3-yl, a 2-methoxy-6-methyl-3-pyridyl, a pyrimidin-5-yl, 3-pyridyl, a 1,3,4-thiadiazol-2-yl, a 5-(4-methylpiperazin-1-yl)-2-pyridyl, 6-(4-methylpiperazin-1-yl)-3-pyridyl, a 2-(4-methylpiperazin-1-yl)pyrimidin-5-yl, a 5-(4-methylpiperazin-1-yl)pyrimidin-2-yl, a 5-(4-methylpiperazin-1-yl)pyrazin-2-yl, a 6-(4-methylpiperazin-1-yl)pyridazin-3-yl.

According to another particular embodiment, the present invention relates to a compound of formula (I) as defined herein above wherein R¹ represents a R'-L- group wherein:
- R' is a (C₃-C₅)heteroalkyl group, optionally substituted by one to two groups selected from a hydroxyl group, a (C₁-C₄)alkyl group and a -COOR^{a} group wherein R^{a} is as defined herein above, and
- L is a methylene or a single bond
   or any pharmaceutically acceptable salt thereof.

Said sub-group of compounds are gathered under the "A4" and "A7" type of compounds within the herein after table 1.

Still according to said embodiment, R¹ may more particularly represent a (1-methyl-4-piperidyl)methyl, a tetrahydropyran-4-ylmethyl, a 1-*tert-*butyloxycarbonylpiperidin-4-yl-methyl, a 7-methyl-7-azaspiro[3.5]nonan-2-yl, a tetrahydropyran-4-yl, a 1-*tert*-butyloxycarbonylpiperidin-4-yl, a 1-ethyloxycarbonylpiperidin-4-yl, a 1-methyl-4-piperidyl, a 1-methyl-3-piperidyl, an oxetan-3-yl, tetrahydrofuran-3-yl, a tetrahydropyran-3-yl, a 6,6-dimethyltetrahydropyran-3-yl, a 4-hydroxytetrahydropyran-3-yl, an oxepan-3-yl, a 2-oxo-piperidin-3-yl, a 2-oxo-piperidin-5-yl.

In the context of the present invention, the term:
- "halogen" is understood to mean chlorine, fluorine, bromine, or iodine, and in particular denotes chlorine, fluorine or bromine,
- "(C₁-Cₓ)alkyl", as used herein, respectively refers to a C₁-Cₓ normal, secondary or tertiary monovalent saturated hydrocarbon radical, for example (C₁-C₆)alkyl. Examples are, but are not limited to, methyl, ethyl, propyl, n-propyl, isopropyl, butyl, isobutyl, *sec-*butyl, *tert*-butyl, pentyl, isopentyl, hexyl and isohexyl groups, and the like,
- "(C₁-C₃)alkanediyl", as used herein, refers to a divalent saturated hydrocarbon radical which is branched or linear, comprises from 1 to 3 carbon atoms, and more particularly a methylene, ethylene or propylene, such as linear propylene or isopropylene, said alkanediyl may be substituted as it is apparent from the following description.
- "(C₃-C₈)cycloalkyl", as used herein, refers to a cyclic saturated hydrocarbon, from 3 to 8 carbon atoms, saturated or partially unsaturated and unsubstituted or substituted. Examples are, but are not limited to, cyclopropyl, cyclobutyl, cyclopentyl, cyclohexyl, cycloheptyl and cyclooctyl.
- "(C₃-C₈)heterocycloalkyl group", as used herein, refers to a (C₃-C₈)cycloalkyl group wherein one or two of the carbon atoms are replaced with a heteroatom such as oxygen, nitrogen or sulphur, and more particularly such as an oxygen or a nitrogen atom. Such heterocycloalkyl group may be saturated or partially saturated and unsubstituted or substituted. Examples are, but are not limited to, morpholine, piperazine, piperidine, pyrrolidine, aziridine, oxane, oxetane, tetrahydropyrane, morpholine, tetrahydrofurane, oxepane, diazepane and dioxane, and more particularly piperidine and piperazine, and even more particularly piperazine.
- "(C₁-Cₓ)alkoxy", as used herein, refers to a -O-(C₁-Cₓ)alkyl moiety, wherein alkyl is as defined above, for example (C₁-C₆)alkoxy. Examples are, but are not limited to, methoxy, ethoxy, 1-propoxy, 2-propoxy, butoxy and pentoxy.
- "spiro(C₅-C₁₁)bicyclic ring" refers to two rings connected through a defining single common atom. Such spiro bicyclic alkyl generally comprises 5 to 11 carbon atoms referring to a "spiro(C₅-C₁₁)bicyclic alkyl group". In a particular embodiment, one or more carbon atoms of the rings are replaced by heteroatom(s) such as oxygen, nitrogen or sulphur, and more particularly such as a nitrogen atom, forming a spiro(C₅-C₁₁)bicyclic hereoalkyl group. Such spirobicyclic ring may be unsubstituted or substituted, in particular by at least one (C₁-C₃)alkyl group such as methyl. Examples are, but are not limited to spiro[3.3]heptane, spiro[2.5]octane, 7-azaspiro[3.5]nonane.
- a "bridged (C₆-C₁₀)cycloalkyl" group, as used herein, refers to a bi- or tricyclic compound where the cycles are cycloalkyls, the rings share three or more atoms and the bridge contains at least one atom, for example 1, 2 or 3 atoms. Such bridged cycloalkyl groups may be substituted by one or more C₁-C₃ alkyl. Examples are, but not limited to adamantyl, 2,6,6-trimethylbicyclo[3.1.1]heptyl, 6,6-dimethylbicyclo[3.1.1]heptyl, bicyclo[3.1.1]heptyl, 1,6,6-trimethylbicyclo[3.1.1]heptyl.
- a "fused phenyl group" refers to a bicyclic radical that contains a phenyl moiety and may be substituted. Said fused phenyl group may be fused to a cycloalkyl or to a heterocycloalkyl and bound to the rest of the molecule either by its phenyl moeity or by said cycloalkyl or heterocycloalkyl. Examples are, but are not limited to indanyl, acetylindolinyl, methylindazolyl, hydroxyindanyl, benzothiazolyl, indolyl, indazolyl, methoxyindanyl and the like.
- a (C₅-C₁₁)heteroaryl group, as used herein, refers to a monocyclic aromatic group or to a bicyclic aromatic group where at least one of the ring is aromatic and wherein one to three ring carbon atom is replaced by a heteroatom, such as nitrogen, oxygen or sulphur. By way of examples of heteroaryl groups, mention may be made of, but not limited to: oxazole, isoxazole, pyridine, pyrimidine, pyridazine, triazine, pyrazine, oxadiazole, furane, pyrazole, thiazole, isothiazole, thiadiazole, imidazole, triazole and the like. In the framework of the present invention, the heteroaryl is advantageously pyridine, imidazole, pyrazine, furane, thiazole, pyrazole, thiadiazole, pyridazine and pyrimidine.
- an aromatic ring means, according to Hückel's rule, that a molecule has 4*n* + 2 π-electrons.
- a (C₁-Cₓ)fluoroalkyl group, as used herein, refers to a (C₁-Cₓ)alkyl as defined herein above in which one or more fluorines have been substituted by hydrogen. In one embodiment all the hydrogen atoms are replaced by fluor atoms, forming perfluoroalkyl groups, such as trifluoromethyl.
- a (C₁-Cₓ)fluoroalkoxy, as used herein, refers to a (C₁-Cₓ)alkoxy as defined herein above in which one or more fluorines have been substituted for hydrogen such as trifluoromethoxy. In one embodiment all the hydrogen atoms are replaced by fluor atoms, forming perfluoroalkoxy groups, such as trifluoromethoxy.

In the context of the present invention, the terms "aromatic ring", and "heteroaryl" include all the positional isomers.

The nomenclature of the following compounds (1) to (154) was generated according to the principles of the International Union of Pure and Applied Chemistry, using Accelrys Draw 4.1 SP1. To avoid any confusion, the "(±)" symbol added to designate a racemic mixture; "*cis*" and "*trans*" prefixes were also used to assign the relative stereochemistry of two adjacent chiral centers.

According to a preferred embodiment of the present invention, the compound of formula (I) is chosen from:
(1). (4*Z*)-4-(1,3-Benzothiazol-6-ylmethylene)-2-(cyclopropylmethylamino)-1*H*-imidazol-5-one.
(2). (4*Z*)-4-(1,3-Benzothiazol-6-ylmethylene)-2-(cyclopropylamino)-1*H-*imidazol-5-one.
(3). (4*Z*)-4-(1,3-Benzothiazol-6-ylmethylene)-2-(cyclobutylamino)-1*H-*imidazol-5-one.
(4). (4*Z*)-4-(1,3-Benzothiazol-6-ylmethylene)-2-(cyclopentylamino)-1*H-*imidazol-5-one.
(5). (4*Z*)-4-(1,3-Benzothiazol-6-ylmethylene)-2-(cyclohexylmethylamino)-1*H*-imidazol-5-one.
(6). (4*Z*)-4-(1,3-Benzothiazol-6-ylmethylene)-2-(cyclohexylamino)-1*H-*imidazol-5-one.
(7). (4*Z*)-4-(1,3-Benzothiazol-6-ylmethylene)-2-(cycloheptylmethylamino)-1*H*-imidazol-5-one.
(8). (4*Z*)-4-(1,3- Benzothiazol-6-ylmethylene)-2-(cycloheptylamino)-1*H-*imidazol-5-one.
(9). (4*Z*)-4-(1,3-Benzothiazol-6-ylmethylene)-2-(cyclooctylamino)-1*H-*imidazol-5-one.
(10). (4*Z*)-4-(1,3-Benzothiazol-6-ylmethylene)-2-[(3-hydroxy-2,2-dimethyl-propyl)aminol-1*H*-imidazol-5-one.
(11). (4*Z*)-4-(1,3-Benzothiazol-6-ylmethylene)-2-(2-benzyloxyethylamino)-1*H*-imidazol-5-one.
(12). (±)-(4*Z*)-4-(1,3-Benzothiazol-6-ylmethylene)-2-[[*cis*-2-methylcyclohexyl] amino] -1*H*-imidazol-5-one.
(13). (4*Z*)-4-(1,3-Benzothiazol-6-ylmethylene)-2-[[(1*R*)-1-cyclohexylethyl]amino]-1*H*-imidazol-5-one.
(14). (4*Z*)-2-(1-Adamantylmethylamino)-4-(1,3-benzothiazol-6-ylmethylene)-1*H*-imidazol-5-one.
(15). (±)-(4*Z*)-2-[1-(1-Adamantyl)ethylamino]-4-(1,3-benzothiazol-6-ylmethylene)-1*H*-imidazol-5-one.
(16). (4*Z*)-2-(1-Adamantylamino)-4-(1,3-benzothiazol-6-ylmethylene)-1*H-*imidazol-5-one.
(17). (4*Z*)-2-(2-Adamantylamino)-4-(1,3-benzothiazol-6-ylmethylene)-1*H-*imidazol-5-one.
(18). (4*Z*)-4-(1,3-Benzothiazol-6-ylmethylene)-2-[[3,5-dimethyl-1-adamantyl] amino]-1*H*-imidazol-5-one.
(19). (4*Z*)-4-(1,3-Benzothiazol-6-ylmethylene)-2-[(*trans*-5-hydroxy-2-adamantyl)amino]-1*H*-imidazol-5-one.
(20). (4*Z*)-4-(1,3-Benzothiazol-6-ylmethylene)-2-[(3-hydroxy-1-adamantyl)amino]-1*H*-imidazol-5-one.
(21). (4*Z*)-4-(1,3-B enzothiazol-6-ylmethylene)-2-[(3-methoxy-1-adamantyl)amino]-1*H*-imidazol-5-one.
(22). (4*Z*)-4-(1,3-Benzothiazol-6-ylmethylene)-2-[[(1*R*,2*R*,3*R*,5*S*)-2,6,6-trimethylnorpinan-3-yl]amino]-1*H*-imidazol-5-one.
(23). (4*Z*)-4-(1,3-Benzothiazol-6-ylmethylene)-2-[[(1*S*,2*S*,3*S*,5*R*)-2,6,6-trimethylnorpinan-3-yl]amino]-1*H*-imidazol-5-one.
(24). (4*Z*)-4-(1,3-Benzothiazol-6-ylmethylene)-2-[[(1*R*,2*R*,5*R*)-6,6-dimethylnorpinan-2-yl]methylamino]-1*H*-imidazol-5-one.
(25). (±)-(4*Z*)-4-(1,3-Benzothiazol-6-ylmethylene)-2-(spiro[2.5]octan-2-ylamino)-1*H*-imidazol-5-one.
(26). (4*Z*)-4-(1,3-Benzothiazol-6-ylmethylene)-2-(spiro[3.3]heptan-2-ylamino)-1*H*-imidazol-5-one.
(27). (4*Z*)-4-(1,3-Benzothiazol-6-ylmethylene)-2-[[(2*R*)-1,7,7-trimethylnorbornan-2-yl]amino]-1*H*-imidazol-5-one.
(28). (±)-(4*Z*)-4-(1,3-Benzothiazol-6-ylmethylene)-2-(norbornan-2-ylamino)-1*H*-imidazol-5-one.
(29). (4*Z*)-4-(1,3-Benzothiazol-6-ylmethylene)-2-[[(1*R*,2*S*,5*R*)-2-isopropyl-5-methyl-cyclohexyl]ammo]-1*H*-imidazol-5-one.
(30). (4*Z*)-4-(1,3-Benzothiazol-6-ylmethylene)-2-[[(1*R*)-1-(cyclohexylmethyl)-2-hydroxy-ethyl]amino]-1*H*-imidazol-5-one.
(31). (4*Z*)-4-(1,3-Benzothiazol-6-ylmethylene)-2-[[(1*R*)-1-(cyclopentylmethyl)-2-hydroxy-ethyl]amino]-1*H*-imidazol-5-one.
(32). (4*Z*)-4-(1,3-Benzothiazol-6-ylmethylene)-2-[[(1*R*)-1-(cyclobutylmethyl)-2-hydroxy-ethyl]amino]-1*H*-imidazol-5-one.
(33). (4*Z*)-4-(1,3-Benzothiazol-6-ylmethylene)-2-[[(1*R*)-1-(cyclopropylmethyl)-2-hydroxy-ethyl]amino]-1*H*-imidazol-5-one.
(34). (4*Z*)-4-(1,3-Benzothiazol-6-ylmethylene)-2-[[(1*R*)-1-(hydroxymethyl)-3-methyl-butyl]amino]-1*H*-imidazol-5-one.
(35). (4*Z*)-4-(1,3-Benzothiazol-6-ylmethylene)-2-[[(1*R*)-1-(methoxymethyl)-3-methyl-butyl]amino]-1*H*-imidazol-5-one.
(36). (4*Z*)-4-(1,3-Benzothiazol-6-ylmethylene)-2-[[(1*S*)-1-(hydroxymethyl)-3-methyl-butyl]amino]-1*H*-imidazol-5-one.
(37). (4*Z*)-4-(1,3-Benzothiazol-6-ylmethylene)-2-[[(1*S*)-1-(methoxymethyl)-3-methyl-butyl]amino]-1*H*-imidazol-5-one.
(38). (4*Z*)-4-(1,3-Benzothiazol-6-ylmethylene)-2-[[(1*R*)-1-(hydroxymethyl)propyl]amino]-1*H*-imidazol-5-one.
(39). (4*Z*)-4-(1,3-Benzothiazol-6-ylmethylene)-2-[[(1*S*)-1-(hydroxymethyl)propyl]amino]-1*H*-imidazol-5-one.
(40). (±)-(4*Z*)-4-(1,3-Benzothiazol-6-ylmethylene)-2-[[1-(fluoromethyl)-3-methyl-butyl]amino]-1*H*-imidazol-5-one.
(41). (±)-(4*Z*)-4-(1,3-Benzothiazol-6-ylmethylene)-2-[(1-cyclohexyl-2-hydroxy-ethyl)amino]-1*H*-imidazol-5-one.
(42). (±)-(4*Z*)-4-(1,3-Benzothiazol-6-ylmethylene)-2-[(1-cyclohexyl-2-methoxy-ethyl)amino]-1*H*-imidazol-5-one.
(43). (±)-(4*Z*)-4-(1,3-Benzothiazol-6-ylmethylene)-2-[(2-cyclohexyl-2-hydroxy-ethyl)amino]-1*H*-imidazol-5-one.
(44). (±)-(4*Z*)-4-(1,3-Benzothiazol-6-ylmethylene)-2-[(2-cyclohexyl-2-methoxy-ethyl)amino]-1*H*-imidazol-5-one.
(45). (±)-(4*Z*)-4-(1,3-Benzothiazol-6-ylmethylene)-2-[[*cis*-2-hydroxycyclopentyl]amino]-1*H*-imidazol-5-one.
(46). (±)-(4*Z*)-4-(1,3-Benzothiazol-6-ylmethylene)-2-[[*trans*-2-hydroxycyclopentyl]amino]-1*H*-imidazol-5-one.
(47). (±)-(4*Z*)-4-(1,3-Benzothiazol-6-ylmethylene)-2-[[*cis*-2-methoxycyclopentyl]amino]-1*H*-imidazol-5-one.
(48). (±)-(4*Z*)-4-(1,3-Benzothiazol-6-ylmethylene)-2-[[*trans*-2-methoxycyclopentyl]amino]-1*H*-imidazol-5-one.
(49). (±)-(4*Z*)-4-(1,3-Benzothiazol-6-ylmethylene)-2-[[*cis*-2-hydroxycyclohexyl]amino]-1*H*-imidazol-5-one.
(50). (±)-(4*Z*)-4-(1,3-Benzothiazol-6-ylmethylene)-2-[[*trans*-2-hydroxycyclohexyl]amino]-1*H*-imidazol-5-one.
(51). (4*Z*)-4-(1,3-Benzothiazol-6-ylmethylene)-2-[[(1*R*,2*S*)-2-hydroxycyclohexyl]amino]-1*H*-imidazol-5-one.
(52). (4*Z*)-4-(1,3-Benzothiazol-6-ylmethylene)-2-[[(1*S*,2*R*)-2-hydroxycyclohexyl]amino]-1*H*-imidazol-5-one.
(53). (4Z)-4-(1,3-Benzothiazol-6-ylmethylene)-2-[[(1*R*,2*R*)-2-hydroxycyclohexyl]amino]-1*H*-imidazol-5-one.
(54). (4*Z*)-4-(1,3-Benzothiazol-6-ylmethylene)-2-[[(1*S*,2*S*)-2-hydroxycyclohexyl]amino]-1*H*-imidazol-5-one.
(55). (±)-(4*Z*)-4-(1,3-Benzothiazol-6-ylmethylene)-2-[[*cis*-3-hydroxycyclohexyl]amino]-1*H*-imidazol-5-one.
(56). (±)-(4*Z*)-4-(1,3-Benzothiazol-6-ylmethylene)-2-[[*trans*-3-hydroxycyclohexyl]amino]-1*H*-imidazol-5-one.
(57). (4*Z*)-4-(1,3-Benzothiazol-6-ylmethylene)-2-[(*trans*-4-hydroxycyclohexyl)amino]-1*H*-imidazol-5-one.
(58). (±)-(4*Z*)-4-(1,3-Benzothiazol-6-ylmethylene)-2-[[*cis*-2-methoxycyclohexyl]amino]-1*H*-imidazol-5-one.
(59). (±)-(4*Z*)-4-(1,3-Benzothiazol-6-ylmethylene)-2-[[*trans*-2-methoxycyclohexyl]amino]-1*H*-imidazol-5-one.
(60). (4*Z*)-4-(1,3-Benzothiazol-6-ylmethylene)-2-[(*trans*-4-methoxycyclohexyl)amino]-1*H*-imidazol-5-one.
(61). (±)-(4*Z*)-4-(1,3-Benzothiazol-6-ylmethylene)-2-[[*cis*-2-hydroxycycloheptyl]amino]-1*H*-imidazol-5-one.
(62). (±)-(4*Z*)-4-(1,3-Benzothiazol-6-ylmethylene)-2-[[*trans*-2-hydroxycycloheptyl]amino]-1*H*-imidazol-5-one.
(63). (4*Z*)-4-(1,3-Benzothiazol-6-ylmethylene)-2-[[(1*R*,2*R*)-2-hydroxycycloheptyl]amino]-1*H*-imidazol-5-one.
(64). (4*Z*)-4-(1,3-Benzothiazol-6-ylmethylene)-2-[[(1*S*,2*S*)-2-hydroxycycloheptyl]amino]-1*H*-imidazol-5-one.
(65). (±)-(4*Z*)-4-(1,3-Benzothiazol-6-ylmethylene)-2-[[*cis*-3-hydroxycycloheptyl]amino]-1*H*-imidazol-5-one.
(66). (±)-(4*Z*)-4-(1,3-Benzothiazol-6-ylmethylene)-2-[[*trans*-3-hydroxycycloheptyl]amino]-1*H*-imidazol-5-one.
(67). (±)-(4*Z*)-4-(1,3-Benzothiazol-6-ylmethylene)-2-[[*cis*-2-methoxycycloheptyl]amino]-1*H*-imidazol-5-one.
(68). (±)-(4*Z*)-4-(1,3-Benzothiazol-6-ylmethylene)-2-[[*trans*-2-methoxycycloheptyl]amino]-1*H*-imidazol-5-one.
(69). Methyl (2*S*)-2-[[(4*Z*)-4-(1,3-benzothiazol-6-ylmethylene)-5-oxo-1*H-*imidazol-2-yl]amino]-3-methyl-butanoate.
(70). Methyl (2*S*)-2-[[(4*Z*)-4-(1,3-benzothiazol-6-ylmethylene)-5-oxo-1*H-*imidazol-2-yl]amino]propanoate.
(71). Methyl (2*S*)-2-[[(4*Z*)-4-(1,3-benzothiazol-6-ylmethylene)-5-oxo-1*H-*imidazol-2-yl]amino]-4-methyl-pentanoate.
(72). Methyl (2*R*)-2-[[(4*Z*)-4-(1,3-benzothiazol-6-ylmethylene)-5-oxo-1*H-*imidazol-2-yl]amino]-4-methyl-pentanoate.
(73). Methyl (2*S*)-2-[[(4*Z*)-4-(1,3-benzothiazol-6-ylmethylene)-5-oxo-1*H-*imidazol-2-yl]amino]-3-hydroxy-butanoate.
(74). (4*Z*)-4-(1,3-Benzothiazol-6-ylmethylene)-2-(benzylamino)-1*H-*imidazol-5-one.
(75). (4*Z*)-4-(1,3-Benzothiazol-6-ylmethylene)-2-(indan-2-ylamino)-1*H-*imidazol-5-one.
(76). (4*Z*)-4-(1,3-Benzothiazol-6-ylmethylene)-2-[(3,4-dimethylphenyl)methylamino]-1*H*-imidazol-5-one.
(77). (4*Z*)-4-(1,3-Benzothiazol-6-ylmethylene)-2-[(2,4-dimethylphenyl)methylamino]-1*H*-imidazol-5-one.
(78). (4*Z*)-4-(1,3-Benzothiazol-6-ylmethylene)-2-[[2-(trifluoromethyl)phenyl]methylamino]-1*H*-imidazol-5-one.
(79). (4*Z*)-4-(1,3-Benzothiazol-6-ylmethylene)-2-[[2-(trifluoromethoxy)phenyl]methylamino]-1*H*-imidazol-5-one.
(80). (±)-(4*Z*)-4-(1,3-Benzothiazol-6-ylmethylene)-2-[[*cis*-2-hydroxyindan-1-yl]amino]-1*H*-imidazol-5-one.
(81). (±)-(4*Z*)-4-(1,3-Benzothiazol-6-ylmethylene)-2-[[*trans*-2-hydroxyindan-1-yl]amino]-1*H*-imidazol-5-one.
(82). (4*Z*)-4-(1,3-Benzothiazol-6-ylmethylene)-2-[[(1*R*,2*R*)-2-hydroxyindan-1-yl]amino]-1*H*-imidazol-5-one.
(83). (4*Z*)-4-(1,3-Benzothiazol-6-ylmethylene)-2-[[(1*S*,2*S*)-2-hydroxyindan-1-yl]amino]-1*H*-imidazol-5-one.
(84). (±)-(4*Z*)-4-(1,3-Benzothiazol-6-ylmethylene)-2-[[*cis*-2-methoxyindan-1-yl]amino]-1*H*-imidazol-5-one.
(85). (±)-(4*Z*)-4-(1,3-Benzothiazol-6-ylmethylene)-2-[[*trans*-2-methoxyindan-1-yl]amino]-1*H*-imidazol-5-one.
(86). Methyl (2*S*)-2-[[(4*Z*)-4-(1,3-benzothiazol-6-ylmethylene)-5-oxo-1H-imidazol-2-yl]amino]-3-phenyl-propanoate.
(87). Methyl (2*R*)-2-[[(4*Z*)-4-(1,3-benzothiazol-6-ylmethylene)-5-oxo-1*H-*imidazol-2-yl]amino]-3-phenyl-propanoate.
(88). (±)-(4*Z*)-4-(1,3-Benzothiazol-6-ylmethylene)-2-[(2-fluoro-1-phenyl-ethyl)amino]-1*H*-imidazol-5-one.
(89). (±)-(4*Z*)-2-[(2-Amino-1-phenyl-ethyl)amino]-4-(1,3-benzothiazol-6-ylmethylene)-1*H*-imidazol-5-one dihydrochloride.
(90). (±)-(4*Z*)-4-(1,3-Benzothiazol-6-ylmethylene)-2-[[2-(methylamino)-1-phenyl-ethyl]amino]-1*H*-imidazol-5-one dihydrochloride.
(91). (±)-(4*Z*)-4-(1,3-Benzothiazol-6-ylmethylene)-2-[[2-(dimethylamino)-1-phenyl-ethyl]amino]-1*H*-imidazol-5-one.
(92). (±)-(4Z)-4-(1,3-Benzothiazol-6-ylmethylene)-2-[(1-benzyl-2-hydroxyethyl)amino]-1*H*-imidazol-5-one.
(93). (4*Z*)-4-(1,3-Benzothiazol-6-ylmethylene)-2-[[(1*R*)-1-benzyl-2-hydroxyethyl]amino]-1*H*-imidazol-5-one.
(94). (±)-(4*Z*)-4-(1,3-Benzothiazol-6-ylmethylene)-2-[(1-benzyl-2-methoxyethyl)amino]-1*H*-imidazol-5-one.
(95). (±)-(4*Z*)-4-(1,3-Benzothiazol-6-ylmethylene)-2-[(2-hydroxy-1-phenylethyl)amino]-1*H*-imidazol-5-one.
(96). (4*Z*)-4-(1,3-Benzothiazol-6-ylmethylene)-2-[[(1*R*)-2-hydroxy-1-phenylethyl]amino]-1*H*-imidazol-5-one.
(97). (4*Z*)-4-(1,3-Benzothiazol-6-ylmethylene)-2-[[(1*S*)-2-hydroxy-1-phenylethyl]amino]-1*H*-imidazol-5-one.
(98). (±)-(4*Z*)-4-(1,3-Benzothiazol-6-ylmethylene)-2-[(2-methoxy-1-phenylethyl)amino]-1*H*-imidazol-5-one.
(99). (±)-(4*Z*)-4-(1,3-Benzothiazol-6-ylmethylene)-2-[(2-hydroxy-2-phenylethyl)amino]-1*H*-imidazol-5-one.
(100). (±)-(4*Z*)-4-(1,3-Benzothiazol-6-ylmethylene)-2-[(2-methoxy-2-phenylethyl)amino]-1*H*-imidazol-5-one.
(101). (±)-(4*Z*)-4-(1,3-Benzothiazol-6-ylmethylene)-2-[(2-hydroxy-3-phenylpropyl)amino]-1*H*-imidazol-5-one.
(102). (±)-(4*Z*)-4-(1,3-Benzothiazol-6-ylmethylene)-2-[(2-methoxy-3-phenylpropyl)amino]-1*H*-imidazol-5-one.
(103). (4*Z*)-4-(1,3-Benzothiazol-6-ylmethylene)-2-[(5-methylpyrazin-2-yl)methylamino]-1*H*-imidazol-5-one.
(104). (4*Z*)-4-(1,3-Benzothiazol-6-ylmethylene)-2-(2-pyridylmethylamino)-1*H*-imidazol-5-one.
(105). (4*Z*)-4-(1,3-Benzothiazol-6-ylmethylene)-2-(3-pyridylmethylamino)-1*H*-imidazol-5-one.
(106). (4*Z*)-4-(1,3-Benzothiazol-6-ylmethylene)-2-(4-pyridylmethylamino)-1*H*-imidazol-5-one.
(107). (4*Z*)-4-(1,3-Benzothiazol-6-ylmethylene)-2-[(5-methyl-2-furyl)methylamino]-1*H*-imidazol-5-one.
(108). (4*Z*)-4-(1,3-Benzothiazol-6-ylmethylene)-2-[(4-methylthiazol-2-yl)methylamino]-1*H*-imidazol-5-one.
(109). (4*Z*)-4-(1,3-Benzothiazol-6-ylmethylene)-2-(3-imidazol-1-ylpropylamino)-1*H*-imidazol-5-one.
(110). (4*Z*)-4-(1,3-Benzothiazol-6-ylmethylene)-2-[2-(2-pyridyl)ethylamino]-1*H*-imidazol-5-one.
(111). (4*Z*)-2-(1,3-Benzothiazol-2-ylmethylamino)-4-(1,3-benzothiazol-6-ylmethylene)-1*H*-imidazol-5-one.
(112). (4*Z*)-4-(1,3-Benzothiazol-6-ylmethylene)-2-[(1-methyl-4-piperidyl)methylamino]-1*H*-imidazol-5-one.
(113). (4*Z*)-4-(1,3-Benzothiazol-6-ylmethylene)-2-(tetrahydropyran-4-ylmethylamino)-1*H*-imidazol-5-one.
(114). *Tert*-butyl 4-[[[(4*Z*)-4-(1,3-benzothiazol-6-ylmethylene)-5-oxo-1*H-*imidazol-2-yl]amino]methyl]piperidine-1-carboxylate.
(115). (4*Z*)-4-(1,3-Benzothiazol-6-ylmethylene)-2-[(7-methyl-7-azaspiro[3.5]nonan-2-yl)amino]-1*H*-imidazol-5-one.
(116). (4*Z*)-4-(1,3-Benzothiazol-6-ylmethylene)-2-(3-fluoro-4-methyl-anilino)-1*H*-imidazol-5-one.
(117). (4*Z*)-4-(1,3-Benzothiazol-6-ylmethylene)-2-(4-fluoroanilino)-1*H-*imidazol-5-one.
(118). (4*Z*)-4-(1,3-Benzothiazol-6-ylmethylene)-2-(4-hexylanilino)-1*H-*imidazol-5-one.
(119). (4*Z*)-4-(1,3-Benzothiazol-6-ylmethylene)-2-[4-(4-methylpiperazin-1-yl)anilino]-1*H*-imidazol-5-one.
(120). (4*Z*)-4-(1,3-Benzothiazol-6-ylmethylene)-2-[3-(difluoromethoxy)anilino]-1*H*-imidazol-5-one.
(121). (4Z)-2-[(1-Acetylindolin-6-yl)amino]-4-(1,3-benzothiazol-6-ylmethylene)-1*H*-imidazol-5-one.
(122). (4*Z*)-4-(1,3-Benzothiazol-6-ylmethylene)-2-[3-(trifluoromethyl)anilino]-1*H*-imidazol-5-one.
(123). (4*Z*)-4-(1,3-Benzothiazol-6-ylmethylene)-2-(indan-5-ylamino)-1*H-*imidazol-5-one.
(124). (4*Z*)-4-(1,3-Benzothiazol-6-ylmethylene)-2-(4-morpholinoanilino)-1*H-*imidazol-5-one.
(125). (4Z)-4-(1,3-Benzothiazol-6-ylmethylene)-2-[(1-methylindazol-7-yl)amino]-1*H*-imidazol-5-one.
(126). (4*Z*)-4-(1,3-Benzothiazol-6-ylmethylene)-2-(pyrimidin-2-ylamino)-1*H-*imidazol-5-one.
(127). (4*Z*)-4-(1,3-Benzothiazol-6-ylmethylene)-2-(2-pyridylamino)-1*H-*imidazol-5-one.
(128). (4*Z*)-4-(1,3-Benzothiazol-6-ylmethylene)-2-[(1-methylpyrazol-3-yl)amino]-1*H*-imidazol-5-one.
(129). (4*Z*)-4-(1,3-Benzothiazol-6-ylmethylene)-2-[(2-methoxy-6-methyl-3-pyridyl)amino]-1H-imidazol-5-one.
(130). (4*Z*)-4-(1,3-Benzothiazol-6-ylmethylene)-2-(pyrimidin-5-ylamino)-1*H-*imidazol-5-one.
(131). (4*Z*)-4-(1,3-Benzothiazol-6-ylmethylene)-2-(3-pyridylamino)-1*H-*imidazol-5-one.
(132). (4*Z*)-4-(1,3-Benzothiazol-6-ylmethylene)-2-(1,3,4-thiadiazol-2-ylamino)-1*H*-imidazol-5-one.
(133). (4*Z*)-4-(1,3-Benzothiazol-6-ylmethylene)-2-[[5-(4-methylpiperazin-1-yl)-2-pyridyl]amino]-1*H*-imidazol-5-one.
(134). (4*Z*)-4-(1,3-Benzothiazol-6-ylmethylene)-2-[[6-(4-methylpiperazin-1-yl)-3-pyridyl]amino]-1*H*-imidazol-5-one.
(135). (4Z)-4-(1,3-Benzothiazol-6-ylmethylene)-2-[[2-(4-methylpiperazin-1-yl)pyrimidin-5-yl]amino]-1*H*-imidazol-5-one.
(136). (4*Z*)-4-(1,3-Benzothiazol-6-ylmethylene)-2-[[5-(4-methylpiperazin-1-yl)pyrimidin-2-yl]amino]-1*H*-imidazol-5-one.
(137). (4*Z*)-4-(1,3-Benzothiazol-6-ylmethylene)-2-[[5-(4-methylpiperazin-1-yl)pyrazin-2-yl]amino]-1*H*-imidazol-5-one.
(138). (4*Z*)-4-(1,3-Benzothiazol-6-ylmethylene)-2-[[6-(4-methylpiperazin-1-yl)pyridazin-3-yl]amino]-1*H*-imidazol-5-one.
(139). (4*Z*)-4-(1,3-Benzothiazol-6-ylmethylene)-2-(tetrahydropyran-4-ylamino)-1*H*-imidazol-5-one.
(140). *Tert*-butyl 4-[[(4Z)-4-(1,3-benzothiazol-6-ylmethylene)-5-oxo-1*H-*imidazol-2-yl]amino]piperidine-1-carboxylate.
(141). Ethyl 4-[[(4Z)-4-(1,3-benzothiazol-6-ylmethylene)-5-oxo-1*H*-imidazol-2-yl]amino]piperidine-1-carboxylate.
(142). (4*Z*)-4-(1,3-benzothiazol-6-ylmethylene)-2-[(1-methyl-4-piperidyl)amino]-1*H*-imidazol-5-one.
(143). (±)-(4*Z*)-4-(1,3-Benzothiazol-6-ylmethylene)-2-[(1-methyl-3-piperidyl)amino]-1*H*-imidazol-5-one.
(144). (4*Z*)-4-(1,3-Benzothiazol-6-ylmethylene)-2-(oxetan-3-ylamino)-1*H-*imidazol-5-one.
(145). (4*Z*)-4-(1,3-benzothiazol-6-ylmethylene)-2-[[(3*R*)-tetrahydrofuran-3-yl]amino]-1*H*-imidazol-5-one.
(146). (4*Z*)-4-(1,3-Benzothiazol-6-ylmethylene)-2-[[(3*S*)-tetrahydrofuran-3-yl]amino]-1*H*-imidazol-5-one.
(147). (4*Z*)-4-(1,3-Benzothiazol-6-ylmethylene)-2-[[(3*R*)-tetrahydropyran-3-yl]amino]-1*H*-imidazol-5-one.
(148). (4*Z*)-4-(1,3-Benzothiazol-6-ylmethylene)-2-[[(3*S*)-tetrahydropyran-3-yl]amino]-1*H*-imidazol-5-one.
(149). (±)-(4*Z*)-4-(1,3-Benzothiazol-6-ylmethylene)-2-[(6,6-dimethyltetrahydropyran-3-yl)amino]-1*H*-imidazol-5-one.
(150). (4*Z*)-4-(1,3-Benzothiazol-6-ylmethylene)-2-[[(3*R*,4*R*)-4-hydroxytetrahydropyran-3-yl]amino]-1*H*-imidazol-5-one.
(151). (±)-(4*Z*)-4-(1,3-Benzothiazol-6-ylmethylene)-2-(oxepan-3-ylamino)-1*H*-imidazol-5-one.
(152). (±)-3-[[(4*Z*)-4-(1,3-Benzothiazol-6-ylmethylene)-5-oxo-1*H*-imidazol-2-yl] amino]piperidin-2-one.
(153). (3*S*)-3-[[(4*Z*)-4-(1,3-Benzothiazol-6-ylmethylene)-5-oxo-1*H*-imidazol-2-yl]amino]piperidin-2-one.
(154). (5*S*)-5-[[(4*Z*)-4-(1,3-Benzothiazol-6-ylmethylene)-5-oxo-1*H*-imidazol-2-yl]amino]piperidin-2-one.
and their pharmaceutically acceptable salts.

According to an even more preferred embodiment of the present invention, the compound of formula (I) is chosen from the group consisting of compounds (4), (5), (6), (7), (8), (9), (12), (13), (14), (16), (17), (18), (19), (20), (22), (23), (24), (25), (27), (28), (30), (31), (32), (34), (35), (36), (38), (40), (41), (42), (43), (44), (46), (48), (49), (50), (53), (55), (56), (57), (59), (60), (61), (62), (63), (64), (65), (66), (67), (68), (69), (70), (71), (72), (73), (74), (77), (78), (80), (81), (83), (85), (86), (89), (90), (92), (93), (94), (95), (98), (99), (104), (106), (108), (117), (119), (125), (127), (128), (135), (146), (147), (148), (149), (150), (151), (154) and their pharmaceutically acceptable salts.

According to an even more preferred embodiment of the present invention, the compound of formula (I) is chosen from the group consisting of compounds (6), (7), (8), (9), (12), (14), (16), (17), (19), (20), (22), (24), (25), (27), (28), (31), (32), (34), (35), (36), (38), (40), (41), (43), (44), (46), (48), (49), (53), (55), (56), (57), (59), (61), (62), (63), (64), (65), (66), (67), (68), (69), (74), (77), (78), (81), (83), (85), (86), (89), (90), (92), (93), (95), (98), (99), (108), (119), (125), (146), (148), (149), (150), (151) and their pharmaceutically acceptable salts.

According to an even more preferred embodiment of the present invention, the compound of formula (I) is chosen from the group consisting of compounds (9), (16), (17), (19), (20), (22), (25), (27), (34), (35), (40), (48), (61), (65), (66), (78), (81), (83), (89), (95), (98), (99) and their pharmaceutically acceptable salts.

According to an alternative embodiment of the present invention, the compound of formula (I) is chosen from the group consisting of compounds (1), (3), (4), (5), (6), (7)), (8), (9), (10), (12), (13), (14), (16), (17), (19), (20), (22), (23), (24), (25), (26), (27), (28), (30), (31), (32), (33), (34), (35), (36), (37), (38), (39), (40), (41), (42), (43), (44), (45), (46), (47), (48), (49), (50), (53), (55), (56), (57), (58), (59), (60), (61), (62), (63), (64), (65), (66), (67), (68), (69), (70), (71), (72), (73), (74), (75), (76), (77), (78), (79), (80), (81), (82), (83), (85), (86), (88), (89), (90), (92), (93), (94), (95), (98), (99), (100), (101), (102), (103), (104), (105), (106), (107), (108), (109), (110), (111), (113), (117), (119), (120), (121), (126), (127), (128), (129), (130), (131), (132), (135), (137), (139), (141), (144), (145), (146), (147), (148), (149), (150), (151), (154) and their pharmaceutically acceptable salts.

According to a preferred embodiment of the present invention, the compound of formula (I) is chosen from the group consisting of compounds (1), (4), (5), (6), (7), (8), (9), (10), (12), (13), (14), (16), (17), (19), (20), (22), (23), (24), (25), (26), (27), (28), (30), (31), (32), (33), (34), (35), (36), (38), (40), (41), (43), (44), (46), (48), (49), (53), (55), (56), (57), (59), (61), (62), (63), (64), (65), (66), (67), (68), (69), (70), (71), (73), (74), (76), (77), (78), (79), (80), (81), (82), (83), (85), (86), (88), (89), (90), (92), (93), (94), (95), (98), (99), (100), (101), (102), (104), (105), (106), (108), (109), (113), (117), (119), (127), (128), (130), (131), (135), (139), (141), (146), (147), (148), (149), (150), (151) and their pharmaceutically acceptable salts.

According to an even more preferred embodiment of the present invention, the compound of formula (I) is chosen from the group consisting of compounds (9), (16), (19), (20, (35, (48, (61, (73, (78, (81), (95) and their pharmaceutically acceptable salts.

According to a further embodiment of the present invention, the compound of formula (I) is chosen from the group consisting of compounds (7), (8), (9), (10), (12), (16), (17), (19), (20), (23), (25), (26), (28), (32), (33), (34), (35), (40), (41), (43), (44), (46), (48), (55), (56), (57), (59), (61), (62), (63), (65), (66), (67), (68), (69), (70), (73), (77), (78), (81), (83), (88), (89), (90), (95), (98), (99), (100), (101), (102), (104), (105), (106), (117), (119), (127), (131), (139), (148), (149), (151) and their pharmaceutically acceptable salts.

The groups of compounds as defined by the list of compounds as identified in Example 4 below through tables 4A to table 4F and specifically identified as most potent kinase inhibitors, as well as multi-target kinase inhibitors, also form part of the present invention.

According to another aspect, a subject-matter of the present invention relates to a compound of formula (I) as defined above or any of its pharmaceutically acceptable salts, or at least any of compounds (1) to (154) or any of its pharmaceutically acceptable salts, for use as a medicament.

« Pharmaceutically acceptable salt thereof » refers to salts which are formed from acid addition salts formed with inorganic acids (e.g. hydrochloric acid, hydrobromic acid,), as well as salts formed with organic acids such as acetic acid, tartaric acid, succinic acid.

Suitable physiologically acceptable acid addition salts of compounds of formula (I) include hydrobromide, tartrate, hydrochloride, succinate and acetate.

The compounds of formula (I), and any of compounds (1) to (154) or any of their pharmaceutically acceptable salts may form solvates or hydrates and the invention includes all such solvates and hydrates.

The terms "hydrates" and "solvates" simply mean that the compounds (I) according to the invention can be in the form of a hydrate or solvate, *i.e.* combined or associated with one or more water or solvent molecules. This is only a chemical characteristic of such compounds, which can be applied for all organic compounds of this type.

The compounds of formula (I) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

**List of abbreviations:**

| **Abbreviation/acronym** | **name** |
|---|---|
| **Ac** | Acetyl |
| **ACN** | Acetonitrile |
| **Alk** | Alkyl |
| **ATP** | Adenosine triphosphate |
| **C** | Concentration |
| **Cpd N°** | Compound number |
| **DCM** | Methylene chloride |
| **DIPEA** | *N*,*N*-Diisopropylethylamine |
| **DMF** | Dimethylformamide |
| **DMSO** | Dimethylsulfoxide |
| **DTT** | Dithiothreitol |
| **Eq** | Equivalent |
| **ESI** | Electrospray ionization |
| **Et** | Ethyl |
| **FC** | flash chromatography |
| **GP** | General protocol |
| **GST** | Glutathione S-transferase |
| **Hal** | Halogen |
| **HEPES** | 4-(2-Hydroxyethyl)-1-piperazineethanesulfonic acid |
| **His- tagged** | Polyhistidine-tagged |
| **HPLC** | High pressure liquid chromatography |
| **IC₅₀** | Half maximal inhibitory concentration |
| **M** | Molarity |
| **Me** | Methyl |
| **MS** | Mass spectroscopy |
| **MW** | Molecular weight |
| **NMR** | Nuclear magnetic resonance |
| **N/A** | Not applicable |
| **n.t.** | Not tested |
| **Rac** | Racemic |
| **r.t.** | Room temperature |
| **SDS-PAGE** | Sodium dodecyl sulfate-polyacrylamide gel electrophoresis |
| **TEA** | Triethylamine |
| **THF** | Tetrahydrofuran |
| **TLC** | Thin layer chromatography |
| **PTLC** | Preparative thin layer chromatography |
| **UV** | Ultraviolet |
| **v/v** | Volume per volume |
| **w/v** | Weight per volume |
| **δ_{H}** | Hydrogen chemical shift |
| **µw** | Microwave irradiation |

The compounds of general formula (I) can be prepared according to scheme 1 below.

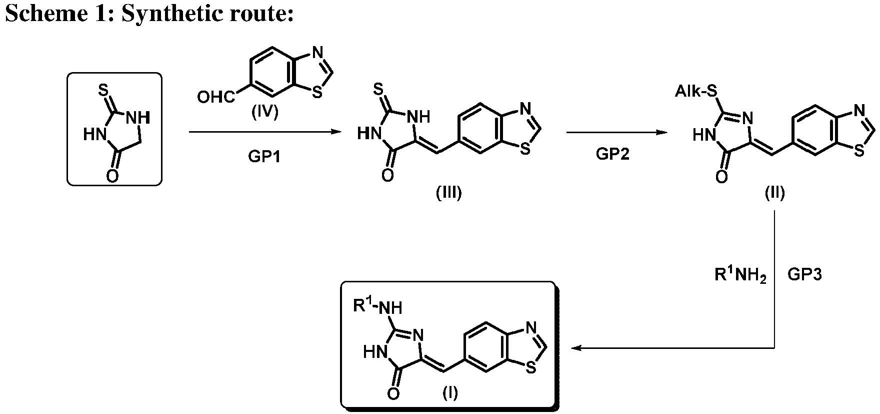

The synthesis of compounds according to the invention is based on a functionalization of a compound of formula (II) by an amine of formula R¹NH₂ wherein R¹ is as defined above, following the general protocol 3 (GP3), described herein after.

According to GP3, the compound of formula (II) may be placed in an aprotic solvent such as THF or dioxane, or a mixture of both. The amine of formula R¹NH₂ may be added, for example in a molar ratio ranging from 2 to 6, in particular of 4, with respect to the compound of formula (II). The reaction mixture may be placed in a sealed tube and may receive energy, for example from a heating block or from microwaves. Upon completion of the reaction, the mixture may be brought back to room temperature.

In an embodiment named GP3-A, the reaction mixture may be stirred at a temperature ranging from -10 °C to 10 °C, for example at 0 °C, for a duration ranging from 30 minutes to 2 hours, for example for 1 hour. Depending on the state of the product obtained (solid, precipitate), purification methods well-known by the person skilled in the art may be performed, for example filtering, washing, triturating, drying *in vacuo*, flash chromatography, precipitating and refluxing.

In an embodiment named GP3-B, wherein the product failed to precipitate, the reaction mixture may be concentrated, in particular *in vacuo*, and purified, in particular by flash chromatography. A second purification step may be performed, in particular selected from reprecipitation, trituration and recrystallization.

In another embodiment named GP3-C, wherein the product failed to precipitate, the reaction mixture may be concentrated, in particular *in vacuo*, and the resulting crude may be purified by trituration in a protic polar solvent such as ethanol. Said trituration may be performed at a temperature comprised between 20 and 100°C, in particular at room temperature. A second purification step may be performed, in particular selected from reprecipitation, trituration and recrystallization.

A compound of formula (II) as defined above may be obtained by the *S*-alkylation of a compound of formula (III) wherein R¹ is as defined above and Alk is a (C₁-C₅)alkyl.

According to the general protocol GP2, a compound of formula (III) may be placed in a polar aprotic solvent such as dimethylformamide (DMF). An alkylhalide of formula Alk-Hal wherein Hal is an halide such as iodine or bromide may then be added dropwise, for example in a molar ratio ranging from 0.75 to 1.50, in particular of 1.05, with respect to the compound of formula (III), in presence of an inorganic base such as K2CO3, for example in a molar ratio ranging from 0.7 to 1.5, in particular of 1, still with respect to the compound of formula (III). The reaction mixture may be stirred during the addition of the alkylhalide.

In a particular embodiment, the resulting mixture may then be stirred, for example from 8 to 16 hours, in particular for 12 hours at room temperature.

In another embodiment, the resulting mixture may be stirred, for example from 2 to 8 hours, in particular for 6 hours, at a temperature ranging from -10 °C to 10 °C, in particular at 0 °C.

A compound of formula (III) as defined above may be obtained from a compound of formula (IV) wherein R¹ is as defined above, following the general protocol GP1.

According to GP1, the compound of formula (IV) may be placed in a protic solvent such as ethanol in presence of 2-thiohydantoin, for example in a molar ratio ranging from 0.85 to 1.15, in particular of 1, with respect to the compound of formula (IV), in presence of an organic base such as piperidine in a molar ratio ranging from 0.85 to 1.15, in particular of 1, still with respect to the compound of formula (II), in presence of an organic acid such as acetic acid for example in a molar ratio ranging from 0.85 to 1.15, in particular of 1, still with respect to the compound of formula (IV). The reaction mixture may be placed in a sealed tube, stirred and heated at a temperature ranging for example from 60 °C to 130 °C, in particular from 80 °C to a duration ranging from 10 to 100 minutes, in particular from 15 to 90 minutes. The reaction mixture may be irradiated, for example by microwaves.

Accordingly, the present invention further relates to the synthesis process for manufacturing new compounds of formula (I) as defined above, comprising at least a step of substituting a compound of formula (II) with a primary amine. The present invention relates to a synthesis process for manufacturing a compound of formula (I) as defined above or any of its pharmaceutically acceptable salt or any of the compounds (1) to (154) as defined above or any of their pharmaceutically acceptable salts, comprising at least a step of coupling a compound of formula (II) below 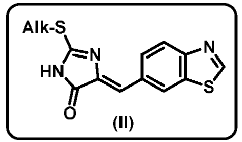 wherein Alk is a (C₁-C₅)alkyl,
with an amine of formula R¹NH₂ wherein R¹ is as defined above.

The present invention further relates to a synthetic intermediate of formula (II) below 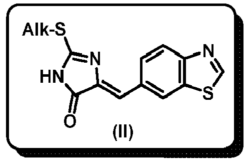 wherein Alk is a (C₁-C₅)alkyl, in particular Alk is selected from the group consisting of an ethyl and a methyl.

The chemical structures, the analytical and spectroscopic data of some compounds of formula (I) of the invention are illustrated respectively in the following Table 2 and Table 3.

Reactions were performed using oven-dried glassware under inert atmosphere of argon. Unless otherwise noted, all reagent-grade chemicals and solvents were obtained from commercial suppliers and were used as received. Reactions were monitored by thin-layer chromatography with silica gel 60 F254 pre-coated aluminium plates (0.25 mm). Visualization was performed under UV light and 254 or 312 nm, or with appropriate TLC stains including, but not limited to: phosphomolybdic acid, KMnO₄, ninhydrin, CAM, vanillin, *p*-anisaldehyde.

Microwave experiments were conducted in an Anton Paar Monowave 400® microwave reactor. The experiments were conducted in a monomode cavity with a power delivery ranging from 0 to 850 W, allowing pressurized reactions (0 to 30 bars) to be performed in sealed glass vials (4 to 30 mL) equipped with snap caps and silicon septa. The temperature (0 to 300 °C) was monitored by a contactless infrared sensor and calibrated with a ruby thermometer. Temperature, pressure, and power profiles were edited and monitored through a touch-screen control panel. Times indicated in the various protocols are the times measured when the mixtures reached the programmed temperature after a ramp period of 3 min.

Chromatographic purifications of compounds were achieved on an automated Interchim Puriflash XS420 equipped with 30 µm spherical silica-filled prepacked columns as stationary phase.

Some compounds of the invention are described with their structure in the below Table 2, which is merely illustrative and does not limit the scope of the present invention.

**Table 2: Structure of compounds (1) to (154). Formulas and molecular weights were generated using Perkin Elmer's Chemdraw® version 17.1.0.105.**

| | | | | | |
|---|---|---|---|---|---|
| 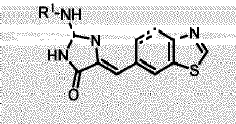 | | | | | |

| **Cpd N°** | **R¹-NH-** | **Class** | **Formula** | **MW** | **Stereochemistry** |
|---|---|---|---|---|---|
| **1** | 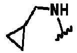 | A1 | C₁₅H₁₄N₄OS | 298.36 | N/A |
| **2** | 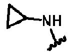 | A1 | C₁₄H₁₂N₄OS | 284.34 | N/A |
| **3** | 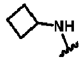 | A1 | C₁₅H₁₄N₄OS | 298.36 | N/A |
| **4** | 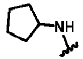 | A1 | C₁₆H₁₆N₄OS | 312.39 | N/A |
| **5** | 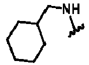 | A1 | C₁₈H₂₀N₄OS | 340.45 | N/A |
| **6** | 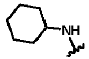 | A1 | C₁₇H₁₈N₄OS | 326.42 | N/A |
| **7** | 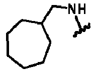 | A1 | C₁₉H₂₂N₄OS | 354.47 | N/A |
| **8** | 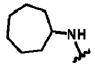 | A1 | C₁₈H₂₀N₄OS | 340.45 | N/A |
| **9** | 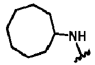 | A1 | C₁₉H₂₂N₄OS | 354.47 | N/A |
| **10** | 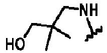 | A1 | C₁₆H₁₈N₄O₂S | 330.41 | N/A |
| **11** | 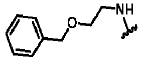 | A1 | C₂₀H₁₈N₄O₂S | 378.45 | N/A |
| **12** | 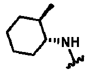 | A1 | C₁₈H₂₀N₄OS | 340.45 | *trans* - rac |
| **13** | 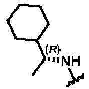 | A1 | C₁₉H₂₂N₄OS | 354.47 | (1*R*) |
| **14** | 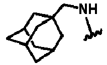 | A1 | C₂₂H₂₄N₄OS | 392.17 | N/A |
| **15** | 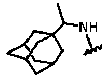 | A1 | C₂₃H₂₆N₄OS | 406.55 | rac |
| **16** | 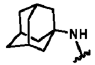 | A1 | C₂₁H₂₂N₄OS | 378.49 | N/A |
| **17** | 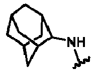 | A1 | C₂₁H₂₂N₄OS | 378.49 | N/A |
| **18** | 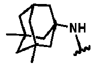 | A1 | C₂₃H₂₆N₄OS | 406.55 | N/A |
| **19** | 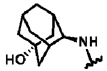 | A1 | C₂₁H₂₂N₄O₂S | 394.49 | *trans* |
| **20** | 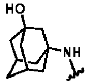 | A1 | C₂₁H₂₂N₄O₂S | 394.49 | N/A |
| **21** | 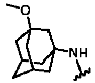 | A1 | C₂₂H₂₄N₄O₂S | 408.52 | N/A |
| **22** | 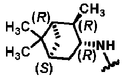 | A1 | C₂₁H₂₄N₄OS | 380.51 | (1*R*,2*R*,*3R*,*5S*) |
| **23** | 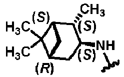 | A1 | C₂₁H₂₄N₄OS | 380.51 | (1*S*,2*S*,*3S*,*5R*) |
| **24** | 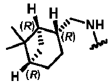 | A1 | C₂₁H₂₄N₄OS | 380.51 | (1*R*,2*R*,5*R*) |
| **25** | 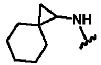 | A1 | C₁₉H₂₀N₄OS | 352.46 | rac |
| **26** | 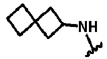 | A1 | C₁₈H₁₈N₄OS | 338.43 | N/A |
| **27** | 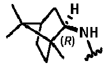 | A1 | C₂₁H₂₄N₄OS | 380.51 | (2*R*) |
| **28** | 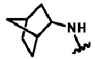 | A1 | C₁₈H₁₈N₄OS | 380.51 | rac |
| **29** | 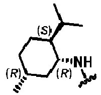 | A1 | C₂₁H₂₆N₄OS | 382.53 | (1*R*,2*S,5R*) |
| **30** | 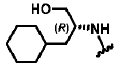 | A1 | C₂₀H₂₄N₄O₂S | 384.50 | (1*R*) |
| **31** | 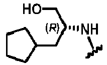 | A1 | C₁₉H₂₂N₄O₂S | 370.47 | (1*R*) |
| **32** | 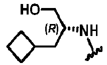 | A1 | C₁₈H₂₀N₄O₂S | 356.44 | (1*R*) |
| **33** | 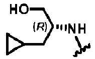 | A1 | C₁₇H₁₈N₄O₂S | 342.42 | (1*R*) |
| **34** | 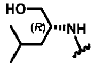 | A1 | C₁₇H₂₀N₄O₂S | 344.43 | (1*R*) |
| **35** | 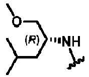 | A1 | C₁₈H₂₂N₄O₂S | 344.43 | (1*R*) |
| **36** | 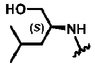 | A1 | C₁₇H₂₀N₄O₂S | 344.43 | (1*S*) |
| **37** | 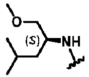 | A1 | C₁₈H₂₂N₄O₂S | 344.43 | (1*S*) |
| **38** | 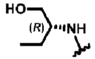 | A1 | C₁₅H₁₆N₄O₂S | 316.38 | (1*R*) |
| **39** | 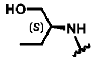 | A1 | C₁₅H₁₆N₄O₂S | 316.38 | (1*S*) |
| **40** | 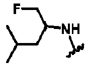 | A1 | C₁₇H₁₉FN₄OS | 346.42 | rac |
| **41** | 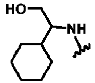 | A1 | C₁₉H₂₂N₄O₂S | 370.47 | rac |
| **42** | 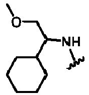 | A1 | C₂₀H₂₄N₄O₂S | 384.50 | rac |
| **43** | 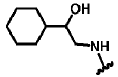 | A1 | C₁₉H₂₂N₄O₂S | 384.50 | rac |
| **44** | 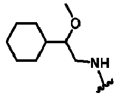 | A1 | C₂₀H₂₄N₄O₂S | 384.50 | rac |
| **45** | 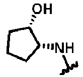 | A1 | C₁₆H₁₆N₄O₂S | 328.39 | *cis* - rac |
| **46** | 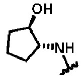 | A1 | C₁₆H₁₆N₄O₂S | 328.39 | *trans* - rac |
| **47** | 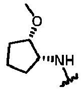 | A1 | C₁₇H₁₈N₄O₂S | 342.42 | *cis* - rac |
| **48** | 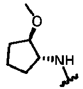 | A1 | C₁₇H₁₈N₄O₂S | 342.42 | *trans* - rac |
| **49** | 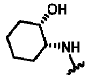 | A1 | C₁₇H₁₈N₄O₂S | 342.42 | *cis* - rac |
| **50** | 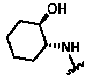 | A1 | C₁₇H₁₈N₄O₂S | 342.42 | *trans* - rac |
| **51** | 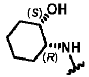 | A1 | C₁₇H₁₈N₄O₂S | 342.42 | (1*R*,2*S*) |
| **52** | 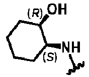 | A1 | C₁₇H₁₈N₄O₂S | 342.42 | (1*S*,2*R*) |
| **53** | 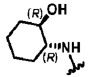 | A1 | C₁₇H₁₈N₄O₂S | 342.42 | (1*R*,2*R*) |
| **54** | 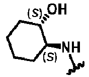 | A1 | C₁₇H₁₈N₄O₂S | 342.42 | (1S,2S) |
| **55** | 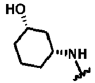 | A1 | C₁₇H₁₈N₄O₂S | 342.42 | *cis* - rac |
| **56** | 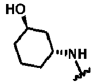 | A1 | C₁₇H₁₈N₄O₂S | 342.42 | *trans* - rac |
| **57** | 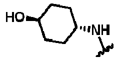 | A1 | C₁₇H₁₈N₄O₂S | 342.42 | *trans* - rac |
| **58** | 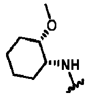 | A1 | C₁₈H₂₀N₄O₂S | 356.44 | *cis* - rac |
| **59** | 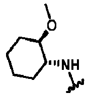 | A1 | C₁₈H₂₀N₄O₂S | 356.44 | *trans* - rac |
| **60** | 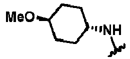 | A1 | C₁₈H₂₀N₄O₂S | 356.44 | *Trans* |
| **61** | 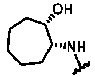 | A1 | C₁₈H₂₀N₄O₂S | 356.44 | *cis* - rac |
| **62** | 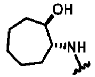 | A1 | C₁₈H₂₀N₄O₂S | 356.44 | *trans* - rac |
| **63** | 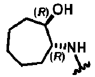 | A1 | C₁₈H₂₀N₄O₂S | 356.44 | (1*R*,2*R*) |
| **64** | 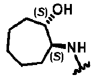 | A1 | C₁₈H₂₀N₄O₂S | 356.44 | (1*S*,2*S*) |
| **65** | 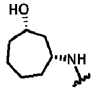 | A1 | C₁₈H₂₀N₄O₂S | 356.44 | *cis* - rac |
| **66** | 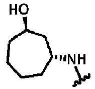 | A1 | C₁₈H₂₀N₄O₂S | 356.44 | *trans* - rac |
| **67** | 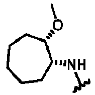 | A1 | C₁₉H₂₂N₄O₂S | 370.47 | *cis* - rac |
| **68** | 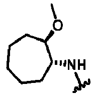 | A1 | C₁₉H₂₂N₄O₂S | 370.47 | *trans* - rac |
| **69** | 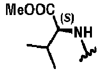 | A1 | C₁₇H₁₈N₄O₃S | 358.42 | (2*S*) |
| **70** | 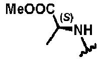 | A1 | C15H₁₄N₄O₃S | 330.36 | (2*S*) |
| **71** | 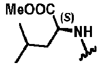 | A1 | C₁₈H₂₀N₄O₃S | 372.44 | (2*S*) |
| **72** | 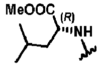 | A1 | C₁₈H₂₀N₄O₃S | 372.44 | (2*R*) |
| **73** | 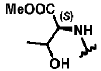 | A1 | C₁₆H₁₆N₄O₄S | 360.39 | (2*S*) |
| **74** | 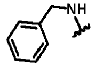 | A2 | C₁₈H₁₄N₄OS | 334.40 | N/A |
| **75** | 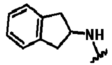 | A2 | C₂₀H₁₆N₄OS | 360.44 | N/A |
| **76** | 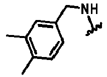 | A2 | C₂₀H₁₈N₄OS | 362.45 | N/A |
| **77** | 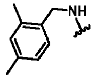 | A2 | C₂₀H₁₈N₄OS | 362.45 | N/A |
| **78** | 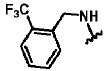 | A2 | C₁₉H₁₃F₃N₄OS | 402.40 | N/A |
| **79** | 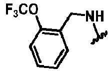 | A2 | C₁₉H₁₃F₃N₄O₂S | 418.39 | N/A |
| **80** | 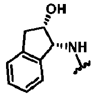 | A2 | C₂₀H₁₆N₄O₂S | 376.43 | *cis* - rac |
| **81** | 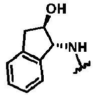 | A2 | C₂₀H₁₆N₄O₂S | 376.43 | *trans* - rac |
| **82** | 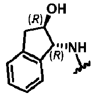 | A2 | C₂₀H₁₆N₄O₂S | 376.43 | (1*R*,2*R*) |
| **83** | 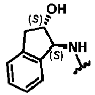 | A2 | C₂₀H₁₆N₄O₂S | 376.43 | (1*S*,2*S*) |
| **84** | 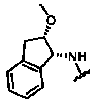 | A2 | C₂₁H₁₈N₄O₂S | 390.46 | *cis* - rac |
| **85** | 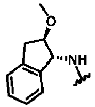 | A2 | C₂₁H₁₈N₄O₂S | 390.46 | *trans* - rac |
| **86** | 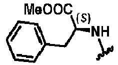 | A2 | C₂₁H₁₈N₄O₃S | 406.46 | (2*S*) |
| **87** | 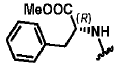 | A2 | C₂₁H₁₈N₄O₃S | 406.46 | (2*R*) |
| **88** | 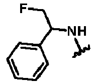 | A2 | C₁₉H₁₅FN₄OS | 366.41 | rac |
| **89** | 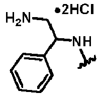 | A2 | C₁₉H₁₉Cl₂N₅OS | 436.36 | rac |
| **90** | 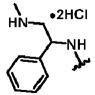 | A2 | C₂₀H₂₁Cl₂N₅OS | 450.38 | rac |
| **91** | 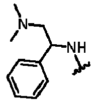 | A2 | C₂₁H₂₁N₅OS | 391.49 | rac |
| **92** | 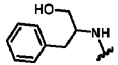 | A2 | C₂₀H₁₈N₄O₂S | 378.45 | rac |
| **93** | 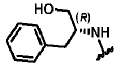 | A2 | C₂₀H₁₈N₄O₂S | 378.45 | (1*R*) |
| **94** | 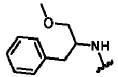 | A2 | C₂₁H₂₀N₄O₂S | 392.48 | rac |
| **95** | 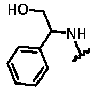 | A2 | C₁₉H₁₆N₄O₂S | 364.42 | rac |
| **96** | 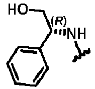 | A2 | C₁₉H₁₆N₄O₂S | 364.42 | (1*R*) |
| **97** | 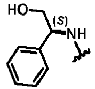 | A2 | C₁₉H₁₆N₄O₂S | 364.42 | (1*S*) |
| **98** | 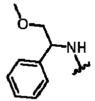 | A2 | C₂₀H₁₈N₄O₂S | 378.45 | rac |
| **99** | 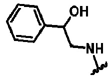 | A2 | C₁₉H₁₆N₄O₂S | 364.42 | rac |
| **100** | 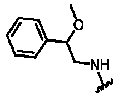 | A2 | C₂₀H₁₈N₄O₂S | 378.45 | rac |
| **101** | 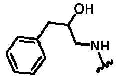 | A2 | C₂₀H₁₈N₄O₂S | 378.45 | rac |
| **102** | 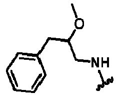 | A2 | C₂₁H₂₀N₄O₂S | 392.48 | rac |
| **103** | 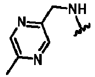 | A3 | C₁₇H₁₄N₆OS | 350.41 | N/A |
| **104** | 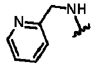 | A3 | C₁₇H₁₃N₅OS | 335.39 | N/A |
| **105** | 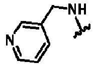 | A3 | C₁₇H₁₃N₅OS | 335.39 | N/A |
| **106** | 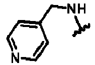 | A3 | C₁₇H₁₃N₅OS | 335.39 | N/A |
| **107** | 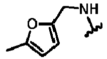 | A3 | C₁₇H₁₄N₄O₂S | 338.39 | N/A |
| **108** | 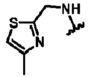 | A3 | C₁₆H₁₃N₅OS₂ | 355.43 | N/A |
| **109** | 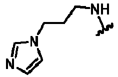 | A3 | C₁₇H₁₆N₆OS | 352.42 | N/A |
| **110** | 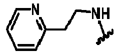 | A3 | C₁₈H₁₅N₅OS | 349.41 | N/A |
| **111** | 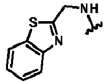 | A3 | C₁₉H₁₃N₅OS₂ | 391.47 | N/A |
| **112** | 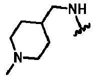 | A4 | C₁₈H₂₁N₅OS | 355.46 | N/A |
| **113** | 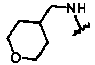 | A4 | C₁₇H₁₈N₄O₂S | 342.42 | N/A |
| **114** | 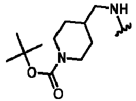 | A4 | C₂₂H₂₇N₅O₃S | 441.55 | N/A |
| **115** | 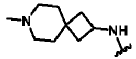 | A4 | C₂₀H₂₃N₅OS | 381.50 | N/A |
| **116** | 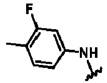 | A5 | C₁₈H₁₃FN₄OS | 352.39 | N/A |
| **117** | 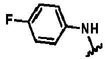 | A5 | C₁₇H₁₁FN₄OS | 338.37 | N/A |
| **118** | 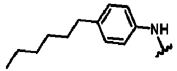 | A5 | C₂₃H₂₄N₄OS | 404.53 | N/A |
| **119** | 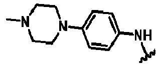 | A5 | C₂₂H₂₂N₆OS | 418.52 | N/A |
| **120** | 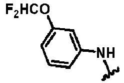 | A5 | C₁₈H₁₂F₂N₄O₂S | 386.37 | N/A |
| **121** | 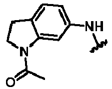 | A5 | C₂₁H₁₇N₅O₂S | 403.47 | N/A |
| **122** | 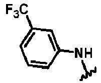 | A5 | C₁₈H₁₁F₃N₄OS | 388.37 | N/A |
| **123** | 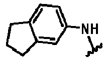 | A5 | C₂₀H₁₆N₄OS | 360.44 | N/A |
| **124** | 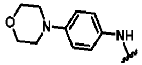 | A5 | C₂₁H₁₉N₅O₂S | 405.48 | N/A |
| **125** | 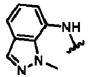 | A5 | C₁₉H₁₄N₆OS | 374.42 | N/A |
| **126** | 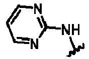 | A6 | C₁₅H₁₀N₆OS | 322.35 | N/A |
| **127** | 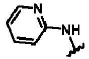 | A6 | C₁₆H₁₁N₅OS | 321.36 | N/A |
| **128** | 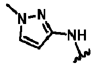 | A6 | C₁₅H₁₂N₆OS | 324.37 | N/A |
| **129** | 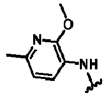 | A6 | C₁₈H₁₅N₅O₂S | 365.41 | N/A |
| **130** | 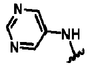 | A6 | C₁₅H₁₀N₆OS | 322.35 | N/A |
| **131** | 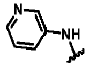 | A6 | C₁₆H₁₁N₅OS | 321.36 | N/A |
| **132** | 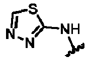 | A6 | C₁₃H₈N₆OS₂ | 328.37 | N/A |
| **133** | 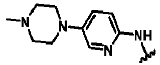 | A6 | C₂₁H₂₁N₇OS | 419.51 | N/A |
| **134** | 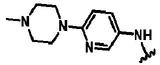 | A6 | C₂₁H₂₁N₇OS | 419.51 | N/A |
| **135** | 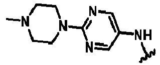 | A6 | C₂₀H₂₀N₈OS | 420.50 | N/A |
| **136** | 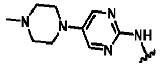 | A6 | C₂₀H₂₀N₈OS | 420.50 | N/A |
| **137** | 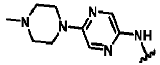 | A6 | C₂₀H₂₀N₈OS | 420.50 | N/A |
| **138** | 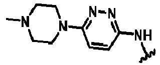 | A6 | C₂₀H₂₀N₈OS | 420.50 | N/A |
| **139** | 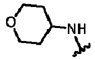 | A7 | C₁₆H₁₆N₄O₂S | 328.39 | N/A |
| **140** | 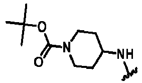 | A7 | C₂₁H₂₅N₅O₃S | 427.52 | N/A |
| **141** | 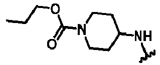 | A7 | C₁₉H₂₁N₅O₃S | 399.47 | N/A |
| **142** | 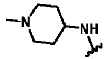 | A7 | C₁₇H₁₉N₅OS | 341.43 | N/A |
| **143** | 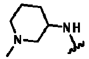 | A7 | C₁₇H₁₉N₅OS | 341.43 | rac |
| **144** | 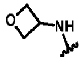 | A7 | C₁₄H₁₂N₄O₂S | 300.34 | N/A |
| **145** | 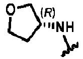 | A7 | C₁₅H₁₄N₄O₂S | 314.36 | (3*R*) |
| **146** | 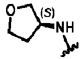 | A7 | C₁₅H₁₄N₄O₂S | 314.36 | (3*S*) |
| **147** | 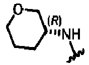 | A7 | C₁₆H₁₆N₄O₂S | 328.39 | (3*R*) |
| **148** | 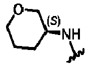 | A7 | C₁₆H₁₆N₄O₂S | 328.39 | (3*S*) |
| **149** | 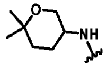 | A7 | C₁₈H₂₀N₄O₂S | 356.44 | rac |
| **150** | 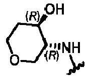 | A7 | C₁₆H₁₆N₄O₃S | 344.39 | (3*R*,4*R*) |
| **151** | 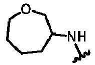 | A7 | C₁₇H₁₈N₄O₂S | 342.42 | rac |
| **152** | 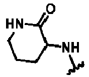 | A7 | C₁₆H₁₅N₅O₂S | 341.39 | rac |
| **153** | 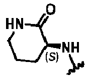 | A7 | C₁₆H₁₅N₅O₂S | 341.39 | (3*S*) |
| **154** | 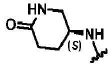 | A7 | C₁₆H₁₅N₅O₂S | 341.39 | (5*S*) |

The below Table 3 describes the analytical and spectroscopic data of the compounds introduced in Table 2.

¹H NMR analyses (400 or 500 MHz) and ¹³C NMR spectra (101 MHz) were recorded with a Bruker ULTRASHIELD 500 or 400 spectrometer. Processing and analyses of the spectra were performed with MestReNova. Data appear in the following order: chemical shifts in ppm which were referenced to the internal solvent signal, multiplicity, number of protons, and coupling constant *J* in Hertz.

Reversed-phase HPLC/MS analyses were carried out with a Waters Alliance 2795 HPLC equipped with an autosampler, an inline membrane degasser, a column oven (T° = 45 °C), a UV detector, and a ZQ quadrupole mass detector working in ionization electrospray mode. Compounds (0.1 to 0.3 mg) were solubilized in a minimum amount of DMSO completed with acetonitrile (Vₜₒₜₐₗ: 1 mL). Standard analytical parameters: flow rate: 1mL/min, Vᵢₙⱼ.: 5µL.
- Acidic conditions: Waters XSelect CSH C18 column (3.5µm, 2.1x 50 mm). Gradient: (H₂O + 0.04% v/v HCOOH (10mM))/ACN from 95/5 to 0/100 in 18.5 min.
- Alkaline conditions: Waters Xbridge C18 column (3.5µm, 2.1x 50 mm). Gradient: (H₂O + 0.06% v/v NH_{3(aq)} (10mM))/ACN from 95/5 to 0/100 in 18.5 min.

**Table 3. Spectroscopic and analytical characterization of compounds (1) to (154)**

| **Cpd N°** | **Structure** | **Formula** | **MW** | **HPLC** | **Description** |
|---|---|---|---|---|---|
| **1** | 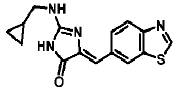 | C₁₅H₁₄N₄OS | 298.36 | >98% | Yellow solid. ¹H NMR (400 MHz, DMSO-*d*₆, 300K) of major tautomer δ_{H} 10.66 (bs, 1H, NH, D₂O exchanged), 9.35 (s, 1H), 8.80 (s, 1H), 8.39 - 8.13 (m, 1H), 8.03 (d, *J* = 8.6 Hz, 1H), 7.56 (bs, 1H, NH, D₂O exchanged), 6.40 (s, 1H), 3.32 - 3.10 (m, 2H), 1.18 - 1.02 (m, 1H), 0.57 - 0.41 (m, 2H), 0.38 - 0.23 (m, 2H). MS (ESI⁺) : [M+H]⁺ 299.2. |
| **2** | 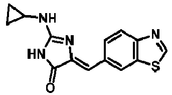 | C₁₄H₁₂N₄OS | 284.34 | >98% | Yellow solid. ¹H NMR (400 MHz, DMSO-*d*₆, 300K) of major tautomer δ_{H} 11.13 (bs, 1H, NH, D₂O exchanged), 9.35 (s, 1H), 8.83 (s, 1H), 8.26 (d, *J* = 8.6 Hz, 1H), 8.13 (bs, 1H, NH, D₂O exchanged), 8.03 (d, *J* = 8.6 Hz, 1H), 6.43 (s, 1H), 3.04 - 2.62 (m, 1H), 0.83 - 0.69 (m, 2H), 0.68 - 0.54 (m, 2H). MS (ESI⁺) : [M+H]⁺ 285.1. |
| **3** | 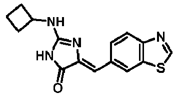 | C₁₅H₁₄N₄OS | 298.36 | >98% | Yellow solid. ¹H NMR (400 MHz, DMSO-*d*₆, 300K) of major tautomer δ_{H} 10.72 (bs, 1H, NH, D₂O exchanged), 9.36 (s, 1H), 8.79 (s, 1H), 8.34 - 8.22 (m, 1H), 8.04 (d, *J* = 8.6 Hz, 1H), 7.83 (bs, 1H, NH, D₂O exchanged), 6.40 (s, 1H), 4.74 - 4.02 (m, 1H), 2.38 - 2.20 (m, 2H), 2.17 - 1.97 (m, 2H), 1.69 (s, 2H). MS (ESI⁺) : [M+H]⁺ 299.2. |
| **4** | 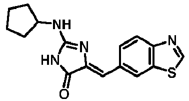 | C₁₆H₁₆N₄OS | 312.39 | >98% | Yellow solid. ¹H NMR (400 MHz, DMSO-*d*₆, 300K) of major tautomer δ_{H} 10.53 (bs, 1H, NH, D₂O exchanged), 9.36 (s, 1H), 8.76 (s, 1H), 8.22 (s, 1H), 8.04 (d, *J* = 8.0 Hz, 1H), 7.84 (bs, 1H, NH, D₂O exchanged), 6.41 (s, 1H), 4.43 - 4.05 (m, 1H), 2.04 - 1.92 (m, 2H), 1.76 - 1.67 (m, 2H), 1.56 (m, 2H), 1.40 (m, 2H). MS (ESI⁺) : [M+H]⁺ 313.2. |
| **5** | 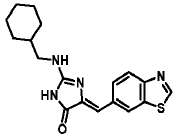 | C₁₈H₂₀N₄OS | 340.45 | 97% | Yellow solid. ¹H NMR (400 MHz, DMSO-*d*₆, 300K) of major tautomer δ_{H} 10.57 (bs, 1H, NH, D₂O exchanged), 9.35 (s, 1H), 8.85 (s, 1H), 8.23 (d, *J* = 8.5 Hz, 1H), 8.03 (d, *J* = 8.5 Hz, 1H), 7.50 (bs, 1H, NH, D₂O exchanged), 6.38 (s, 1H), 3.28 - 3.00 (m, 2H), 1.83 - 1.49 (m, 6H), 1.35 - 1.09 (m, 3H), 1.08 - 0.82 (m, 2H). MS (ESI⁺) : [M+H]⁺ 341.2. |
| **6** | 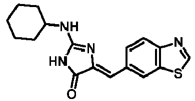 | C₁₇H₁₈N₄OS | 326.42 | >98% | Yellow solid. ¹H NMR (400 MHz, DMSO-*d*₆, 300K) of major tautomer δ_{H} 10.62 (bs, 1H, NH, D₂O exchanged), 9.35 (s, 1H), 8.90 - 8.65 (m, 1H), 8.37 - 8.13 (m, 1H), 8.03 (d, *J* = 8.4 Hz, 1H), 7.49 (bs, 1H, NH, D₂O exchanged), 6.39 (s, 1H), 3.97 - 3.46 (m, 1H), 2.07 - 1.80 (m, 2H), 1.80 - 1.65 (m, 2H), 1.65 - 1.53 (m, 1H), 1.49 - 1.05 (m, 5H). MS (ESI⁺) : [M+H]⁺ 327.2. |
| **7** | 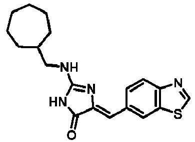 | C₁₉H₂₂N₄OS | 354.47 | >98% | Yellow solid. ¹H NMR (400 MHz, DMSO-*d*₆, 300K) of major tautomer δ_{H}. 10.61 (bs, 1H, NH, D₂O exchanged), 9.35 (s, 1H), 8.88 (s, 1H), 8.22 (d, *J* = 8.6 Hz, 1H), 8.03 (d, *J* = 8.5 Hz, 1H), 7.53 (bs, 1H, NH, D₂O exchanged), 6.39 (s, 1H), 3.29 - 3.02 (m, 2H), 1.95 - 1.36 (m, 11H), 1.33 - 1.12 (m, 2H). MS (ESI⁺) : [M+H]⁺ 355.2. |
| **8** | 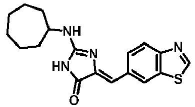 | C₁₈H₂₀N₄OS | 340.45 | >98% | Yellow solid. ¹H NMR (400 MHz, DMSO-*d*₆, 300K) of major tautomer δ_{H} 10.40 (bs, 1H, NH, D₂O exchanged), 9.35 (s, 1H), 9.05 - 8.69 (m, 1H), 8.42 - 8.13 (m, 1H), 8.03 (d, *J* = 8.5 Hz, 1H), 7.54 (bs, 1H, NH, D₂O exchanged), 6.39 (s, 1H), 3.98 (s, 1H), 1.93 (s, 2H), 1.78 - 1.35 (m, 10H). MS (ESI⁺) : [M+H]⁺ 341.2. |
| **9** | 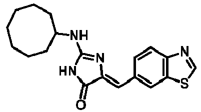 | C₁₉H₂₂N₄0S | 354.47 | >98% | Yellow solid. ¹H NMR (400 MHz, DMSO-*d*₆, 300K) of major tautomer δ_{H} 10.43 (bs, 1H, NH, D₂O exchanged), 9.35 (s, 1H), 8.98 (m, 1H), 8.18 (d, *J* = 8.6 Hz, 1H), 8.02 (d, *J* = 8.5 Hz, 1H), 7.56 (bs, 1H, NH, D₂O exchanged), 6.40 (s, 1H), 4.25-3.91 (m, 1H), 1.94-1.35 (m, 14H). MS (ESI⁺) : [M+H]⁺ 355.2. |
| **10** | 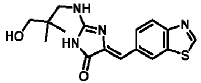 | C₁₆H₁₈NaO₂S | 330.41 | >98% | Yellow solid. ¹H NMR (400 MHz, DMSO-*d*₆, 300K) of major tautomer δ_{H} 10.59 (bs, 1H, NH, D₂O exchanged), 9.37 (s, 1H), 8.82 - 8.62 (m, 1H), 8.22 (d, *J* = 8.6 Hz, 1H), 8.04 (d, *J* = 8.6 Hz, 1H), 7.45 (bs, 1H, NH, D₂O exchanged), 6.43 (bs, 1H, OH, D₂O exchanged), 5.02 (s, 1H), 3.33 - 3.24 (m, 2H), 3.22 - 3.11 (m, 2H), 1.09 - 0.65 (m, 6H). MS (ESI⁺) : [M+H]⁺ 331.2. |
| **11** | 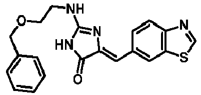 | C₂₀H_{I8}N₄O₂S | 378.45 | >98% | Yellow solid. ¹H NMR (500 MHz, DMSO-*d*₆, 300K) of major tautomer δ_{H} 10.62 (s, 1H, NH, D₂O exchanged), 9.35 (s, 1H), 8.82 (s, 1H), 8.39 - 8.25 (m, 1H), 8.03 (d, *J* = 8.5 Hz, 1H), 7.72 -7.42 (m, 1H), 7.40 -7.20 (m, 5H), 6.42 (s, 1H), 4.61 - 4.50 (m, 2H), 3.88 - 3.47 (m, 4H). MS (ESI⁺) : [M+H]⁺ 379.2. |
| **12** | 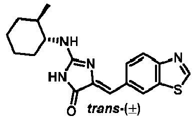 | C₁₈H₂₀N₄OS | 340.45 | >98% | Yellow solid. ¹H NMR (400 MHz, DMSO-*d*₆, 300K) of major tautomer δ_{H} 10.49 (bs, 1H, NH, D₂O exchanged), 9.34 (s, 1H), 8.92 - 8.69 (m, 1H), 8.27 - 8.15 (m, 1H), 8.02 (d, *J* = 8.6 Hz, 1H), 7.42 (bs, 1H, NH, D₂O exchanged), 6.36 (s, 1H), 3.65 - 3.34 (m, 1H), 3.23 - 2.84 (m, 1H), 2.01 - 1.13 (m, 8H), 0.98 - 0.84 (m, 3H). MS (ESI⁺) : [M+H]⁺ 341.2. |
| **13** | | C₁₉H₂₂N₄0S | 354.47 | >97% | Yellow solid. ¹H NMR (400 MHz, DMSO-*d*₆, 300K) of major tautomer δ_{H} 10.34 (bs, 1H, NH, D₂O exchanged), 9.34 (d, *J* = 1.4 Hz, 1H), 8.92 - 8.73 (m, 1H), 8.26 - 8.18 (m, 1H), 8.03 (d, *J* = 8.5 Hz, 1H), 7.37 (bs, 1H, NH, D₂O exchanged), 6.37 (s, 1H), 4.12 - 3.39 (m, 1H), 1.72 (d, *J* = 13.8 Hz, 4H), 1.65 - 1.57 (m, 1H), 1.46 (s, 1H), 1.26 - 0.96 (m, 8H). MS (ESI⁺) : [M+H]⁺ 355.2. |
| **14** | 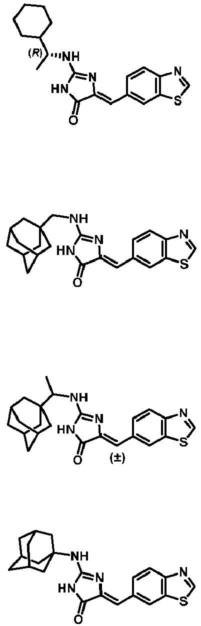 | C₂₂H₂₄N₄OS | 392.17 | >98% | Yellow solid. ¹H NMR (400 MHz, DMSO-*d*₆, 300K) of major tautomer δ_{H} 10.33 (bs, 1H, NH, D₂O exchanged), 9.35 (s, 1H), 8.85 (s, 1H), 8.35 - 8.14 (m, 1H), 8.03 (d, *J* = 8.5 Hz, 1H), 7.41 (bs, 1H, NH, D₂O exchanged), 6.38 (s, 1H), 3.25 - 2.80 (m, 2H), 1.96 (s, 3H), 1.72 - 1.43 (m, 12H). MS (ESI⁺) : [M+H]⁺ 393.3. |
| **15** | | C₂₃H₂₆N₄OS | 406.55 | >98% | Yellow solid. ¹H NMR (400 MHz, DMSO-*d*₆, 343K) of major tautomer δ_{H} 10.22 (bs, 1H, NH, D₂O exchanged), 9.30 (s, 1H), 8.82 (s, 1H), 8.22 (d, *J* = 8.8 Hz, 1H), 8.02 (d, *J* = 8.6 Hz, 1H), 7.14 (bs, 1H, NH, D₂O exchanged), 6.39 (s, 1H), 3.76 - 3.59 (m, 1H), 1.99 (s, 3H), 1.77 - 1.49 (m, 12H), 1.17 - 1.05 (m, 3H). MS (ESI⁺) : [M+H]⁺ 407.3. |
| **16** | | C₂₁H₂₂N₄OS | 378.49 | 95% | Yellow solid. ¹H NMR (400 MHz, DMSO-*d*₆, 300K) of major tautomer δ_{H} 9.99 (bs, 1H, D₂O exchanged), 9.35 (s, 1H), 9.02 (s, 1H), 8.17 - 8.13 (m, 1H), 8.03 (d, *J* = 8.5 Hz, 1H), 7.09 (bs, 1H, D₂O exchanged), 6.42 (s, 1H), 2.26 - 2.02 (m, 10H), 1.80 - 1.64 (m, 5H). MS (ESI⁺) : [M+H]⁺ 379.2. |
| **17** | 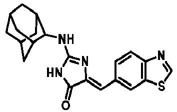 | C₂₁H₂₂N₄OS | 378.49 | 93% | Yellow solid. ¹H NMR (400 MHz, DMSO-*d*₆, 343K) of major tautomer δ_{H} 10.06 (bs, 1H, D₂O exchanged), 9.31 (s, 1H), 8.87 (s, 1H), 8.20 (d, *J* = 8.7 Hz, 1H), 8.01 (d, *J* = 8.5 Hz, 1H), 7.35 (bs, 1H, D₂O exchanged), 6.44 (s, 1H), 4.15 - 3.95 (m, 1H), 2.13 - 1.56 (m, 14H). MS (ESI⁺) : [M+H]⁺ 379.2. |
| **18** | 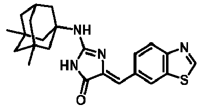 | C₂₃H₂₆N₄OS | 406.55 | 97% | Yellow solid. ¹H NMR (400 MHz, DMSO-*d*₆, 300K) of major tautomer δ_{H} 10.04 (bs, 1H, NH, D₂O exchanged), 9.35 (s, 1H), 9.17 (s, 1H), 8.02 (s, 2H), 7.16 (bs, 1H, NH, D₂O exchanged), 6.41 (s, 1H), 2.23 - 2.13 (m, 1H), 2.02 - 1.70 (m, 6H), 1.49 - 1.28 (m, 4H), 1.25 - 1.14 (m, 2H), 0.98 - 0.85 (m, 6H). MS (ESI⁺) : [M+H]⁺ 407.2. |
| **19** | 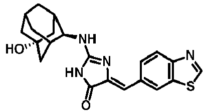 | C₂₁H₂₂N₄O₂S | 394.49 | >98% | Yellow solid. ¹H NMR (400 MHz, DMSO-*d*₆, 300K) of major tautomer δ_{H} 9.93 (bs, 1H, NH, D₂O exchanged), 9.36 (s, 1H), 8.98 - 8.76 (m, 1H), 8.37 - 8.11 (m, 1H), 8.11 - 7.97 (m, 1H), 7.48 (bs, 1H, NH, D₂O exchanged), 6.42 (s, 1H), 4.57 - 4.36 (bs, 1H, OH, D₂O exchanged), 4.20 - 3.78 (m, 1H), 2.25 - 2.00 (m, 3H), 1.97 - 1.74 (m, 4H), 1.72 - 1.60 (m, 4H), 1.49 - 1.32 (m, 2H). MS (ESI⁺) : [M+H]⁺ 395.2. |
| **20** | 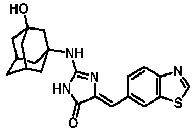 | C₂₁H₂₂N₄O₂S | 394.49 | 97% | Yellow solid. ¹H NMR (400 MHz, DMSO-*d*₆, 300K) of major tautomer δ_{H} 10.01 (bs, 1H, NH, D₂O exchanged), 9.35 (s, 1H), 9.00 (s, 1H), 8.15 (dd, *J* = 8.6, 1.6 Hz, 1H), 8.02 (d, *J* = 8.5 Hz, 1H), 7.17 (bs, 1H, NH, D₂O exchanged), 6.42 (s, 1H), 4.62 (bs, 1H, OH, D₂O exchanged), 2.28 - 2.17 (m, 2H), 2.12 - 2.01 (m, 5H), 1.99 - 1.91 (m, 1H), 1.69 - 1.45 (m, 6H). MS (ESI⁺) : [M+H]⁺ 395.2. |
| **21** | 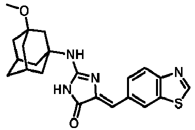 | C₂₂H₂₄N₄O₂S | 408.52 | 96% | Yellow solid. ¹H NMR (400 MHz, DMSO-*d*₆, 300K) of major tautomer δ_{H} 10.08 (bs, 1H, NH, D₂O exchanged), 9.35 (s, 1H), 9.04 (s, 1H), 8.12 (d, *J* = 8.6 Hz, 1H), 8.02 (d, *J* = 8.6 Hz, 1H), 7.27 (bs, 1H, NH, D₂O exchanged), 6.43 (s, 1H), 3.18 (s, 3H), 2.38 - 1.91 (m, 8H), 1.83 - 1.47 (m, 6H). MS (ESI⁺) : [M+H]⁺ 409.3. |
| **22** | 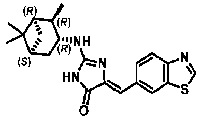 | C₂₁H₂₄N₄OS | 380.51 | 95% | Yellow solid. ¹H NMR (400 MHz, DMSO-*d*₆, 300K) of major tautomer δ_{H} 10.64 (bs, 1H, NH, D₂O exchanged), 9.34 (s, 1H), 9.12 - 8.81 (m, 1H), 8.17 (d, *J* = 8.6 Hz, 1H), 8.01 (d, *J* = 8.5 Hz, 1H), 7.71 (bs, 1H, NH, D₂O exchanged), 6.39 (s, 1H), 4.57 - 4.23 (m, 1H), 3.98 - 3.66 (m, 1H), 2.64 - 2.52 (m, 1H), 2.40 - 2.28 (m, 1H), 2.21 - 2.04 (m, 1H), 2.04 - 1.87 (m, 1H), 1.87 - 1.61 (m, 2H), 1.38 - 0.89 (m, 9H). MS (ESI⁺) : [M+H]⁺ 381.2. |
| **23** | 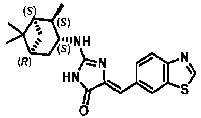 | C₂₁H₂₄N₄OS | 380.51 | 95% | Yellow solid. ¹H NMR (400 MHz, DMSO-*d*₆, 300K) of major tautomer δ_{H} 10.64 (bs, 1H, NH, D₂O exchanged), 9.34 (s, 1H), 9.12 - 8.81 (m, 1H), 8.17 (d, *J* = 8.6 Hz, 1H), 8.01 (d, *J* = 8.5 Hz, 1H), 7.71 (bs, 1H, NH, D₂O exchanged), 6.39 (s, 1H), 4.57 - 4.23 (m, 1H), 3.98 - 3.66 (m, 1H), 2.64 - 2.52 (m, 1H), 2.40 - 2.28 (m, 1H), 2.21 - 2.04 (m, 1H), 2.04 - 1.87 (m, 1H), 1.87 - 1.61 (m, 2H), 1.38 - 0.89 (m, 9H). MS (ESI⁺) : [M+H]⁺ 381.2. |
| **24** | 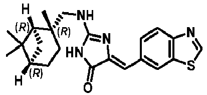 | C₂₁H₂₄N₄OS | 380.51 | 94% | Yellow solid. ¹H NMR (400 MHz, DMSO-*d*₆, 343K) of major tautomer 10.51 (bs, 1H, NH, D₂O exchanged), 9.30 (s, 1H), 8.95 (s, 1H), 8.14 (d, *J* = 8.7 Hz, 1H), 7.99 (d, *J* = 8.5 Hz, 1H), 7.36 (bs, 1H, NH, D₂O exchanged), 6.40 (s, 1H), 3.54 - 3.35 (m, 2H), 2.42 - 2.29 (m, 2H), 2.09 - 1.80 (m, 5H), 1.72 - 1.58 (m, 1H), 1.20 (s, 6H), 0.92 (d, *J* = 9.5 Hz, 1H). MS (ESI⁺) : [M+H]⁺ 381.2. |
| **25** | 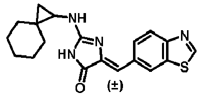 | C₁₉H₂₀N₄OS | 352.46 | >98% | Yellow solid. ¹H NMR (400 MHz, DMSO-*d*₆, 300K) of major tautomer δ_{H} 10.68 (bs, 1H, NH, D₂O exchanged), 9.35 (s, 1H), 9.14-8.80 (m, 1H), 8.16 (d, *J* = 8.6 Hz, 1H), 8.01 (d, *J* = 8.5 Hz, 1H), 7.67 (bs, 1H, NH, D₂O exchanged), 6.43 (s, 1H), 2.72 - 2.58 (m, 1H), 1.92 - 1.20 (m, 10H), 0.71 (dd, *J* = 7.9, 5.1 Hz, 1H), 0.55 (t, *J* = 4.7 Hz, 1H). MS (ESI⁺) : [M+H]⁺ 353.2. |
| **26** | 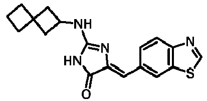 | C₁₈H₁₈N₄OS | 338.43 | >98% | Yellow solid. ¹H NMR (400 MHz, DMSO-*d*₆, 300K) of major tautomer δ_{H} 10.71 (bs, 1H, NH, D₂O exchanged), 9.35 (s, 1H), 8.80 (s, 1H), 8.32 - 8.17 (m, 1H), 8.03 (d, *J* = 8.6 Hz, 1H), 7.75 (bs, 1H, NH, D₂O exchanged), 6.39 (s, 1H), 4.61 - 3.67 (m, 2H), 2.45 - 2.32 (m, 2H), 2.16 - 1.99 (m, 3H), 1.99 - 1.88 (m, 2H), 1.87 - 1.74 (m, 2H). MS (ESI⁺) : [M+H]⁺ 339.2. |
| **27** | 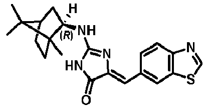 | C₂₁H₂₄N₄OS | 380.51 | 96% | Yellow solid. ¹H NMR (400 MHz, DMSO-*d*₆, 300K) of major tautomer δ_{H} 10.19 (bs, 1H, NH, D₂O exchanged), 9.34 (s, 1H), 8.92 (s, 1H), 8.19 (dd, *J* = 8.7, 1.6 Hz, 1H), 8.01 (d, *J* = 8.6 Hz, 1H), 7.58 (bs, 1H, NH, D₂O exchanged), 6.38 (s, 1H), 4.50 - 3.70 (m, 1H), 2.39 - 2.17 (m, 1H), 1.67 (s, 3H), 1.42 -1.20 (m, 2H), 1.14-0.61 (m, 10H). MS (ESI⁺) : [M+H]⁺ 381.2. |
| **28** | 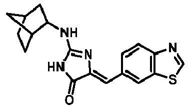 | C₁₈H₁₈N₄OS | 338.43 | >98% | Yellow solid. ¹H NMR (400 MHz, DMSO-*d*₆, 300K) of major tautomer δ_{H} 10.30 (bs, 1H, NH, D₂O exchanged), 9.35 (s, 1H), 8.97 - 8.71 (m, 1H), 8.40 - 8.20 (m, 1H), 8.04 (d, *J* = 8.6 Hz, 1H), 7.65 (bs, 1H, NH, D₂O exchanged), 6.40 (s, 1H), 4.30-3.71 (m, 1H), 2.47 - 2.34 (m, 1H), 2.27 - 2.12 (m, 1H), 2.08 - 1.86 (m, 1H), 1.74 - 1.25 (m, 6H), 1.18 - 1.02 (m, 1H). MS (ESI⁺) : [M+H]⁺ 339.2 |
| **29** | 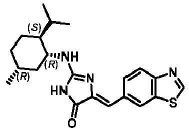 | C₂₁H₂₆N₄OS | 382.53 | 91% | Yellow solid. ¹H NMR (400 MHz, DMSO-*d*₆, 300K) of major tautomer δ_{H} 10.52 (bs, 1H, NH, D₂O exchanged), 9.34 (s, 1H), 8.84 (s, 1H), 8.27 - 8.15 (m, 1H), 8.01 (d, *J* = 8.6 Hz, 1H), 7.37 (bs, 1H, NH, D₂O exchanged), 6.36 (s, 1H), 3.97 - 3.66 (m, 1H), 2.11 - 1.20 (m, 7H), 1.19 - 0.58 (m, 11H) MS (ESI⁺) : [M+H]⁺ 383.3. |
| **30** | 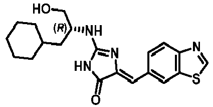 | C₂₀H₂₄N₄O₂S | 384.50 | 96% | Yellow solid. ¹H NMR (400 MHz, DMSO-*d*₆, 373K) of major tautomer δ_{H} 10.07 (bs, 1H, NH, D₂O exchanged), 9.28 (s, 1H), 9.03 - 8.63 (m, 1H), 8.35 - 8.04 (m, 1H), 8.04 - 7.90 (m, 1H), 6.90 (bs, 1H, NH, D₂O exchanged), 6.42 (s, 1H), 4.54 (bs, 1H, OH, D₂O exchanged), 4.14 - 3.93 (m, 1H), 3.66 - 3.44 (m, 2H), 1.95 - 0.90 (m, 13H). MS (ESI⁺) : [M+H]⁺ 385.2. |
| **31** | 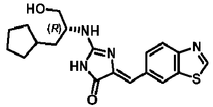 | C₁₉H₂₂N₄O₂S | 370.47 | >98% | Yellow solid. ¹H NMR (400 MHz, DMSO-*d*₆, 373K) of major tautomer δ_{H} 10.11 (bs, 1H, NH, D₂O exchanged), 9.28 (s, 1H), 9.03 - 8.85 (m, 1H), 8.28 - 8.09 (m, 1H), 8.07 - 7.95 (m, 1H), 6.91 (bs, 1H, NH, D₂O exchanged), 6.43 (s, 1H), 4.55 (bs, 1H, OH, D₂O exchanged), 4.08 - 3.86 (m, 1H), 3.66 - 3.44 (m, 2H), 1.99 - 1.08 (m, 11H). MS (ESI⁺) : [M+H]⁺ 371.2. |
| **32** | 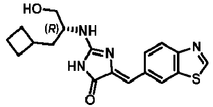 | C₁₈H₂₀N₄O₂S | 356.44 | >98% | Yellow solid. ¹H NMR (400 MHz, DMSO-*d*₆, 300K) of major tautomer δ_{H} 10.38 (bs, 1H, NH, D₂O exchanged), 9.35 (s, 1H), 9.03 - 8.72 (m, 1H), 8.19 (d, *J* = 8.7 Hz, 1H), 8.01 (d, *J* = 8.5 Hz, 1H), 7.22 (bs, 1H, NH, D₂O exchanged), 6.40 (s, 1H), 4.86 (bs, 1H, OH, D₂O exchanged), 4.14 - 3.75 (m, 1H), 3.71 - 3.39 (m, 2H), 2.44 - 2.30 (m, 1H), 2.18 - 1.92 (m, 2H), 1.88 - 1.45 (m, 6H). MS (ESI⁺) : [M+H]⁺ 357.2. |
| **33** | 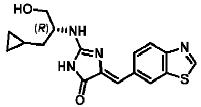 | C₁₇H₁₈N₄O₂S | 342.2 | >98% | Yellow solid. ¹H NMR (400 MHz, DMSO-*d*₆, 323K) of major tautomer δ_{H} 10.37 (bs, 1H, NH, D₂O exchanged), 9.33 (s, 1H), 8.98 - 8.72 (m, 1H), 8.34 - 8.12 (m, 1H), 8.02 (d, *J* = 8.5 Hz, 1H), 7.23 (bs, 1H, NH, D₂O exchanged), 6.41 (s, 1H), 4.77 (bs, 1H, OH, D₂O exchanged), 4.11 - 3.87 (m, 1H), 3.70 - 3.44 (m, 2H), 1.69 - 1.51 (m, 1H), 1.51 - 1.40 (m, 1H), 0.87 - 0.71 (m, 1H), 0.52 - 0.37 (m, 2H), 0.25 - 0.03 (m, 2H). MS (ESI⁺) : [M+H]⁺ 343.1. |
| **34** | 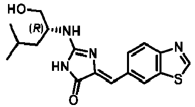 | C₁₇H₂₀N₄O₂S | 344.43 | >98% | Yellow solid. ¹H NMR (400 MHz, DMSO-*d*₆, 300K) of major tautomer δ_{H} 10.42 (bs, 1H, NH, D₂O exchanged), 9.35 (s, 1H), 8.92 (s, 1H), 8.19 (d, *J* = 8.7 Hz, 1H), 8.01 (d, *J* = 8.6 Hz, 1H), 7.26 (bs, 1H, NH, D₂O exchanged), 6.40 (s, 1H), 4.87 (bs, 1H, OH, D₂O exchanged), 4.34 - 4.04 (m, 1H), 3.62 - 3.42 (m, 2H), 1.86 - 1.57 (m, 1H), 1.60 - 1.34 (m, 2H), 1.15 - 0.77 (m, 6H). MS (ESI⁺) : [M+H]⁺ 345.2. |
| **35** | 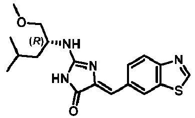 | C₁₈H₂₂N₄O₂S | 358.46 | >98% | Yellow solid. ¹H NMR (400 MHz, DMSO-*d*₆, 373K) of major tautomer δ_{H} 10.16 (bs, 1H, NH, D₂O exchanged), 9.27 (s, 1H), 8.89 (s, 1H), 8.15 (d, *J* = 8.6 Hz, 1H), 7.99 (d, *J* = 8.4 Hz, 1H), 7.01 (bs, 1H, NH, D₂O exchanged), 6.43 (s, 1H), 4.19 (s, 1H), 3.53 - 3.41 (m, 2H), 3.34 (s, 3H), 1.83 - 1.66 (m, 1H), 1.62 - 1.39 (m, 2H), 1.14-0.81 (m, 6H). MS (ESI⁺) : [M+H]⁺ 359.2. |
| **36** | 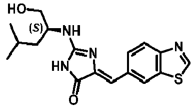 | C₁₇H₂₀N₄O₂S | 344.43 | >98% | Yellow solid. ¹H NMR (400 MHz, DMSO-*d*₆, 300K) of major tautomer δ_{H} 10.42 (bs, 1H, NH, D₂O exchanged), 9.35 (s, 1H), 8.92 (s, 1H), 8.19 (d, *J* = 8.7 Hz, 1H), 8.01 (d, *J* = 8.6 Hz, 1H), 7.26 (bs, 1H, NH, D₂O exchanged), 6.40 (s, 1H), 4.87 (bs, 1H, OH, D₂O exchanged), 4.34 - 4.04 (m, 1H), 3.62 - 3.42 (m, 2H), 1.86 - 1.57 (m, 1H), 1.60 - 1.34 (m, 2H), 1.15 - 0.77 (m, 6H). MS (ESI⁺) : [M+H]⁺ 345.2. |
| **37** | 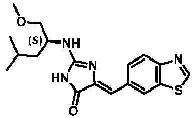 | C₁₈H₂₂N₄O₂S | 358.46 | >98% | Yellow solid. ¹H NMR (400 MHz, DMSO-*d*₆, 373K) of major tautomer δ_{H} 10.16 (bs, 1H, NH, D₂O exchanged), 9.27 (s, 1H), 8.89 (s, 1H), 8.15 (d, *J* = 8.6 Hz, 1H), 7.99 (d, *J* = 8.4 Hz, 1H), 7.01 (bs, 1H, NH, D₂O exchanged), 6.43 (s, 1H), 4.19 (s, 1H), 3.53 - 3.41 (m, 2H), 3.34 (s, 3H), 1.83 - 1.66 (m, 1H), 1.62 - 1.39 (m, 2H), 1.14-0.81 (m, 6H). MS (ESI⁺) : [M+H]⁺ 359.2. |
| **38** | 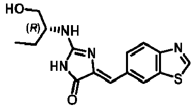 | C₁₅H₁₆N₄O₂S | 316.38 | 88% | Yellow solid. ¹H NMR (400 MHz, DMSO-*d*₆, 333K) of major tautomer δ_{H} 10.41 (bs, 1H, NH, D₂O exchanged), 9.32 (s, 1H), 8.81 (s, 1H), 8.23 (d, *J* = 8.7 Hz, 1H), 8.02 (d, *J* = 8.7 Hz, 1H), 7.19 (bs, 1H, NH, D₂O exchanged), 6.41 (s, 1H), 4.89 - 4.62 (m, 1H, OH, D₂O exchanged), 3.81 (s, 1H), 3.53 (s, 2H), 1.76 - 1.62 (m, 1H), 1.62 - 1.45 (m, 1H), 1.00 - 0.87 (m, 3H). MS (ESI⁺) : [M+H]⁺ 317.5. |
| **39** | 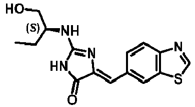 | C₁₅H₁₆N₄O₂S | 316.38 | >96% | Yellow solid. ¹H NMR (400 MHz, DMSO-*d*₆, 333K) of major tautomer δ_{H} 10.41 (bs, 1H, NH, D₂O exchanged), 9.32 (s, 1H), 8.81 (s, 1H), 8.23 (d, *J* = 8.7 Hz, 1H), 8.02 (d, *J* = 8.7 Hz, 1H), 7.19 (bs, 1H, NH, D₂O exchanged), 6.41 (s, 1H), 4.86 (bs, 1H, OH, D₂O exchanged), 3.81 (s, 1H), 3.53 (s, 2H), 1.76 - 1.62 (m, 1H), 1.62 - 1.45 (m, 1H), 1.00 - 0.87 (m, 3H). MS (ESI⁺) : [M+H]⁺ 317.5. |
| **40** | 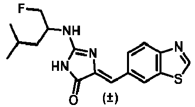 | C₁₇H₁₉FN₄OS | 346.42 | 91% | Yellow solid. ¹H NMR (400 MHz, DMSO-*d*₆, 373K) of major tautomer δ_{H} 10.28 (bs, 1H, NH, D₂O exchanged), 9.28 (s, 1H), 8.85 (s, 1H), 8.17 (d, *J* = 8.2 Hz, 1H), 8.00 (d, *J* = 8.5 Hz, 1H), 7.25 (bs, 1H, NH, D₂O exchanged), 6.46 (s, 1H), 4.64 - 4.52 (m, 1H), 4.50 - 4.40 (m, 1H), 4.37 - 4.22 (m, 1H), 1.84 - 1.71 (m, 1H), 1.65 - 1.55 (m, 1H), 1.52 - 1.41 (m, 1H), 1.04 - 0.81 (m, 6H). MS (ESI⁺) : [M+H]⁺ 347.2. |
| **41** | 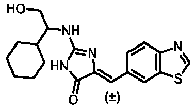 | C₁₉H₂₂N₄O₂S | 370.47 | >98% | Yellow solid. ¹H NMR (400 MHz, DMSO-*d*₆, 343K) of major tautomer δ_{H} 10.26 (bs, 1H, NH, D₂O exchanged), 9.31 (s, 1H), 8.84 (s, 1H), 8.20 (d, *J* = 8.7 Hz, 1H), 8.02 (d, *J* = 8.6 Hz, 1H), 7.11 (bs, 1H, NH, D₂O exchanged), 6.41 (s, 1H), 4.81 - 4.55 (m, 1H, OH, D₂O exchanged), 3.91 - 3.69 (m, 1H), 3.67 - 3.50 (m, 2H), 1.90 - 1.53 (m, 6H), 1.34 - 0.98 (m, 5H). MS (ESI⁺) : [M+H]⁺ 371.2. |
| **42** | 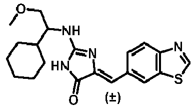 | C₂₀H₂₄N₄O₂S | 384.0 | >98% | Yellow solid. ¹H NMR (400 MHz, DMSO-*d*₆) of major tautomer δ_{H} 10.27 (bs, 1H, NH, D₂O exchanged), 9.38 - 9.24 (m, 1H), 8.94 - 8.72 (m, 1H), 8.39 - 8.12 (m, 1H), 8.12 - 7.96 (m, 1H), 7.24 (bs, 1H, NH, D₂O exchanged), 6.50 - 6.25 (m, 1H), 4.03 - 3.83 (m, 1H), 3.60 - 3.44 (m, 2H), 3.31 (s, 3H), 1.86 - 1.53 (m, 6H), 1.30 - 1.03 (m, 5H). MS (ESI⁺) : [M+H]⁺ 385.2. |
| **43** | 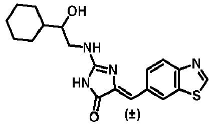 | C₁₉H₂₂N₄O₂S | 370.47 | >98% | Yellow solid. ¹H NMR (400 MHz, DMSO-*d*₆, 298K) of major tautomer δ_{H} 10.54 (bs, 1H, NH, D₂O exchanged), 9.35 (s, 1H), 8.94 (s, 1H), 8.29 - 8.09 (m, 1H), 8.01 (d, *J* = 8.6 Hz, 1H), 7.42 (bs, 1H, NH, D₂O exchanged), 6.41 (s, 1H), 4.95 - 4.72 (bs, 1H, OH, D₂O exchanged), 3.82 - 3.36 (m, 2H), 3.25 - 3.08 (m, 1H), 2.02 - 1.50 (m, 5H), 1.45 - 0.91 (m, 6H). MS (ESI⁺) : [M+H]⁺ 371.3. |
| **44** | 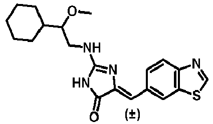 | C₂₀H₂₄N₄O₂S | 384.50 | 97% | Yellow solid. ¹H NMR (400 MHz, DMSO-*d*₆, 323K) of major tautomer δ_{H} 10.46 (bs, 1H, NH, D₂O exchanged), 9.33 (s, 1H), 8.96 (s, 1H), 8.15 (d, *J* = 8.7 Hz, 1H), 8.01 (d, *J* = 8.5 Hz, 1H), 7.42 (bs, 1H, NH, D₂O exchanged), 6.43 (s, 1H), 3.71 - 3.55 (m, 1H), 3.45 - 3.33 (m, 4H), 3.32 - 3.23 (m, 1H), 1.90 - 1.50 (m, 6H), 1.28 - 1.07 (m, 5H). MS (ESI⁺) : [M+H]⁺ 385.2. |
| **45** | 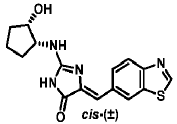 | C₁₆H₁₆N₄O₂S | 328.39 | >98% | Yellow solid. ¹H NMR (400 MHz, DMSO-*d*₆, 373K) of major tautomer δ_{H} 10.13 (bs, 1H, NH, D₂O exchanged), 9.28 (s, 1H), 8.87 - 8.67 (m, 1H), 8.41 - 8.13 (m, 1H), 8.09 - 7.94 (m, 1H), 6.73 (bs, 1H, NH, D₂O exchanged), 6.44 (s, 1H), 4.73 (bs, 1H, OH, D₂O exchanged), 4.22 - 3.96 (m, 2H), 2.11 - 1.51 (m, 6H). MS (ESI⁺) : [M+H]⁺ 329.2. |
| **46** | 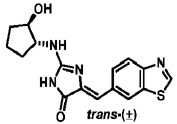 | C₁₆H₁₆N₄O₂S | 328.39 | >98% | Yellow solid. ¹H NMR (400 MHz, DMSO-*d*₆, 323K) of major tautomer δ_{H} 10.53 (bs, 1H, NH, D₂O exchanged), 9.33 (s, 1H), 8.93 - 8.63 (m, 1H), 8.35 - 8.12 (m, 1H), 8.03 (d, *J* = 8.3 Hz, 1H), 7.61 (bs, 1H, NH, D₂O exchanged), 6.42 (s, 1H), 4.94 (bs, 1H, OH, D₂O exchanged), 4.10 - 3.75 (m, 2H), 2.18 - 2.03 (m, 1H), 1.98 - 1.82 (m, 1H), 1.79 - 1.42 (m, 4H). MS (ESI⁺) : [M+H]⁺ 329.2. |
| **47** | 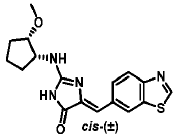 | C₁₇H₁₈N₄O₂S | 342.42 | 98% | Yellow solid. ¹H NMR (400 MHz, DMSO-*d*₆, 343K) of major tautomer δ_{H} 10.18 (bs, 1H, NH, D₂O exchanged), 9.31 (s, 1H), 8.79 (s, 1H), 8.44 - 8.15 (m, 1H), 8.15 -7.97 (m, 1H), 6.96 (bs, 1H, NH, D₂O exchanged), 6.44 (s, 1H), 4.24 (s, 1H), 3.81 (s, 1H), 3.30 (s, 3H), 2.13 - 1.90 (m, 1H), 1.89 - 1.50 (m, 5H). MS (ESI⁺) : [M+H]⁺ 343.2. |
| **48** | 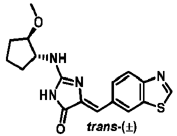 | C₁₇H₁₈N₄O₂S | 342.42 | 97% | Yellow solid. ¹H NMR (400 MHz, DMSO-*d*₆, 343K) of major tautomer δ_{H} 10.12 (bs, 1H, NH, D₂O exchanged), 9.28 (s, 1H), 8.97 - 8.76 (m, 1H), 8.25 - 8.09 (m, 1H), 8.01 (d, *J* = 8.3 Hz, 1H), 7.28 (bs, 1H, NH, D₂O exchanged), 6.46 (s, 1H), 4.21 - 4.12 (m, 1H), 3.86 - 3.79 (m, 1H), 3.38 (s, 3H), 2.17 - 2.04 (m, 1H), 2.00 -1.88 (m, 1H), 1.80 -1.58 (m, 4H). MS (ESI⁺) : [M+H]⁺ 343.2. |
| **49** | 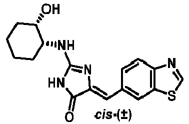 | C₁₇H₁₈N₄O₂S | 342.42 | >98% | Yellow solid. ¹H NMR (400 MHz, DMSO-*d*₆, 343K) of major tautomer δ_{H} 10.18 (bs, 1H, NH, D₂O exchanged), 9.31 (s, 1H), 8.80 (s, 1H), 8.22 (d, *J* = 8.3 Hz, 1H), 8.02 (d, *J* = 8.6 Hz, 1H), 6.86 (bs, 1H, NH, D₂O exchanged), 6.42 (s, 1H), 4.77 - 4.63 (m, 1H, OH, D₂O exchanged), 4.02 - 3.74 (m, 2H), 1.88 - 1.47 (m, 6H), 1.46 - 1.25 (m, 2H). MS (ESI⁺) : [M+H]⁺ 343.2. |
| **50** | 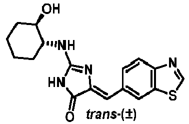 | C₁₇H₁₈N₄O₂S | 342.42 | >98% | Yellow solid. ¹H NMR (400 MHz, DMSO-*d*₆, 343K) of major tautomer δ_{H} 10.45 (bs, 1H, NH, D₂O exchanged), 9.30 (s, 1H), 8.83 (s, 1H), 8.31 - 8.09 (m, 1H), 8.09 - 7.96 (m, 1H), 7.35 (bs, 1H, NH, D₂O exchanged), 6.39 (s, 1H), 4.64 (bs, 1H, OH, D₂O exchanged), 3.59 - 3.31 (m, 2H), 2.13 - 1.84 (m, 2H), 1.78 - 1.58 (m, 2H), 1.48 - 1.11 (m, 4H). MS (ESI⁺) : [M+H]⁺ 343.2. |
| **51** | 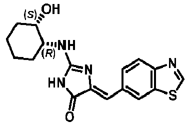 | C₁₇H₁₈N₄O₂S | 342.42 | >98% | Yellow solid. ¹H NMR (400 MHz, DMSO-*d*₆, 343K) of major tautomer δ_{H} 10.18 (bs, 1H, NH, D₂O exchanged), 9.31 (s, 1H), 8.80 (s, 1H), 8.22 (d, *J* = 8.3 Hz, 1H), 8.02 (d, *J* = 8.6 Hz, 1H), 6.86 (bs, 1H, NH, D₂O exchanged), 6.42 (s, 1H), 4.77 - 4.63 (m, 1H, OH, D₂O exchanged), 4.02 - 3.74 (m, 2H), 1.88 - 1.47 (m, 6H), 1.46 - 1.25 (m, 2H). MS (ESI⁺) : [M+H]⁺ 343.2. |
| **52** | 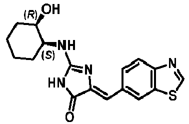 | C₁₇H₁₈N₄O₂S | 342.42 | >98% | Yellow solid. ¹H NMR (400 MHz, DMSO-*d*₆, 343K) of major tautomer δ_{H} 10.18 (bs, 1H, NH, D₂O exchanged), 9.31 (s, 1H), 8.80 (s, 1H), 8.22 (d, *J* = 8.3 Hz, 1H), 8.02 (d, *J* = 8.6 Hz, 1H), 6.86 (bs, 1H, NH, D₂O exchanged), 6.42 (s, 1H), 4.77 - 4.63 (m, 1H, OH, D₂O exchanged), 4.02 - 3.74 (m, 2H), 1.88 - 1.47 (m, 6H), 1.46 - 1.25 (m, 2H). MS (ESI⁺) : [M+H]⁺ 343.2. |
| 53 | 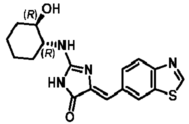 | C₁₇H₁₈N₄O₂S | 342.42 | 97% | Yellow solid. ¹H NMR (400 MHz, DMSO-*d*₆, 343K) of major tautomer δ_{H} 10.45 (bs, 1H, NH, D₂O exchanged), 9.30 (s, 1H), 8.83 (s, 1H), 8.31 - 8.09 (m, 1H), 8.09 - 7.96 (m, 1H), 7.35 (bs, 1H, NH, D₂O exchanged), 6.39 (s, 1H), 4.64 (bs, 1H, OH, D₂O exchanged), 3.59 - 3.31 (m, 2H), 2.13 - 1.84 (m, 2H), 1.78 - 1.58 (m, 2H), 1.48 - 1.11 (m, 4H). MS (ESI⁺) : [M+H]⁺ 343.2. |
| 54 | 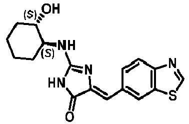 | C₁₇H₁₈N₄O₂S | 342.42 | >98% | Yellow solid. ¹H NMR (400 MHz, DMSO-*d*₆, 343K) of major tautomer δ_{H} 10.45 (bs, 1H, NH, D₂O exchanged), 9.30 (s, 1H), 8.83 (s, 1H), 8.31 - 8.09 (m, 1H), 8.09 - 7.96 (m, 1H), 7.35 (bs, 1H, NH, D₂O exchanged), 6.39 (s, 1H), 4.64 (bs, 1H, OH, D₂O exchanged), 3.59 - 3.31 (m, 2H), 2.13 - 1.84 (m, 2H), 1.78 - 1.58 (m, 2H), 1.48 - 1.11 (m, 4H). MS (ESI⁺) : [M+H]⁺ 343.2. |
| 55 | 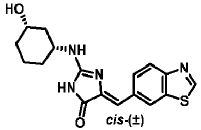 | C₁₇H₁₈N₄O₂S | 342.42 | >98% | Yellow solid. ¹H NMR (400 MHz, DMSO-*d*₆, 373K) of major tautomer δ_{H} 10.24 (bs, 1H, NH, D₂O exchanged), 9.28 (s, 1H), 8.91 - 8.53 (m, 1H), 8.31 - 8.05 (m, 1H), 8.02 (d, *J* = 8.2 Hz, 1H), 7.24 (bs, 1H, NH, D₂O exchanged), 6.42 (s, 1H), 4.39 (bs, 1H, OH, D₂O exchanged), 3.88 - 3.75 (m, 1H), 3.66 - 3.53 (m, 1H), 2.21 - 2.05 (m, 1H), 1.94 - 1.69 (m, 3H), 1.41 - 1.14 (m, 4H). MS (ESI⁺) : [M+H]⁺ 343.2. |
| 56 | 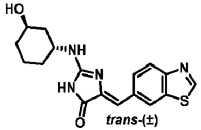 | C₁₇H₁₈N₄O₂S | 342.42 | >98% | Yellow solid. ¹H NMR (400 MHz, DMSO-*d*₆, 323K) of major tautomer δ_{H} 10.34 (bs, 1H, NH, D₂O exchanged), 9.33 (s, 1H), 8.97 - 8.55 (m, 1H), 8.37 - 8.09 (m, 1H), 8.03 (d, *J* = 8.4 Hz, 1H), 7.40 (bs, 1H, NH, D₂O exchanged), 6.40 (s, 1H), 4.53 - 4.32 (bs, 1H, OH, D₂O exchanged), 4.24 - 4.02 (m, 1H), 4.01 - 3.91 (m, 1H), 1.90 - 1.60 (m, 4H), 1.59 - 1.34 (m, 4H). MS (ESI⁺) : [M+H]⁺ 343.1. |
| **57** | 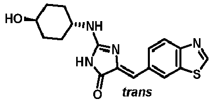 | C₁₇H₁₈N₄O₂S | 342.42 | >98% | Yellow solid. ¹H NMR (400 MHz, DMSO-*d*₆, 343K) δ_{H} 10.34 (bs, 1H, NH, D₂O exchanged), 9.31 (s, 1H), 9.05 - 8.54 (m, 1H), 8.41 - 8.08 (m, 1H), 8.02 (d, *J* = 8.4 Hz, 1H), 7.36 (bs, 1H, NH, D₂O exchanged), 6.41 (s, 1H), 4.36 (d, *J* = 4.4 Hz, 1H, OH, D₂O exchanged), 3.79 - 3.55 (m, 1H), 3.52 - 3.34 (m, 1H), 2.12 - 1.76 (m, 4H), 1.55 - 1.20 (m, 4H). MS (ESI⁺) : [M+H]⁺ 343.2. |
| **58** | 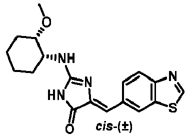 | C₁₈H₂₀N₄O₂S | 356.44 | 98% | Yellow solid. ¹H NMR (400 MHz, DMSO-*d*₆, 343K) of major tautomer δ_{H} 10.06 (bs, 1H, NH, D₂O exchanged), 9.31 (s, 1H), 8.81 (s, 1H), 8.23 (d, *J* = 8.1 Hz, 1H), 8.02 (d, *J* = 8.9 Hz, 1H), 6.96 (bs, 1H, NH, D₂O exchanged), 6.44 (s, 1H), 4.15 - 3.83 (m, 1H), 3.59 - 3.44 (m, 1H), 3.32 (s, 3H), 2.00 - 1.88 (m, 1H), 1.81 - 1.57 (m, 3H), 1.57 - 1.30 (m, 4H). MS (ESI⁺) : [M+H]⁺ 357.2. |
| **59** | 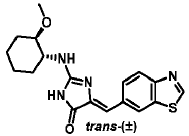 | C₁₈H₂₀N₄O₂S | 356.44 | >98% | Yellow solid. ¹H NMR (400 MHz, DMSO-*d*₆, 323K) of major tautomer δ_{H} 10.62 (bs, 1H, NH, D₂O exchanged), 9.32 (s, 1H), 8.84 (s, 1H), 8.20 (d, *J* = 8.7 Hz, 1H), 8.01 (d, *J* = 8.6 Hz, 1H), 7.65 (bs, 1H, NH, D₂O exchanged), 6.39 (s, 1H), 3.82 - 3.53 (m, 1H), 3.31 (s, 3H), 3.25 - 3.12 (m, 1H), 2.15 - 1.90 (m, 2H), 1.76 - 1.59 (m, 2H), 1.47 - 1.15 (m, 4H). MS (ESI⁺) : [M+H]⁺ 357.2. |
| **60** | 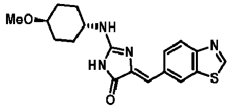 | C₁₈H₂₀N₄O₂S | 356.44 | >98% | Yellow solid. ¹H NMR (400 MHz, DMSO-*d*₆, 343K) of major tautomer δ_{H} 10.51 (bs, 1H, NH, D₂O exchanged), 9.30 (s, 1H), 8.83 (s, 1H), 8.20 (d, *J* = 8.6 Hz, 1H), 8.02 (d, *J* = 8.6 Hz, 1H), 7.41 (bs, 1H, NH, D₂O exchanged), 6.41 (s, 1H), 3.82 - 3.62 (m, 1H), 3.27 (s, 3H), 3.22 - 3.12 (m, 1H), 2.10 - 1.93 (m, 4H), 1.50 - 1.36 (m, 2H), 1.36 - 1.22 (m, 2H). MS (ESI⁺) : [M+H]⁺ 357.2. |
| **61** | 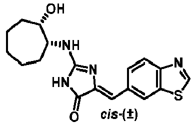 | C₁₈H₂₀N₄O₂S | 356.44 | >98% | Yellow solid. ¹H NMR (400 MHz, DMSO-*d*₆, 300K) of major tautomer δ_{H} 10.15 (bs, 1H, NH, D₂O exchanged), 9.35 (s, 1H), 8.90 (s, 1H), 8.22 (d, *J* = 9.0 Hz, 1H), 8.02 (d, *J* = 8.5 Hz, 1H), 7.07 (bs, 1H, NH, D₂O exchanged), 6.42 (s, 1H), 4.96 (bs, 1H, OH, D₂O exchanged), 4.08 - 3.77 (m, 2H), 1.98 - 1.29 (m, 10H). MS (ESI⁺) : [M+H]⁺ 357.2. |
| **62** | 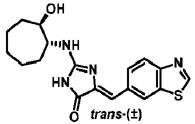 | C₁₈H₂₀N₄O₂S | 356.44 | >98% | Yellow solid. ¹H NMR (400 MHz, DMSO-*d*₆, 323K) of major tautomer δ_{H} 10.46 (bs, 1H, NH, D₂O exchanged), 9.33 (s, 1H), 9.08 - 8.72 (m, 1H), 8.39 - 8.09 (m, 1H), 8.02 (d, *J* = 8.5 Hz, 1H), 7.45 (bs, 1H, NH, D₂O exchanged), 6.40 (s, 1H), 4.76 (bs, 1H, OH, D₂O exchanged), 3.89 - 3.53 (m, 2H), 2.03 - 1.37 (m, 10H). MS (ESI⁺) : [M+H]⁺ 357.2. |
| **63** | 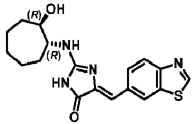 | C₁₈H₂₀N₄O₂S | 356.44 | >98% | Yellow solid. ¹H NMR (400 MHz, DMSO-*d*₆, 323K) of major tautomer δ_{H} 10.46 (bs, 1H, NH, D₂O exchanged), 9.33 (s, 1H), 9.08 - 8.72 (m, 1H), 8.39 - 8.09 (m, 1H), 8.02 (d, *J* = 8.5 Hz, 1H), 7.45 (bs, 1H, NH, D₂O exchanged), 6.40 (s, 1H), 4.76 (bs, 1H, OH, D₂O exchanged), 3.89 - 3.53 (m, 2H), 2.03 - 1.37 (m, 10H). MS (ESI⁺) : [M+H]⁺ 357.2. |
| **64** | 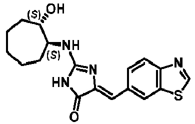 | C₁₈H₂₀N₄O₂S | 356.44 | 97% | Yellow solid. ¹H NMR (400 MHz, DMSO-*d*₆, 323K) of major tautomer δ_{H} 10.46 (bs, 1H, NH, D₂O exchanged), 9.33 (s, 1H), 9.08 - 8.72 (m, 1H), 8.39 - 8.09 (m, 1H), 8.02 (d, *J* = 8.5 Hz, 1H), 7.45 (bs, 1H, NH, D₂O exchanged), 6.40 (s, 1H), 4.76 (bs, 1H, OH, D₂O exchanged), 3.89 - 3.53 (m, 2H), 2.03 - 1.37 (m, 10H). MS (ESI⁺) : [M+H]⁺ 357.4. |
| **65** | 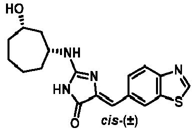 | C₁₈H₂₀N₄O₂S | 356.44 | >98% | Yellow solid. ¹H NMR (400 MHz, DMSO-*d*₆, 373K) of major tautomer δ_{H} 10.15 (bs, 1H, NH, D₂O exchanged), 9.28 (s, 1H), 8.99 - 8.54 (m, 1H), 8.28 - 8.06 (m, 1H), 8.02 (d, *J* = 7.8 Hz, 1H), 7.17 (bs, 1H, NH, D₂O exchanged), 6.43 (s, 1H), 4.23 (bs, 1H, OH, D₂O exchanged), 4.05 - 3.91 (m, 1H), 3.90 - 3.77 (m, 1H), 2.21 - 2.05 (m, 1H), 2.05 - 1.39 (m, 9H). MS (ESI⁺) : [M+H]⁺ 357.2. |
| **66** | 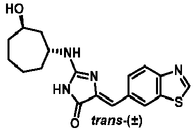 | C₁₈H₂₀N₄O₂S | 356.44 | >98% | Yellow solid. ¹H NMR (400 MHz, DMSO-*d*₆, 323K) of major tautomer δ_{H} 10.33 (bs, 1H, NH, D₂O exchanged), 9.33 (s, 1H), 8.99 - 8.65 (m, 1H), 8.34 - 8.07 (m, 1H), 8.02 (d, *J* = 8.4 Hz, 1H), 7.51 (bs, 1H, NH, D₂O exchanged), 6.41 (s, 1H), 4.47 (bs, 1H, OH, D₂O exchanged), 4.19 - 4.01 (m, 1H), 3.96 - 3.83 (m, 1H), 2.08 - 1.89 (m, 3H), 1.86 - 1.30 (m, 7H). MS (ESI⁺) : [M+H]⁺ 357.2. |
| **67** | 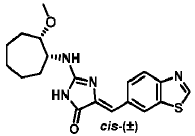 | C₁₉H₂₂N₄O₂S | 370.47 | >98% | Yellow solid. ¹H NMR (400 MHz, DMSO-*d*₆, 300K) of major tautomer δ_{H} 10.04 (bs, 1H, NH, D₂O exchanged), 9.35 (s, 1H), 8.89 (s, 1H), 8.23 (d, *J* = 9.0 Hz, 1H), 8.02 (d, *J* = 8.5 Hz, 1H), 7.21 (bs, 1H, NH, D₂O exchanged), 6.43 (s, 1H), 4.25 - 3.85 (m, 1H), 3.74 - 3.49 (m, 1H), 3.31 (s, 3H), 2.03 - 1.28 (m, 10H). MS (ESI⁺) : [M+H]⁺ 371.2. |
| **68** | 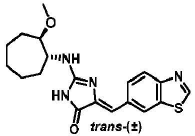 | C₁₉H₂₂N₄O₂S | 370.47 | 98% | Yellow solid. ¹H NMR (400 MHz, DMSO-*d*₆, 343K) of major tautomer δ_{H} 10.39 (bs, 1H, NH, D₂O exchanged), 9.30 (s, 1H), 8.90 (s, 1H), 8.18 (d, *J* = 8.6 Hz, 1H), 8.01 (d, *J* = 8.6 Hz, 1H), 7.47 (bs, 1H, NH, D₂O exchanged), 6.41 (s, 1H), 4.00 - 3.83 (m, 1H), 3.51 - 3.38 (m, 1H), 3.31 (s, 3H), 1.93 - 1.40 (m, 10H). MS (ESI⁺) : [M+H]⁺ 371.2. |
| **69** | 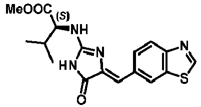 | C₁₇H₁₈N₄O₃S | 358.42 | 97% | Yellow solid. ¹H NMR (400 MHz, DMSO-*d*₆, 300K) of major tautomer δ_{H} 10.41 (bs, 1H, NH, D₂O exchanged), 9.38 (s, 1H), 8.83 (s, 1H), 8.39 - 7.86 (m, 2H + NH, D₂O exchanged), 6.48 (s, 1H), 4.50 - 4.26 (m, 1H), 3.73 (s, 3H), 2.29 - 2.16 (m, 1H), 1.16 - 0.80 (m, 6H). MS (ESI⁺) : [M+H]⁺ 359.2. |
| **70** | 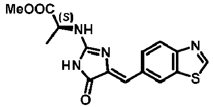 | C₁₅H₁₄N₄O₃S | 330.36 | 97% | Yellow solid. ¹H NMR (400 MHz, DMSO-*d*₆, 300K) of major tautomer δ_{H} 10.85 (bs, 1H, NH, D₂O exchanged), 9.37 (s, 1H), 8.83 (s, 1H), 8.19 (d, *J* = 8.5 Hz, 1H), 8.14 (bs, 1H, NH, D₂O exchanged ed), 8.03 (d, *J* = 8.6 Hz, 1H), 6.47 (s, 1H), 4.68 - 4.45 (m, 1H), 3.71 (s, 3H), 1.45 (d, *J* = 7.2 Hz, 3H). MS (ESI⁺) : [M+H]⁺ 331.1. |
| **71** | 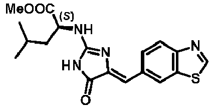 | C₁₈H₂₀N₄O₃S | 372.44 | 91% | Yellow solid. ¹H NMR (400 MHz, DMSO-*d*₆, 300K) of major tautomer δ_{H} 10.75 (bs, 1H, NH, D₂O exchanged), 9.37 (s, 1H), 8.85 (s, 1H), 8.19 (d, *J* = 8.6 Hz, 1H), 8.09 (bs, 1H, NH, D₂O exchanged), 8.03 (d, *J* = 8.6 Hz, 1H), 6.47 (s, 1H), 4.70 - 4.41 (m, 1H), 3.71 (s, 3H), 1.84 - 1.57 (m, 3H), 1.07 - 0.77 (m, 6H). MS (ESI⁺) : [M+H]⁺ 373.2. |
| **72** | 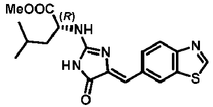 | C₁₈H₂₀N₄O₃S | 372.44 | 98% | Yellow solid. ¹H NMR (400 MHz, DMSO-*d*₆, 300K) of major tautomer δ_{H} 10.75 (bs, 1H, NH, D₂O exchanged), 9.37 (s, 1H), 8.85 (s, 1H), 8.19 (d, *J* = 8.6 Hz, 1H), 8.09 (bs, 1H, NH, D₂O exchanged), 8.03 (d, *J* = 8.6 Hz, 1H), 6.47 (s, 1H), 4.70 - 4.41 (m, 1H), 3.71 (s, 3H), 1.84 - 1.57 (m, 3H), 1.07 - 0.77 (m, 6H). MS (ESI⁺) : [M+H]⁺ 373.2. |
| **73** | 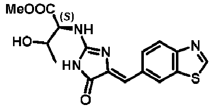 | C₁₆H₁₆N₄O₄S | 360.4 | >98% | Yellow solid. ¹H NMR (400 MHz, DMSO-*d*₆, 300K) of major tautomer δ_{H} 10.28 (bs, 1H, NH, D₂O exchanged), 9.37 (s, 1H), 8.78 (s, 1H), 8.21 (d, *J* = 8.5 Hz, 1H), 8.03 (d, *J* = 8.6 Hz, 1H), 7.58 - 7.34 (bs, 1H, NH, D₂O exchanged), 6.49 (s, 1H), 5.31 (bs, 1H, OH, D₂O exchanged), 4.66 - 4.49 (m, 1H), 4.35 - 4.16 (m, 1H), 3.72 (s, 3H), 1.20 (d, *J* = 6.3 Hz, 3H). MS (ESI⁺) : [M+H]⁺ 361.2. |
| **74** | 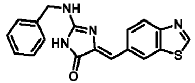 | C₁₈H₁₄N₄OS | 334.40 | 97% | Yellow solid. ¹H NMR (400 MHz, DMSO-*d*₆, 300K) of major tautomer δ_{H} 10.86 (bs, 1H, NH, D₂O exchanged), 9.36 (s, 1H), 8.84 (s, 1H), 8.26 - 8.20 (m, 1H), 8.19 - 8.06 (bs, 1H, NH, D₂O exchanged), 8.03 (d, *J* = 8.6 Hz, 1H), 7.55 - 7.20 (m, 5H), 6.43 (s, 1H), 4.67 - 4.48 (m, 2H). MS (ESI⁺) : [M+H]⁺ 335.2. |
| **75** | 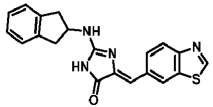 | C₂₀H₁₆N₄OS | 360.44 | >98% | Yellow solid. ¹H NMR (500 MHz, DMSO-*d*₆, 300K) of major tautomer δ_{H} 10.55 (bs, 1H, NH, D₂O exchanged), 9.35 (s, 1H), 8.81 (s, 1H), 8.44 - 8.17 (m, 1H), 8.03 (d, *J* = 8.5 Hz, 1H), 7.92 (bs, 1H, NH, D₂O exchanged), 7.32 - 7.22 (m, 2H), 7.20 - 7.17 (m, 2H), 6.44 (s, 1H), 4.96 - 4.44 (m, 1H), 3.38 - 3.31 (m, 2H), 3.09 - 2.88 (m, 2H). MS (ESI⁺) : [M+H]⁺ 361.1. |
| **76** | 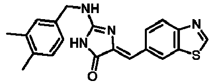 | C₂₀H₁₈N₄OS | 362.45 | >98% | Yellow solid. ¹H NMR (500 MHz, DMSO-*d*₆, 300K) of major tautomer δ_{H} 10.79 (bs, 1H, NH, D₂O exchanged), 9.36 (s, 1H), 8.86 (s, 1H), 8.24 (d, *J* = 8.6 Hz, 1H), 8.03 (d, *J* = 8.5 Hz, 1H), 7.99 (bs, 1H, NH, D₂O exchanged), 7.28 - 7.06 (m, 3H), 6.42 (s, 1H), 4.50 (s, 2H), 2.21 (s, 3H), 2.18 (s, 3H) MS (ESI⁺) : [M+H]⁺ 363.2. |
| **77** | 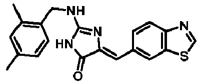 | C₂₀H₁₈N₄OS | 362.45 | >98% | Yellow solid. ¹H NMR (500 MHz, DMSO-*d*₆, 300K) of major tautomer δ_{H} 10.69 (bs, 1H, NH, D₂O exchanged), 9.36 (s, 1H), 8.88 (s, 1H), 8.22 (d, *J* = 7.9 Hz, 1H), 8.15 (bs, 1H, NH, D₂O exchanged), 8.03 (d, *J* = 8.0 Hz, 1H), 7.29 - 7.17 (m, 1H), 7.14 - 7.03 (m, 1H), 7.03 - 6.89 (m, 1H), 6.43 (s, 1H), 4.54 (s, 2H), 2.36 (s, 3H), 2.25 (s, 3H). MS (ESI⁺) : [M+H]⁺ 363.2. |
| **78** | 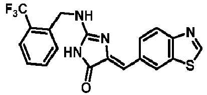 | C₁₉H₁₃F₃N₄OS | 402.40 | >98% | Yellow solid. ¹H NMR (500 MHz, DMSO-*d*₆, 300K) of major tautomer δ_{H} 10.96 (bs, 1H, NH, D₂O exchanged), 9.34 (s, 1H), 8.87 (s, 1H), 8.23 (bs, 1H, NH, D₂O exchanged), 8.10 - 7.93 (m, 2H), 7.77 (d, *J* = 7.8 Hz, 1H), 7.74 - 7.62 (m, 2H), 7.55 - 7.43 (m, 1H), 6.44 (s, 1H), 4.80 (s, 2H) MS (ESI⁺) : [M+H]⁺ 403.1. |
| **79** | 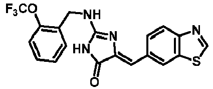 | C₁₉H₁₃F₃N₄O₂S | 418.39 | 98% | Yellow solid. ¹H NMR (500 MHz, DMSO-*d*₆, 300K) of major tautomer δ_{H} 10.93 (bs, 1H, NH, D₂O exchanged), 9.36 (s, 1H), 8.83 (s, 1H), 8.23 (d, *J* = 8.5 Hz, 1H), 8.14 (bs, 1H, NH, D₂O exchanged), 8.02 (d, *J* = 8.5 Hz, 1H), 7.57 - 7.36 (m, 3H), 7.27 (d, *J* = 7.6 Hz, 1H), 6.45 (s, 1H), 4.63 (s, 2H). MS (ESI⁺) : [M+H]⁺ 419.2. |
| **80** | 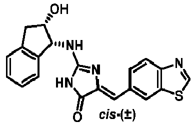 | C₂₀H₁₆N₄O₂S | 376.43 | >98% | Yellow solid. ¹H NMR (400 MHz, DMSO-*d*₆, 323K) of major tautomer δ_{H} 10.25 (bs, 1H, NH, D₂O exchanged), 9.31 (s, 1H), 8.80 (s, 1H), 8.30 (d, *J* = 8.7 Hz, 1H), 8.01 (d, *J* = 8.6 Hz, 1H), 7.55 - 7.07 (m, 4H + NH, D₂O exchanged), 6.51 (s, 1H), 5.54 - 5.40 (m, 1H), 5.34 (bs, 1H, OH, D₂O exchanged), 4.68 - 4.57 (m, 1H), 3.19 - 3.13 (m, 1H), 2.96 - 2.84 (m, 1H). MS (ESI⁺) : [M+H]⁺ 377.2. |
| **81** | 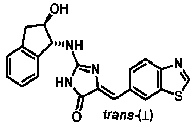 | C₂₀H₁₆N₄O₂S | 376.43 | >98% | Yellow solid. ¹H NMR (400 MHz, DMSO-*d*₆, 373K) δ_{H} 10.18 (bs, 1H, NH, D₂O exchanged), 9.27 (s, 1H), 8.87 - 8.56 (m, 1H), 8.27 - 8.06 (m, 1H), 8.01 (d, *J* = 8.4 Hz, 1H), 7.67 (bs, 1H, NH, D₂O exchanged), 7.39 - 7.11 (m, 4H), 6.49 (s, 1H), 5.55 - 4.90 (m, 1H + OH, D₂O exchanged), 4.49 (q, *J* = 6.7 Hz, 1H), 3.26 (dd, *J* = 15.7, 7.1 Hz, 1H), 2.82 (dd, *J* = 15.7, 6.9 Hz, 1H). MS (ESI⁺) : [M+H]⁺ 377.2. |
| **82** | 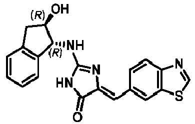 | C₂₀H₁₆N₄O₂S | 376.43 | >98% | Yellow solid. ¹H NMR (400 MHz, DMSO-*d*₆, 373K) δ_{H} 10.18 (bs, 1H, NH, D₂O exchanged), 9.27 (s, 1H), 8.87 - 8.56 (m, 1H), 8.27 - 8.06 (m, 1H), 8.01 (d, *J* = 8.4 Hz, 1H), 7.67 (bs, 1H, NH, D₂O exchanged), 7.39 - 7.11 (m, 4H), 6.49 (s, 1H), 5.55 - 4.90 (m, 1H + OH, D₂O exchanged), 4.49 (q, *J* = 6.7 Hz, 1H), 3.26 (dd, *J* = 15.7, 7.1 Hz, 1H), 2.82 (dd, *J* = 15.7, 6.9 Hz, 1H). MS (ESI⁺) : [M+H]⁺ 377.1. |
| **83** | 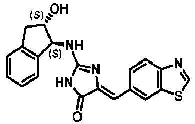 | C₂₀H₁₆N₄O₂S | 376.43 | >98% | Yellow solid. ¹H NMR (400 MHz, DMSO-*d*₆, 373K) δ_{H} 10.18 (bs, 1H, NH, D₂O exchanged), 9.27 (s, 1H), 8.87 - 8.56 (m, 1H), 8.27 - 8.06 (m, 1H), 8.01 (d, *J* = 8.4 Hz, 1H), 7.67 (bs, 1H, NH, D₂O exchanged), 7.39 - 7.11 (m, 4H), 6.49 (s, 1H), 5.55 - 4.90 (m, 1H + OH, D₂O exchanged), 4.49 (q, *J* = 6.7 Hz, 1H), 3.26 (dd, *J* = 15.7, 7.1 Hz, 1H), 2.82 (dd, *J* = 15.7, 6.9 Hz, 1H). MS (ESI⁺) : [M+H]⁺ 377.1. |
| **84** | 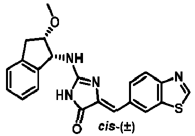 | C₂₁H₁₈N₄O₂S | 390.46 | >98% | Yellow solid. ¹H NMR (400 MHz, DMSO-*d*₆, 300K) of major tautomer δ_{H} 10.22 (bs, 1H, NH, D₂O exchanged), 9.34 (s, 1H), 8.82 (s, 1H), 8.44 - 8.23 (m, 1H), 8.02 (d, *J* = 8.6 Hz, 1H), 7.51 (bs, 1H, NH, D₂O exchanged), 7.44 - 7.15 (m, 4H), 6.51 (s, 1H), 5.77 - 5.54 (m, 1H), 4.39 - 4.19 (m, 1H), 3.35 (s, 3H), 3.19 - 3.01 (m, 2H). MS (ESI⁺) : [M+H]⁺ 391.2. |
| **85** | 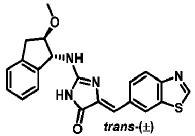 | C₂₁H₁₈N₄O₂S | 390.46 | >98% | Yellow solid. ¹H NMR (400 MHz, DMSO-*d*₆, 300K) of major tautomer δ_{H} 10.73 (bs, 1H, NH, D₂O exchanged), 9.35 (s, 1H), 8.82 (s, 1H), 8.23 (dd, *J* = 8.6, 1.6 Hz, 1H), 8.12 (bs, 1H, NH, D₂O exchanged), 8.01 (d, *J* = 8.6 Hz, 1H), 7.44 - 7.10 (m, 4H), 6.48 (s, 1H), 5.60 - 5.22 (m, 1H), 4.30 - 4.13 (m, 1H), 3.46 (s, 3H), 3.41 - 3.33 (m, 1H), 2.93 - 2.74 (m, 1H). MS (ESI⁺) : [M+H]⁺ 391.2. |
| **86** | 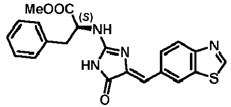 | C₂₁H₁₈NaO₃S | 406.46 | >98% | Yellow solid. ¹H NMR (400 MHz, DMSO-*d*₆, 300K) of major tautomer δ_{H} 10.68 (bs, 1H, D₂O exchanged), 9.38 (s, 1H), 8.82 (s, 1H), 8.19 (d, *J* = 8.6 Hz, 1H), 8.03 (d, *J* = 8.5 Hz, 1H), 8.00 (bs, 1H, NH, D₂O exchanged), 7.39 - 7.18 (m, 5H), 6.47 (s, 1H), 4.82 - 4.66 (m, 1H), 3.68 (s, 3H), 3.26 - 3.09 (m, 2H). MS (ESI⁺) : [M+H]⁺ 407.2. |
| **87** | 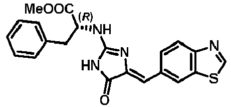 | C₂₁H₁₈NaO₃S | 406.46 | >98% | Yellow solid. ¹H NMR (400 MHz, DMSO-*d*₆, 300K) of major tautomer δ_{H} 10.68 (bs, 1H, D₂O exchanged), 9.38 (s, 1H), 8.82 (s, 1H), 8.19 (d, *J* = 8.6 Hz, 1H), 8.03 (d, *J* = 8.5 Hz, 1H), 8.00 (bs, 1H, NH, D₂O exchanged), 7.39 - 7.18 (m, 5H), 6.47 (s, 1H), 4.82 - 4.66 (m, 1H), 3.68 (s, 3H), 3.26 - 3.09 (m, 2H). MS (ESI⁺) : [M+H]⁺ 407.1. |
| **88** | 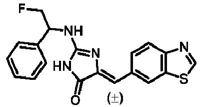 | C₁₉H₁₅FN₄OS | 366.41 | 94% | Yellow solid. ¹H NMR (400 MHz, DMSO-*d*₆, 300K) of major tautomer δ_{H} 10.76 (bs, 1H, NH, D₂O exchanged), 9.37 (s, 1H), 8.83 (s, 1H), 8.45 (bs, 1H, NH, D₂O exchanged), 8.19 (d, *J* = 8.6 Hz, 1H), 8.03 (d, *J* = 8.6 Hz, 1H), 7.58 - 7.16 (m, 5H), 6.44 (s, 1H), 5.58 - 5.22 (m, 1H), 4.96 - 4.52 (m, 2H). MS (ESI⁺) : [M+H]⁺ 367.1. |
| **89** | 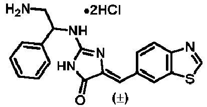 | C₁₉H₁₉Cl₂N₅OS | 436.36 | >98% | Yellow solid. ¹H NMR (400 MHz, DMSO-*d*₆, 323K) δ_{H} 9.38 (s, 1H), 8.88 - 8.73 (m, 1H), 8.28 (bs, 3H, NH, D₂O exchanged), 8.14 - 8.02 (m, 2H), 7.53 (d, *J* = 7.3 Hz, 2H), 7.44 (t, *J* = 7.6 Hz, 2H), 7.35 (t, *J* = 7.3 Hz, 1H), 6.55 (s, 1H), 5.53 - 5.35 (m, 1H), 4.45 (bs, 3H, NH, D₂O exchanged), 3.50 - 3.37 (m, 1H), 3.34 - 3.22 (m, 1H). MS (ESI⁺) : [M+H]⁺ 364.1 (+2HCl). |
| **90** | 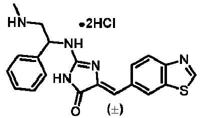 | C₂₀H₂₁Cl₂N₅OS | 450.38 | 84% | Yellow solid. ¹H NMR (400 MHz, DMSO-*d*₆ + D₂O, 300K) δ_{H} 9.39 (s, 1H), 8.82 (s, 1H), 8.18 (s, 1H), 8.06 (d, *J* = 8.3 Hz, 1H), 7.73 - 7.28 (m, 5H), 6.52 (s, 1H), 5.61 - 5.43 (m, 1H), 3.43 - 3.33 (m, 1H), 3.14 - 3.04 (m, 1H), 2.66 (s, 3H). MS (ESI⁺) : [M+H]⁺ 378.2 (+2HCl). |
| **91** | 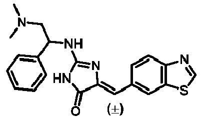 | C₂₁H₂₁N₅OS | 391.49 | >98% | Yellow solid. ¹H NMR (400 MHz, DMSO-*d*₆, 323K) δ_{H} 10.56 (bs, 1H, NH, D₂O exchanged), 9.34 (s, 1H), 8.86 - 8.66 (m, 1H), 8.15 - 7.96 (m, 2H), 7.83 (bs, 1H, NH, D₂O exchanged), 7.51 - 7.21 (m, 5H), 6.40 (s, 1H), 5.17 - 4.80 (m, 1H), 2.81 (dd, *J* = 12.5, 9.4 Hz, 1H), 2.49 - 2.42 (m, 1H), 2.26 (s, 6H). MS (ESI⁺) : [M+H]⁺ 392.1. |
| **92** | 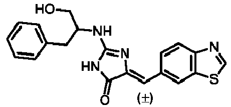 | C₂₀H₁₈N₄O₂S | 378.45 | >98% | Yellow solid. ¹H NMR (400 MHz, DMSO-*d*₆, 300K) of major tautomer δ_{H} 10.45 (bs, 1H, NH, D₂O exchanged), 9.38 (s, 1H), 9.04 - 8.83 (m, 1H), 8.31 - 8.10 (m, 1H), 8.05 (d, *J* = 8.4 Hz, 1H), 7.46 (bs, 1H, NH, D₂O exchanged), 7.42 - 7.26 (m, 4H), 7.26 - 7.10 (m, 1H), 6.41 (s, 1H), 5.33 - 4.75 (m, 1H, OH, D₂O exchanged), 4.30 - 3.84 (m, 1H), 3.68 - 3.39 (m, 2H), 3.11 - 2.70 (m, 2H). MS (ESI⁺) : [M+H]⁺ 379.2. |
| **93** | 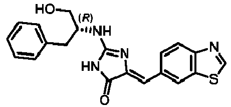 | C₂₀H₁₈N₄O₂S | 378.45 | >98% | Yellow solid. ¹H NMR (400 MHz, DMSO-*d*₆, 300K) of major tautomer δ_{H} 10.45 (bs, 1H, NH, D₂O exchanged), 9.38 (s, 1H), 9.04 - 8.83 (m, 1H), 8.31 - 8.10 (m, 1H), 8.05 (d, *J* = 8.4 Hz, 1H), 7.46 (bs, 1H, NH, D₂O exchanged), 7.42 - 7.26 (m, 4H), 7.26 - 7.10 (m, 1H), 6.41 (s, 1H), 5.33 - 4.75 (m, 1H, OH, D₂O exchanged), 4.30 - 3.84 (m, 1H), 3.68 - 3.39 (m, 2H), 3.11 - 2.70 (m, 2H). MS (ESI⁺) : [M+H]⁺ 379.2. |
| **94** | 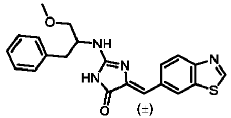 | C₂₁H₂₀N₄O₂S | 392.48 | 96% | Yellow solid. ¹H NMR (400 MHz, DMSO-*d*₆, 373K) δ_{H} 10.09 (bs, 1H, NH, D₂O exchanged), 9.29 (s, 1H), 8.96 - 8.72 (m, 1H), 8.33 - 8.07 (m, 1H), 8.03 (d, *J* = 8.0 Hz, 1H), 7.39 - 7.26 (m, 4H), 7.25 - 7.18 (m, 1H), 7.12 (bs, 1H, NH, D₂O exchanged), 6.44 (s, 1H), 4.38 - 4.26 (m, 1H), 3.57 - 3.44 (m, 2H), 3.35 (s, 3H), 3.05 - 2.90 (m, 2H). MS (ESI⁺) : [M+H]⁺ 393.2. |
| **95** | 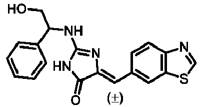 | C₁₉H₁₆N₄O₂S | 364.42 | >98% | Yellow solid. ¹H NMR (400 MHz, DMSO-*d*₆, 323K) δ_{H} 10.50 (bs, 1H, NH, D₂O exchanged), 9.34 (s, 1H), 9.00 - 8.63 (m, 1H), 8.25 - 8.07 (m, 1H), 8.07 - 7.97 (m, 1H), 7.92 (bs, 1H, NH, D₂O exchanged), 7.51 - 7.43 (m, 2H), 7.37 (t, *J* = 7.6 Hz, 2H), 7.26 (t, *J* = 7.3 Hz, 1H), 6.40 (s, 1H), 5.25 - 4.77 (m, 1H + OH, D₂O exchanged), 3.75 (d, *J* = 6.1 Hz, 2H). MS (ESI⁺) : [M+H]⁺ 365.2. |
| **96** | 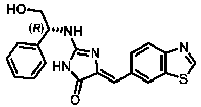 | C₁₉H₁₆N₄O₂S | 364.42 | >98% | Yellow solid. ¹H NMR (400 MHz, DMSO-*d*₆, 323K) δ_{H} 10.50 (bs, 1H, NH, D₂O exchanged), 9.34 (s, 1H), 9.00 - 8.63 (m, 1H), 8.25 - 8.07 (m, 1H), 8.07 - 7.97 (m, 1H), 7.92 (bs, 1H, NH, D₂O exchanged), 7.51 - 7.43 (m, 2H), 7.37 (t, *J* = 7.6 Hz, 2H), 7.26 (t, *J* = 7.3 Hz, 1H), 6.40 (s, 1H), 5.25 - 4.77 (m, 1H + OH, D₂O exchanged), 3.75 (d, *J* = 6.1 Hz, 2H). MS (ESI⁺) : [M+H]⁺ 365.2. |
| **97** | 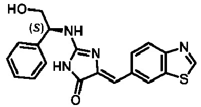 | C₁₉H₁₆N₄O₂S | 364.42 | >98% | Yellow solid. ¹H NMR (400 MHz, DMSO-*d*₆, 323K) δ_{H} 10.50 (bs, 1H, NH, D₂O exchanged), 9.34 (s, 1H), 9.00 - 8.63 (m, 1H), 8.25 - 8.07 (m, 1H), 8.07 - 7.97 (m, 1H), 7.92 (bs, 1H, NH, D₂O exchanged), 7.51 - 7.43 (m, 2H), 7.37 (t, *J* = 7.6 Hz, 2H), 7.26 (t, *J* = 7.3 Hz, 1H), 6.40 (s, 1H), 5.25 - 4.77 (m, 1H + OH, D₂O exchanged), 3.75 (d, *J* = 6.1 Hz, 2H). MS (ESI⁺) : [M+H]+ 365.2. |
| **98** | 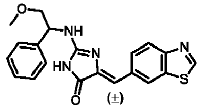 | C₂₀H₁₈N₄O₂S | 378.45 | >98% | Yellow solid. ¹H NMR (400 MHz, DMSO-*d*₆, 323K) δ_{H} 10.55 (bs, 1H, NH, D₂O exchanged), 9.34 (s, 1H), 8.82 (s, 1H), 8.29 - 7.92 (m, 2H + NH, D₂O exchanged), 7.48 (d, *J* = 7.6 Hz, 2H), 7.38 (t, *J* = 7.5 Hz, 2H), 7.28 (t, *J* = 7.3 Hz, 1H), 6.42 (s, 1H), 5.35 - 5.11 (m, 1H), 3.88 - 3.58 (m, 2H), 3.34 (s, 3H). MS (ESI⁺) : [M+H]⁺ 379.2. |
| **99** | 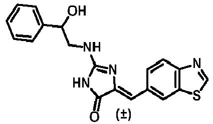 | C₁₉H₁₆N₄O₂S | 364.42 | >98% | Yellow solid. ¹H NMR (400 MHz, DMSO-*d*₆, 300K) of major tautomer δ_{H} 10.57 (bs, 1H, NH, D₂O exchanged), 9.37 (s, 1H), 9.05 - 8.80 (m, 1H), 8.30 - 8.13 (m, 1H), 8.05 (d, *J* = 8.5 Hz, 1H), 7.57 (bs, 1H, NH, D₂O exchanged), 7.53 - 7.23 (m, 5H), 6.44 (s, 1H), 5.69 (bs, 1H, OH, D₂O exchanged), 5.19 - 4.67 (m, 1H), 3.85 - 3.50 (m, 1H), 3.48 - 3.33 (m, 1H). MS (ESI⁺) : [M+H]⁺ 365.2. |
| **100** | 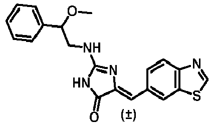 | C₂₀H₁₈N₄O₂S | 378.45 | 98% | Yellow solid. ¹H NMR (400 MHz, DMSO-*d*₆, 300K) of major tautomer δ_{H} 10.57 (bs, 1H, NH, D₂O exchanged), 9.37 (s, 1H), 8.94 (s, 1H), 8.20 (dd, *J* = 8.6, 1.6 Hz, 1H), 8.04 (d, *J* = 8.5 Hz, 1H), 7.67 (bs, 1H, NH, D₂O exchanged), 7.53 - 7.26 (m, 5H), 6.45 (s, 1H), 4.73 - 4.41 (m, 1H), 3.80 - 3.42 (m, 2H), 3.21 (s, 3H). MS (ESI⁺) : [M+H]⁺ 379.2. |
| **101** | 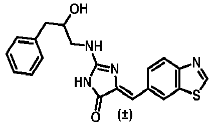 | C₂₀H₁₈N₄O₂S | 378.45 | 98% | Yellow solid. ¹H NMR (400 MHz, DMSO-*d*₆, 373K) of major tautomer δ_{H} 10.13 (bs, 1H, NH, D₂O exchanged), 9.29 (s, 1H), 8.75 - 8.59 (m, 1H), 8.21 - 8.05 (m, 1H), 8.01 (d, *J* = 8.1 Hz, 1H), 7.35 - 7.15 (m, 5H), 7.14 - 6.92 (bs, 1H, NH, D₂O exchanged), 6.44 (s, 1H), 5.12 - 4.60 (m, 1H, OH, D₂O exchanged), 4.12 - 3.86 (m, 1H), 3.54 (dd, *J* = 13.4, 3.8 Hz, 1H), 3.34 (dd, *J* = 13.3, 7.0 Hz, 1H), 2.89 - 2.72 (m, 2H). MS (ESI⁺) : [M+H]⁺ 379.2. |
| **102** | 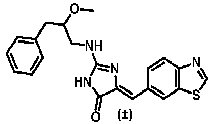 | C₂₁H₂₀N₄O₂S | 392.48 | 96% | Yellow solid. ¹H NMR (400 MHz, DMSO-*d*₆, 323K) of major tautomer δ_{H} 10.44 (bs, 1H, NH, D₂O exchanged), 9.34 (s, 1H), 8.94 - 8.73 (m, 1H), 8.36 - 8.10 (m, 1H), 8.00 (d, *J* = 8.6 Hz, 1H), 7.41 (bs, 1H, NH, D₂O exchanged), 7.36 - 7.17 (m, 5H), 6.43 (s, 1H), 3.77 - 3.66 (m, 1H), 3.60 - 3.41 (m, 2H), 3.35 (s, 3H), 2.91 - 2.78 (m, 2H). MS (ESI⁺) : [M+H]⁺ 393.2. |
| **103** | 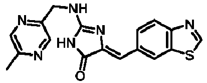 | C₁₇H₁₄N₆OS | 350.41 | 97% | Yellow solid. ¹H NMR (500 MHz, Methanol-*d*₄, 300K) of major tautomer δ_{H} 9.24 (s, 1H), 8.89 - 8.56 (m, 2H), 8.52 (s, 1H), 8.22 - 7.78 (m, 2H), 6.65 (s, 1H), 4.81 (s, 2H), 2.54 (s, 3H). MS (ESI⁺) : [M+H]⁺ 351.2. |
| **104** | 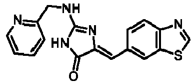 | C₁₇H₁₃N₅OS | 335.39 | >98% | Yellow solid. ¹H NMR (500 MHz, DMSO-*d*₆, 300K) of major tautomer δ_{H} 10.84 (bs, 1H, NH, D₂O exchanged), 9.35 (s, 1H), 8.79 (s, 1H), 8.57 (d, *J* = 4.9 Hz, 1H), 8.21 (d, *J* = 8.6 Hz, 1H), 8.12 (bs, 1H, NH, D₂O exchanged), 8.01 (d, *J* = 8.5 Hz, 1H), 7.81 (td, *J* = 7.7, 1.9 Hz, 1H), 7.51 - 7.43 (m, 1H), 7.34 - 7.26 (m, 1H), 6.44 (s, 1H), 4.72 - 4.48 (m, 2H). MS (ESI⁺) : [M+H]⁺ 336.2. |
| **105** | 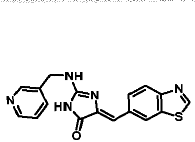 | C₁₇H₁₃N₅OS | 335.39 | >98% | Yellow solid. ¹H NMR (400 MHz, DMSO-*d*₆, 300K) δ_{H} 10.95 (bs, 1H, NH, D₂O exchanged), 9.35 (s, 1H), 8.91 - 8.75 (m, 1H), 8.73 - 8.57 (m, 1H), 8.47 (dd, *J* = 4.8, 1.7 Hz, 1H), 8.30 - 8.20 (m, 1H), 8.15 (bs, 1H, NH, D₂O exchanged), 8.03 (d, *J* = 8.5 Hz, 1H), 7.89 - 7.78 (m, 1H), 7.39 (dd, *J* = 7.8, 4.8 Hz, 1H), 6.45 (s, 1H), 4.72 - 4.48 (m, 2H). MS (ESI⁺) : [M+H]⁺: 336.2. |
| **106** | 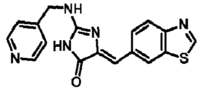 | C₁₇H₁₃N₅OS | 335.39 | >98% | Yellow solid. ¹H NMR (400 MHz, DMSO-*d*₆, 300K) of major tautomer δ_{H} 11.04 (bs, 1H, NH, D₂O exchanged), 9.36 (s, 1H), 8.79 (s, 1H), 8.55 (d, *J* = 5.9 Hz, 2H), 8.20 (d, *J* = 8.4 Hz, 1H), 8.15 (bs, 1H, NH, D₂O exchanged), 8.02 (d, *J* = 8.6 Hz, 1H), 7.41 (d, *J* = 4.6 Hz, 2H), 6.45 (s, 1H), 4.72 - 4.54 (m, 2H). MS (ESI⁺) : [M+H]⁺: 336.2. |
| **107** | 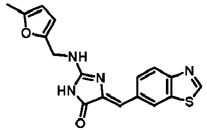 | C₁₇H₁₄N₄O₂S | 338.39 | >98% | Yellow solid. ¹H NMR (400 MHz, DMSO-*d*₆, 300K) of major tautomer δ_{H} 10.77 (bs, 1H, NH, D₂O exchanged), 9.36 (s, 1H), 8.86 (s, 1H), 8.27 (d, *J* = 8.6 Hz, 1H), 8.04 (d, *J* = 8.6 Hz, 1H), 7.99 (bs, 1H, NH, D₂O exchanged), 6.45 (s, 1H), 6.26 (d, *J* = 3.1 Hz, 1H), 6.02 (dd, *J* = 3.0, 1.3 Hz, 1H), 4.53 (s, 2H), 2.25 (s, 3H). MS (ESI⁺) : [M+H]⁺ 339.2. |
| **108** | 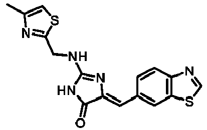 | C₁₆H₁₃N₅OS₂ | 355.43 | >98% | Yellow solid. ¹H NMR (500 MHz, DMSO-*d*₆, 300K) of major tautomer δ_{H} 11.01 (bs, 1H, NH, D₂O exchanged), 9.36 (s, 1H), 8.88 (s, 1H), 8.33 (bs, 1H, NH, D₂O exchanged), 8.22 (d, *J* = 8.5 Hz, 1H), 8.02 (d, *J* = 8.6 Hz, 1H), 7.18 (s, 1H), 6.49 (s, 1H), 4.84 (s, 2H), 2.36 (s, 3H). MS (ESI⁺) : [M+H]⁺ 356.2. |
| **109** | 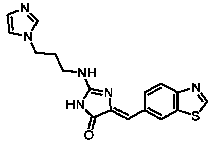 | C₁₇H₁₆N₆OS | 352.42 | >98% | Yellow solid. ¹H NMR (500 MHz, DMSO-*d*₆, 300K) of major tautomer δ_{H} 10.86 (bs, 1H, NH, D₂O exchanged), 9.36 (s, 1H), 8.86 - 8.71 (m, 1H), 8.39 - 8.20 (m, 1H), 8.05 (d, *J* = 8.6 Hz, 1H), 7.74 (s, 1H), 7.65 (bs, 1H, NH, D₂O exchanged), 7.26 (s, 1H), 6.95 (s, 1H), 6.42 (s, 1H), 4.08 (t, *J* = 7.0 Hz, 2H), 3.45 - 3.28 (m, 2H), 2.12 - 1.97 (m, 2H). MS (ESI⁺) : [M+H]⁺ 353.2. |
| **110** | 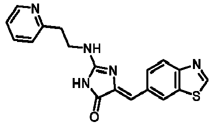 | C₁₈H₁₅N₅OS | 349.41 | 95% | Yellow solid. ¹H NMR (500 MHz, DMSO-*d*₆, 300K) of major tautomer δ_{H} 10.67 (bs, 1H, NH, D₂O exchanged), 9.36 (s, 1H), 8.87 (s, 1H), 8.54 (d, *J* = 4.9 Hz, 1H), 8.30 - 8.18 (m, 1H), 8.04 (d, *J* = 8.6 Hz, 1H), 7.75 (td, *J* = 7.6, 1.9 Hz, 1H), 7.53 (bs, 1H, NH, D₂O exchanged verifier), 7.35 (d, *J* = 7.8 Hz, 1H), 7.27 - 7.22 (m, 1H), 6.41 (s, 1H), 3.85 - 3.68 (m, 2H), 3.16 - 3.03 (m, 2H). MS (ESI⁺) : [M+H]⁺ 350.1. |
| **111** | 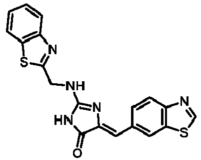 | C₁₉H₁₃N₅OS₂ | 391.47 | 98% | Yellow solid. ¹H NMR (500 MHz, DMSO-*d*₆, 300K) of major tautomer δ_{H} 11.13 (bs, 1H, NH, D₂O exchanged), 9.35 (s, 1H), 8.86 (s, 1H), 8.47 (bs, 1H, NH, D₂O exchanged), 8.19 (d, *J* = 8.5 Hz, 1H), 8.06 (d, *J* = 8.1 Hz, 1H), 8.00 (t, *J* = 9.0 Hz, 2H), 7.51 (t, *J* = 7.6 Hz, 1H), 7.41 (t, *J* = 7.6 Hz, 1H), 6.50 (s, 1H), 5.12 - 4.91 (m, 2H). MS (ESI⁺) : [M+H]⁺ 392.2. |
| **112** | 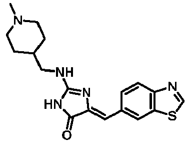 | C₁₈H₂₁N₅OS | 355.46 | 92% | Yellow solid. ¹H NMR (500 MHz, Methanol-*d*₄, 300K) of major tautomer δ_{H} 9.24 (s, 1H), 8.81 - 7.74 (m, 3H), 6.65 (s, 1H), 3.59 - 3.36 (m, 2H), 3.28 - 3.14 (m, 2H), 2.73 - 2.42 (m, 5H), 2.09 - 1.76 (m, 3H), 1.63 - 1.38 (m, 2H). MS (ESI⁺) : [M+H]⁺ 356.2. |
| **113** | 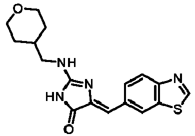 | C₁₇H₁₈N₄O₂S | 342.42 | 97% | Yellow solid. ¹H NMR (500 MHz, DMSO-*d*₆, 300K) of major tautomer δ_{H} 10.64 (bs, 1H, NH, D₂O exchanged), 9.35 (s, 1H), 8.83 (s, 1H), 8.36 - 8.18 (m, 1H), 8.03 (d, *J* = 8.5 Hz, 1H), 7.56 (bs, 1H, NH, D₂O exchanged), 6.40 (s, 1H), 3.97 - 3.78 (m, 2H), 3.31 - 3.11 (m, 3H), 2.03 - 1.74 (m, 1H), 1.63 (d, *J* = 13.1 Hz, 2H), 1.43 - 1.10 (m, 3H). MS (ESI⁺) : [M+H]⁺ 343.3. |
| **114** | 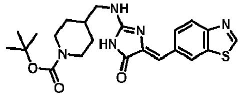 | C₂₂H₂₇N₅O₃S | 441.55 | >98% | Yellow solid. ¹H NMR (400 MHz, DMSO-*d*₆, 300K) of major tautomer δ_{H} 10.64 (bs, 1H, NH, D₂O exchanged), 9.35 (s, 1H), 8.83 (s, 1H), 8.38 - 8.17 (m, 1H), 8.02 (d, *J* = 8.5 Hz, 1H), 7.56 (bs, 1H, NH, D₂O exchanged), 6.40 (s, 1H), 4.15 - 3.81 (m, 2H), 3.31 - 3.01 (m, 2H), 2.90 - 2.57 (m, 2H), 1.96 - 1.60 (m, 3H), 1.39 (s, 9H), 1.19 - 0.94 (m, 2H).. MS (ESI⁺) : [M+H]⁺ 442.3. |
| **115** | 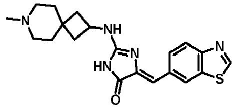 | C₂₀H₂₃N₅OS | 381.5 | 90% | Yellow solid. ¹H NMR (400 MHz, DMSO-*d*₆, 300K) of major tautomer δ_{H} 10.22 (bs, 1H, NH, D₂O exchanged), 9.33 (s, 1H), 8.93 - 8.64 (m, 1H), 8.33 - 8.09 (m, 1H + NH, D₂O exchanged), 8.02 (d, *J* = 8.1 Hz, 1H), 6.39 (s, 1H), 4.45 - 4.14 (m, 1H), 2.33 - 2.16 (m, 4H), 2.11 (s, 3H), 1.95 - 1.74 (m, 3H), 1.65 - 1.45 (m, 5H). MS (ESI⁺) : [M+H]⁺ 382.3. |
| **116** | 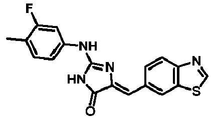 | C₁₈H₁₃FN₄OS | 352.39 | >98% | Yellow solid. ¹H NMR (500 MHz, DMSO-*d*₆, 300K) δ_{H} 10.89 (s, 1H, NH, D₂O exchanged), 10.08 (bs, 1H, NH, D₂O exchanged), 9.42 (s, 1H), 8.95 (s, 1H), 8.25 (d, *J* = 7.0 Hz, 1H), 8.09 (d, *J* = 8.5 Hz, 1H), 7.92 (d, *J* = 12.9 Hz, 1H), 7.38 (d, *J* = 8.2 Hz, 1H), 7.29 (t, *J* = 8.5 Hz, 1H), 6.68 (s, 1H), 2.23 (s, 3H). MS (ESI⁺) : [M+H]+ 353.1. |
| **117** | 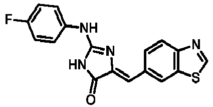 | C₁₇H₁₁FN₄OS | 338.37 | >98% | Yellow solid. ¹H NMR (500 MHz, DMSO-*d*₆, 300K) δ_{H} 10.87 (bs, 1H, NH, D₂O exchanged), 9.97 (bs, 1H, NH, D₂O exchanged), 9.41 (s, 1H), 8.81 (s, 1H), 8.36 (d, *J* = 8.9 Hz, 1H), 8.10 (d, *J* = 8.6 Hz, 1H), 7.91 - 7.76 (m, 2H), 7.26 (t, *J* = 8.8 Hz, 2H), 6.65 (s, 1H).MS (ESI⁺) : [M+H]+ 339.1. |
| **118** | 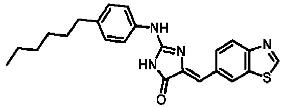 | C₂₃H₂₄N₄OS | 404.53 | >98% | Yellow solid. ¹H NMR (400 MHz, DMSO-*d*₆, 300K) δ_{H} 10.76 (bs, 1H, NH, D₂O exchanged), 9.85 (bs, 1H, NH, D₂O exchanged), 9.41 (s, 1H), 8.85 (s, 1H), 8.49 - 8.24 (m, 1H), 8.22 - 8.02 (m, 1H), 7.90 - 7.54 (m, 2H), 7.37 - 7.11 (m, 2H), 6.63 (s, 1H), 2.67 - 2.53 (m, 2H), 1.72 - 1.47 (m, 2H), 1.29 (s, 6H), 0.87 (s, 3H). MS (ESI⁺) : [M+H]+ 405.3. |
| **119** | 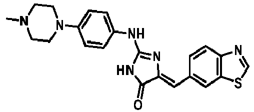 | C₂₂H₂₂N₆OS | 418.52 | 97% | Yellow solid. ¹H NMR (400 MHz, DMSO-*d*₆, 300K) δ_{H} 10.73 (bs, 1H, NH, D₂O exchanged), 9.73 (bs, 1H, NH, D₂O exchanged), 9.39 (s, 1H), 8.88 - 8.77 (m, 1H), 8.43 - 8.30 (m, 1H), 8.09 (d, *J* = 8.5 Hz, 1H), 7.73 - 7.53 (m, 2H), 6.99 (d, *J* = 8.5 Hz, 2H), 6.57 (s, 1H), 3.18 - 3.10 (m, 4H), 2.48 - 2.44 (m, 4H), 2.23 (s, 3H). MS (ESI⁺) : [M+H]⁺ 419.2. |
| **120** | 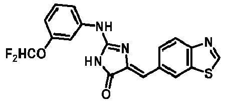 | C₁₈H₁₂F₂N₄O₂S | 386.37 | 96% | Yellow solid. ¹H NMR (500 MHz, DMSO-*d*₆, 300K) δ_{H} 10.87 (bs, 1H, NH, D₂O exchanged), 10.19 (bs, 1H, NH, D₂O exchanged), 9.42 (s, 1H), 8.88 (s, 1H), 8.37 - 8.27 (m, 1H), 8.17 (s, 1H), 8.06 (d, *J* = 8.5 Hz, 1H), 7.49 - 7.30 (m, 3H), 6.98 - 6.86 (m, 1H), 6.71 (s, 1H). MS (ESI⁺) : [M+H]⁺ 387.1. |
| **121** | 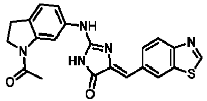 | C₂₁H₁₇N₅O₂S | 403.47 | 98% | Yellow solid. ¹H NMR (400 MHz, DMSO-*d*₆, 300K) δ_{H} 10.57 (bs, 1H, NH, D₂O exchanged), 9.89 (bs, 1H, NH, D₂O exchanged), 9.39 (s, 1H), 9.27 - 9.00 (m, 2H), 8.32 - 8.00 (m, 2H), 7.36 - 7.06 (m, 2H), 6.64 (s, 1H), 4.31 - 4.04 (m, 2H), 3.20 - 3.06 (m, 2H), 2.31 (s, 3H). MS (ESI⁺) : [M+H]+ 404.2. |
| **122** | 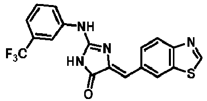 | C₁₈H₁₁F₃N₄OS | 388.37 | 95% | Yellow solid. ¹H NMR (500 MHz, DMSO-*d*₆, 300K) δ_{H} 11.02 (bs, 1H, NH, D₂O exchanged), 10.34 (bs, 1H, NH, D₂O exchanged), 9.42 (s, 1H), 8.96 (s, 1H), 8.85 (s, 1H), 8.24 (d, *J* = 8.5 Hz, 1H), 8.05 (d, *J* = 8.5 Hz, 1H), 7.75 (d, *J* = 8.3 Hz, 1H), 7.62 (t, *J* = 7.8 Hz, 1H), 7.46 (d, *J* = 7.6 Hz, 1H), 6.74 (s, 1H). MS (ESI⁺) : [M+H]+ 389.1. |
| **123** | 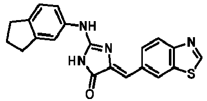 | C₂₀H₁₆N₄OS | 360.44 | >98% | Yellow solid. ¹H NMR (500 MHz, DMSO-*d*₆, 300K) δ_{H} 10.72 (bs, 1H, NH, D₂O exchanged), 9.83 (bs, 1H, NH, D₂O exchanged), 9.40 (s, 1H), 9.03 (s, 1H), 8.21 (d, *J* = 8.5 Hz, 1H), 8.08 (d, *J* = 8.5 Hz, 1H), 7.93 (s, 1H), 7.38 (d, *J* = 8.1 Hz, 1H), 7.23 (d, *J* = 8.1 Hz, 1H), 6.62 (s, 1H), 2.96 (t, *J* = 7.3 Hz, 2H), 2.86 (t, *J* = 7.3 Hz, 2H), 2.08 (p, *J* = 7.4 Hz, 2H). MS (ESI⁺) : [M+H]+ 361.1. |
| **124** | 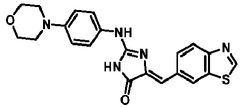 | C₂₁H₁₉N₅O₂S | 405.48 | >98% | Yellow solid. ¹H NMR (500 MHz, DMSO-*d*₆, 300K) δ_{H} 10.72 (bs, 1H, NH, D₂O exchanged), 9.73 (bs, 1H, NH, D₂O exchanged), 9.39 (s, 1H), 8.83 (s, 1H), 8.35 (d, *J* = 8.7 Hz, 1H), 8.09 (d, *J* = 8.5 Hz, 1H), 7.66 (d, *J* = 8.2 Hz, 2H), 7.01 (d, *J* = 8.6 Hz, 2H), 6.58 (s, 1H), 3.81 - 3.72 (m, 4H), 3.15 - 3.06 (m, 4H). MS (ESI⁺) : [M+H]⁺ 406.2. |
| **125** | 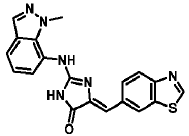 | C₁₉H₁₄N₆OS | 374.42 | 97% | Yellow solid. ¹H NMR (500 MHz, DMSO-*d*₆, 300K) of major tautomer δ_{H} 11.21 (bs, 1H, NH, D₂O exchanged), 10.40 (bs, 1H, NH, D₂O exchanged), 9.39 (s, 1H), 8.74 - 8.59 (m, 1H), 8.16 - 7.91 (m, 3H), 7.72 - 7.41 (m, 1H), 7.27 - 6.88 (m, 2H), 6.50 (s, 1H), 4.19 (s, 3H). MS (ESI⁺) : [M+H]⁺ 375.1. |
| **126** | 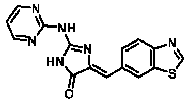 | C₁₅H₁₀N₆OS | 322.35 | 98% | Yellow solid. ¹H NMR (400 MHz, DMSO-*d*₆, 300K) δ_{H} 11.56 (bs, 1H, NH, D₂O exchanged), 11.37 (bs, 1H, NH, D₂O exchanged), 9.44 (s, 1H), 9.12 - 8.84 (m, 1H), 8.65 (d, *J* = 4.9 Hz, 2H), 8.42 - 8.25 (m, 1H), 8.12 (d, *J* = 8.5 Hz, 1H), 7.38 - 7.06 (m, 1H), 6.81 (s, 1H). MS (ESI⁺) : [M+H]+ 323.2. |
| **127** | 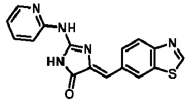 | C₁₆H₁₁N₅OS | 321.36 | 96% | Yellow solid. ¹H NMR (400 MHz, DMSO-*d*₆, 300K) δ_{H} 11.20 (bs, 1H, NH, D₂O exchanged), 10.97 (bs, 1H, NH, D₂O exchanged), 9.43 (s, 1H), 8.88 (s, 1H), 8.49 - 8.26 (m, 2H), 8.12 (d, *J* = 8.6 Hz, 1H), 7.84 (t, *J* = 8.0 Hz, 1H), 7.66 - 7.33 (m, 1H), 7.23 - 6.98 (m, 1H), 6.76 (s, 1H). MS (ESI⁺) : [M+H]⁺ 322.2. |
| **128** | 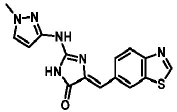 | C₁₅H₁₂N₆OS | 324.37 | >98% | Pale yellow solid. ¹H NMR (400 MHz, DMSO-*d*₆, 300K) of major tautomer δ_{H} 10.58 (bs, 2H, NH, D₂O exchanged), 9.40 (s, 1H), 8.81 (s, 1H), 8.48 - 8.03 (m, 2H), 7.80 - 7.63 (m, 1H), 6.61 (s, 1H), 6.49 - 6.23 (m, 1H), 3.80 (s, 3H). MS (ESI⁺) : [M+H]⁺ 325.1. |
| **129** | 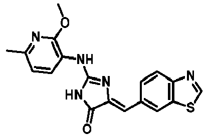 | C₁₈H₁₅N₅O₂S | 365.41 | 98% | Yellow solid. ¹H NMR (500 MHz, DMSO-*d*₆, 300K) δ_{H} 10.30 (bs, 1H, NH, D₂O exchanged), 9.42 (s, 1H), 9.06 (bs, 1H, NH, D₂O exchanged), 8.90 - 8.72 (m, 1H), 8.72 - 8.54 (m, 1H), 8.44 - 8.23 (m, 1H), 8.11 (d, *J* = 8.5 Hz, 1H), 6.99 (d, *J* = 7.7 Hz, 1H), 6.69 (s, 1H), 3.99 (s, 3H), 2.41 (s, 3H). MS (ESI⁺) : [M+H]⁺ 366.2. |
| **130** | 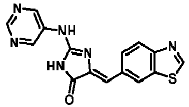 | C₁₅H₁₀N₆OS | 322.35 | >98% | Yellow solid. ¹H NMR (500 MHz, DMSO-*d*₆, 300K) δ_{H} 11.30 (bs, 1H, NH, D₂O exchanged), 10.37 (bs, 1H, NH, D₂O exchanged), 9.42 (s, 1H), 9.41 - 9.02 (m, 2H), 8.92 (s, 1H), 8.88 - 8.69 (m, 1H), 8.45 - 8.17 (m, 1H), 8.10 (d, *J* = 8.5 Hz, 1H), 6.93 - 6.49 (m, 1H). MS (ESI⁺) : [M+H]⁺ 323.1. |
| **131** | 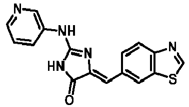 | C₁₆H₁₁N₅OS | 321.36 | >98% | Yellow solid. ¹H NMR (400 MHz, DMSO-*d*₆, 300K) δ_{H} 11.03 (bs, 1H, NH, D₂O exchanged), 10.16 (bs, 1H, NH, D₂O exchanged), 9.42 (s, 1H), 9.06 - 8.90 (m, 1H), 8.87 - 8.78 (m, 1H), 8.44 - 8.21 (m, 3H), 8.11 (d, *J* = 8.5 Hz, 1H), 7.49 - 7.41 (m, 1H), 6.70 (s, 1H). MS (ESI⁺) : [M+H]+ 322.2. |
| **132** | 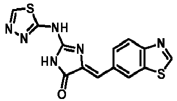 | C₁₃H₈N₆OS₂ | 328.37 | 95% | Yellow solid. ¹H NMR (400 MHz, DMSO-*d*₆, 300K) δ_{H} 12.52 (bs, 1H, NH, D₂O exchanged), 11.38 (bs, 1H, NH, D₂O exchanged), 9.44 (s, 1H), 9.18 - 9.03 (m, 1H), 8.87 - 8.71 (m, 1H), 8.53 - 8.24 (m, 1H), 8.15 (d, *J* = 8.4 Hz, 1H), 6.76 (s, 1H). MS (ESI⁺) : [M+H]⁺ 329.2. |
| **133** | 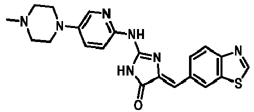 | C₂₁H₂₁N₇OS | 419.51 | 95% | Yellow solid. ¹H NMR (400 MHz, DMSO-*d*₆, 300K) δ_{H} 11.02 (bs, 1H, NH, D₂O exchanged), 10.78 (bs, 1H, NH, D₂O exchanged), 9.42 (s, 1H), 8.99 - 8.65 (m, 1H), 8.47 - 8.22 (m, 1H), 8.20 - 8.06 (m, 1H), 8.01 (s, 1H), 7.60 - 7.49 (m, 1H), 7.48 - 7.22 (m, 1H), 6.67 (s, 1H), 3.23 - 3.06 (m, 4H), 2.62 - 2.52 (m, 4H), 2.28 (s, 3H). MS (ESI⁺) : [M+H]+ 420.3. |
| **134** | 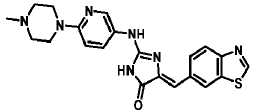 | C₂₁H₂₁N₇OS | 419.51 | >98% | Yellow solid. ¹H NMR (400 MHz, DMSO-*d*₆, 300K) δ_{H} 10.92 (bs, 1H, NH, D₂O exchanged), 9.79 (bs, 1H, NH, D₂O exchanged), 9.39 (s, 1H), 8.87 - 8.72 (m, 1H), 8.50 - 8.37 (m, 1H), 8.36 - 8.23 (m, 1H), 8.08 (d, *J* = 8.5 Hz, 1H), 8.04 - 7.90 (m, 1H), 6.92 (d, *J* = 9.1 Hz, 1H), 6.57 (s, 1H), 3.53 - 3.43 (m, 4H), 2.45 - 2.39 (m, 4H), 2.23 (s, 3H). MS (ESI⁺) : [M+H]+ 420.3. |
| **135** | 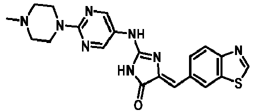 | C₂₀H₂₀N₈OS | 420.50 | >98% | Yellow solid. ¹H NMR (400 MHz, DMSO-*d*₆, 300K) δ_{H} 11.12 (bs, 1H, NH, D₂O exchanged), 9.79 (bs, 1H, NH, D₂O exchanged), 9.39 (s, 1H), 8.93 - 8.52 (m, 3H), 8.32 - 8.16 (m, 1H), 8.06 (d, *J* = 8.6 Hz, 1H), 6.58 (s, 1H), 3.86 - 3.65 (m, 4H), 2.48 - 2.36 (m, 4H), 2.26 (s, 3H). MS (ESI⁺) : [M+H]⁺ 421.2. |
| **136** | 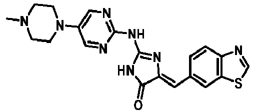 | C₂₀H₂₀N₈OS | 420.50 | >98% | Yellow solid. ¹H NMR (400 MHz, DMSO-*d*₆, 373K) δ_{H} 11.00 (bs, 2H, NH, D₂O exchanged), 9.35 (s, 1H), 8.79 (s, 1H), 8.38 (s, 2H), 8.25 - 7.98 (m, 2H), 6.72 (s, 1H), 3.26 - 3.17 (m, 4H), 2.54 - 2.51 (m, 4H), 2.27 (s, 3H). MS (ESI⁺) : [M+H]⁺ 421.2. |
| **137** | 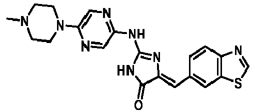 | C₂₀H₂₀N₈OS | 420.50 | >98% | Yellow solid. ¹H NMR (400 MHz, DMSO-*d*₆, 300K) of major tautomer δ_{H} 11.35 (bs, 1H, NH, D₂O exchanged), 10.82 (bs, 1H, NH, D₂O exchanged), 9.42 (s, 1H), 9.01- 7.83 (m, 5H), 6.64 (s, 1H), 3.60 - 3.44 (m, 4H), 2.48 - 2.41 (m, 4H), 2.24 (s, 3H). MS (ESI⁺) : [M+H]⁺ 421.2. |
| **138** | 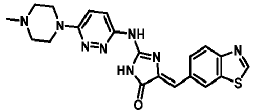 | C₂₀H₂₀N₈OS | 420.50 | >98% | Yellow solid. ¹H NMR (400 MHz, DMSO-*d*₆, 323K) δ_{H} 10.99 (bs, 2H, NH, D₂O exchanged), 9.40 (s, 1H), 8.76 - 8.59 (m, 1H), 8.25 - 8.00 (m, 2H), 7.71 - 7.53 (m, 1H), 7.47 (d, *J* = 9.7 Hz, 1H), 6.68 (s, 1H), 3.60 - 3.48 (m, 4H), 2.48 - 2.42 (m, 4H), 2.25 (s, 3H). MS (ESI⁺) : [M+H]⁺ 421.2. |
| **139** | 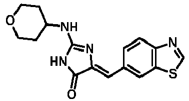 | C₁₆H₁₆N₄O₂S | 328.40 | 95% | Yellow solid. ¹H NMR (500 MHz, DMSO-*d*₆, 300K) of major tautomer δ_{H} 10.68 (bs, 1H, NH, D₂O exchanged), 9.35 (s, 1H), 8.81 (s, 1H), 8.37 - 8.21 (m, 1H), 8.03 (d, *J* = 8.7 Hz, 1H), 7.66 (bs, 1H, NH, D₂O exchanged), 6.41 (s, 1H), 4.21 - 3.70 (m, 3H), 3.55 - 3.33 (m, 2H), 2.00 - 1.79 (m, 2H), 1.67 - 1.50 (m, 2H). MS (ESI⁺) : [M+H]⁺ 329.2. |
| **140** | 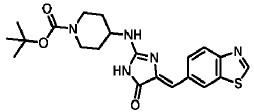 | C₂₁H₂₅N₅O₃S | 427.52 | >98% | Yellow solid. ¹H NMR (500 MHz, DMSO-*d*₆, 300K) of major tautomer δ_{H} 10.66 (bs, 1H, NH, D₂O exchanged), 9.35 (s, 1H), 8.81 (s, 1H), 8.32 - 8.13 (m, 1H), 8.02 (d, *J* = 8.6 Hz, 1H), 7.62 (bs, 1H, NH, D₂O exchanged), 6.42 (s, 1H), 4.22 - 3.66 (m, 3H), 3.08 - 2.73 (m, 2H), 2.00 - 1.79 (m, 2H), 1.54 - 1.30 (m, 11H). MS (ESI⁺) : [M+H]⁺ 428.3. |
| **141** | 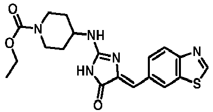 | C₁₉H₂₁N₅O₃S | 399.47 | >98% | Yellow solid. ¹H NMR (500 MHz, DMSO-*d*₆, 300K) of major tautomer δ_{H} 10.79 (bs, 1H, NH, D₂O exchanged), 9.36 (s, 1H), 8.81 (s, 1H), 8.45 - 8.16 (m, 1H), 8.13 - 7.95 (m, 1H), 7.68 (bs, 1H, NH, D₂O exchanged), 6.42 (s, 1H), 4.19 - 3.77 (m, 5H), 3.1 7 - 2.82 (m, 2H), 2.12 - 1.79 (m, 2H), 1.60 - 1.33 (m, 2H), 1.20 (t, *J* = 6.8 Hz, 3H). MS (ESI⁺) : [M+H]⁺ 400.2. |
| **142** | 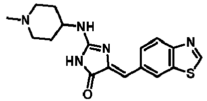 | C₁₇H₁₉N₅OS | 341.43 | 96% | Yellow solid. ¹H NMR (400 MHz, DMSO-*d*₆, 300K) of major tautomer δ_{H} 9.74 (bs, 1H, NH, D₂O exchanged), 9.34 (s, 1H), 8.82 - 8.66 (m, 1H), 8.36 - 8.09 (m, 1H), 8.03 (d, *J* = 8.4 Hz, 1H), 7.78 (bs, 1H, NH, D₂O exchanged), 6.40 (s, 1H), 3.89 - 3.56 (m, 1H), 2.81- 2.62 (m, 2H), 2.16 (s, 3H), 2.01 (s, 2H), 1.94 - 1.82 (m, 2H), 1.64 - 1.50 (m, 2H). MS (ESI⁺) : [M+H]⁺ 342.2. |
| **143** | 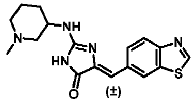 | C₁₇H₁₉N₅OS | 341.43 | >98% | Yellow solid. ¹H NMR (400 MHz, DMSO-*d*₆, 300K) of major tautomer δ_{H} 10.59 (bs, 1H, NH, D₂O exchanged), 9.36 (s, 1H), 8.80 (s, 1H), 8.43 - 8.18 (m, 1H), 8.04 (d, *J* = 8.5 Hz, 1H), 7.68 (bs, 1H, NH, D₂O exchanged), 6.44 (s, 1H), 4.40 - 3.95 (m, 1H), 3.15 - 2.96 (m, 2H), 2.47 - 2.28 (m, 3H), 1.94 -1.43 (m, 4H), 1.18 (t, *J* = 7.3 Hz, 2H). MS (ESI⁺) : [M+H]⁺ 342.2. |
| **144** | 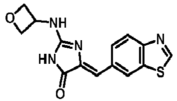 | C₁₄H₁₂N₄O₂S | 300.34 | >98% | Yellow solid. ¹H NMR (400 MHz, DMSO-*d*₆, 302K) δ_{H} 10.92 (bs, 1H, NH, D₂O exchanged), 9.37 (s, 1H), 8.92 - 8.73 (m, 1H), 8.44 (bs, 1H, NH, D₂O exchanged), 8.31 - 8.16 (m, 1H), 8.04 (d, *J* = 8.5 Hz, 1H), 6.46 (s, 1H), 5.19 - 4.93 (m, 1H), 4.90 - 4.73 (m, 2H), 4.72 - 4.54 (m, 2H). MS (ESI⁺) : [M+H]⁺ 301.1. |
| **145** | 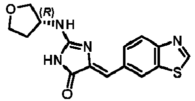 | C₁₅H₁₄N₄O₂S | 314.36 | >98% | Yellow solid. ¹H NMR (400 MHz, DMSO-*d*₆, 300K) of major tautomer δ_{H} 10.71 (bs, 1H, NH, D₂O exchanged), 9.36 (s, 1H), 8.81 (s, 1H), 8.28 (d, *J* = 8.7 Hz, 1H), 8.04 (d, *J* = 8.6 Hz, 1H), 7.97 (bs, 1H, NH, D₂O exchanged), 6.44 (s, 1H), 4.48 (s, 1H), 4.00 - 3.80 (m, 2H), 3.80 - 3.69 (m, 1H), 3.69 - 3.59 (m, 1H), 2.30 - 2.17 (m, 1H), 2.01 - 1.85 (m, 1H). MS (ESI⁺) : [M+H]⁺ 315.2. |
| **146** | 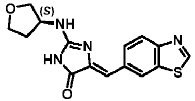 | C₁₅H₁₄N₄O₂S | 314.36 | >98% | Yellow solid. ¹H NMR (400 MHz, DMSO-*d*₆, 300K) of major tautomer δ_{H} 10.71 (bs, 1H, NH, D₂O exchanged), 9.36 (s, 1H), 8.81 (s, 1H), 8.28 (d, *J* = 8.7 Hz, 1H), 8.04 (d, *J* = 8.6 Hz, 1H), 7.97 (bs, 1H, NH, D₂O exchanged), 6.44 (s, 1H), 4.48 (s, 1H), 4.00 - 3.80 (m, 2H), 3.80 - 3.69 (m, 1H), 3.69 - 3.59 (m, 1H), 2.30 - 2.17 (m, 1H), 2.01 - 1.85 (m, 1H). MS (ESI⁺) : [M+H]⁺ 315.2. |
| **147** | 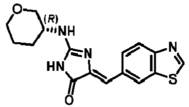 | C₁₆H₁₆N₄O₂S | 328.39 | >98% | Yellow solid. ¹H NMR (400 MHz, DMSO-*d*₆, 300K) of major tautomer δ_{H} 10.45 (bs, 1H, NH, D₂O exchanged), 9.36 (s, 1H), 8.77 (s, 1H), 8.28 (s, 1H), 8.04 (d, *J* = 8.6 Hz, 1H), 7.62 (bs, 1H, NH, D₂O exchanged), 6.43 (s, 1H), 4.18 - 3.36 (m, 5H), 2.09 - 1.88 (m, 1H), 1.81 - 1.46 (m, 3H). MS (ESI⁺) : [M+H]+ 329.2 |
| **148** | 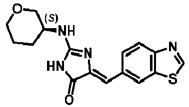 | C₁₆H₁₆N₄O₂S | 328.39 | >98% | Yellow solid. ¹H NMR (400 MHz, DMSO-*d*₆, 300K) of major tautomer δ_{H} 10.45 (bs, 1H, NH, D₂O exchanged), 9.36 (s, 1H), 8.77 (s, 1H), 8.28 (s, 1H), 8.04 (d, *J* = 8.6 Hz, 1H), 7.62 (bs, 1H, NH, D₂O exchanged), 6.43 (s, 1H), 4.18 - 3.36 (m, 5H), 2.09 - 1.88 (m, 1H), 1.81 - 1.46 (m, 3H). MS (ESI⁺) : [M+H]⁺ 329.2. |
| **149** | 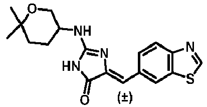 | C₁₈H₂₀N₄O₂S | 356.44 | >98% | Yellow solid. ¹H NMR (400 MHz, DMSO-*d*₆, 373K) of major tautomer δ_{H} 10.20 (bs, 1H, NH, D₂O exchanged), 9.28 (s, 1H), 8.89 - 8.64 (m, 1H), 8.33 - 8.11 (m, 1H), 8.02 (d, *J* = 8.4 Hz, 1H), 7.14 (bs, 1H, NH, D₂O exchanged), 6.45 (s, 1H), 3.95 - 3.72 (m, 2H), 3.62 - 3.49 (m, 1H), 2.01 - 1.76 (m, 2H), 1.73 - 1.60 (m, 1H), 1.58 - 1.46 (m, 1H), 1.29 - 1.15 (m, 6H). MS (ESI⁺) : [M+H]⁺ 357.2. |
| **150** | 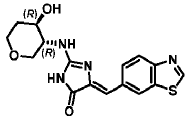 | C₁₆H₁₆N₄O₃S | 344.39 | 97% | Yellow solid. ¹H NMR (400 MHz, DMSO-*d*₆, 343K) of major tautomer δ_{H} 10.12 (bs, 1H, NH, D₂O exchanged), 9.32 (s, 1H), 8.89 - 8.54 (m, 1H), 8.34 - 8.08 (m, 1H), 8.04 (d, *J* = 8.4 Hz, 1H), 7.36 (bs, 1H, NH, D₂O exchanged), 6.45 (s, 1H), 4.96 (bs, 1H, OH, D₂O exchanged), 4.01 (d, *J* = 11.2 Hz, 1H), 3.93 - 3.79 (m, 1H), 3.78 - 3.58 (m, 2H), 3.41 (t, *J* = 10.7 Hz, 1H), 3.33 - 3.20 (m, 1H), 1.94 (d, *J* = 13.3 Hz, 1H), 1.63 - 1.47 (m, 1H). MS (ESI⁺) : [M+H]⁺ 345.2. |
| **151** | 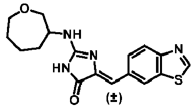 | C₁₇H₁₈N₄O₂S | 342.42 | >98% | Yellow solid. ¹H NMR (400 MHz, DMSO-*d*₆, 300K) of major tautomer δ_{H} 10.31 (bs, 1H, NH, D₂O exchanged), 9.35 (s, 1H), 8.79 (s, 1H), 8.35 - 8.17 (m, 1H), 8.03 (d, *J* = 8.6 Hz, 1H), 7.51 (bs, 1H, NH, D₂O exchanged), 6.42 (s, 1H), 4.32 - 3.99 (m, 1H), 3.89 - 3.53 (m, 4H), 1.96 - 1.50 (m, 6H). MS (ESI⁺) : [M+H]⁺ 343.2. |
| **152** | 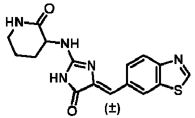 | C₁₆H₁₅N₅O₂S | 341.39 | >98% | Yellow solid. ¹H NMR (400 MHz, DMSO-*d*₆, 300K) of major tautomer δ_{H} 10.75 (bs, 1H, NH, D₂O exchanged), 9.36 (s, 1H), 8.84 (s, 1H), 8.23 (d, *J* = 8.6 Hz, 1H), 8.03 (d, *J* = 8.5 Hz, 1H), 7.78 (bs, 2H, NH, D₂O exchanged), 6.43 (s, 1H), 4.49 - 4.16 (m, 1H), 3.28 - 3.12 (m, 2H), 2.30 - 2.09 (m, 1H), 1.88 (s, 3H). MS (ESI⁺) : [M+H]⁺ 342.2. |
| **153** | 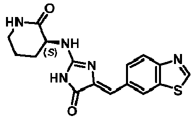 | C₁₆H₁₅N₅O₂S | 341.39 | >98% | Yellow solid. ¹H NMR (400 MHz, DMSO-*d*₆, 300K) of major tautomer δ_{H} 10.75 (bs, 1H, NH, D₂O exchanged), 9.36 (s, 1H), 8.84 (s, 1H), 8.23 (d, *J* = 8.6 Hz, 1H), 8.03 (d, *J* = 8.5 Hz, 1H), 7.78 (bs, 2H, NH, D₂O exchanged), 6.43 (s, 1H), 4.49 - 4.16 (m, 1H), 3.28 - 3.12 (m, 2H), 2.30 - 2.09 (m, 1H), 1.88 (s, 3H). MS (ESI⁺) : [M+H]⁺ 342.2. |
| **154** | 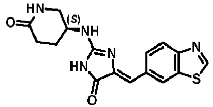 | C₁₆H₁₅N₅O₂S | 341.39 | >98% | Yellow solid. ¹H NMR (400 MHz, DMSO-*d*₆, 300K) of major tautomer δ_{H} 10.76 (bs, 1H, NH, D₂O exchanged), 9.36 (s, 1H), 8.82 (s, 1H), 8.40 - 8.20 (m, 1H), 8.09 - 8.00 (m, 1H), 7.79 (bs, 1H, NH, D₂O exchanged), 7.52 (bs, 1H, NH, D₂O exchanged), 6.44 (s, 1H), 4.38 - 3.73 (m, 1H), 3.58 - 3.39 (m, 2H), 2.36 - 1.78 (m, 4H). MS (ESI⁺) : [M+H]⁺ 342.2. |

### PATHOLOGIES

The compounds of formula (I) may be useful in the treatment and/or in the prevention of cognitive deficits associated with Down syndrome (Trisomy 21); Alzheimer's disease and related diseases; dementia; tauopathies; and other neurodegenerative diseases (Parkinson's disease; Pick disease, including Niemann-Pick Type C Disease); CDKL5 Deficiency Disorder; McDermid syndrome; autism; diabetes; regulation of folate and methionine metabolism; osteoarthritis, in particular knee osteoarthritis; Duchenne muscular dystrophy; several cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointerstinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such as Leishmaniasis and *Trypanosoma brucei* and for regulating body temperature.

According to a particular embodiment, the compounds of formula (I) of the present invention may be useful in the treatment and/or in the prevention of cognitive deficits associated with Down syndrome (Trisomy 21); Alzheimer's disease and related diseases; dementia; tauopathies; other neurodegenerative diseases (Parkinson's disease; Pick disease, including Niemann-Pick Type C Disease); CDKL5 Deficiency Disorder; diabetes; regulation of folate and methionine metabolism; osteoarthritis, in particular knee osteoarthritis; Duchenne muscular dystrophy; several cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointerstinal cancer and breast cancer, such as Triple-negative breast cancer (TNBC) and viral infections, such as caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and for regulating body temperature. Said diseases are more particularly associated with the abnormalities in DYRK1A and/or CLK1 dosage.

Still according to this particular embodiment, the compounds of formula (I) of the present invention may be useful in the treatment and/or prevention of Down syndrome, Alzheimer's disease, dementia, tauopathies, Parkinson's disease, Niemann-Pick Type C Disease, CDKL5 Deficiency Disorder and Phelan-McDermid syndrome and their associated cognitive and motor conditions, more particularly due to high expression and activity of DYRK1A.

Still according to this particular embodiment, the compounds of formula (I) of the present invention may be useful in the treatment and/or prevention of Down syndrome, Alzheimer's disease and related Tauopathies, Parkinson's disease, the cognitive/motor disorders associated therewith, or one or more symptoms of such diseases. As a typical symptom of such diseases is a decline in learning and memory and social interactions.

Still according to this particular embodiment, the compounds of formula (I) of the present invention may be useful in combatting cognitive decline associated with Down syndrome (Trisomy 21), in learning and memory, in particular associated with the cognitive or neurodegenerative disorders as mentioned above.

Still according to this particular embodiment, the compounds of formula (I) of the present invention may be useful in the treatment and/or prevention of diabetes.

Still according to this particular embodiment, the compounds of formula (I) of thre present invention may be useful in the treatment and/or prevention of viral infections, in particular caused by such as caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV), and in particular by Herpes, Coronaviruses, Cytomegaloviruses and Influenzas. These infections may be associated with high expression and activity of DYRK1A and/or CLK1, and optionally additionally with dual inhibitors of CLKs/DYRKS.

Acute respiratory disease has recently been caused by a new coronavirus (SARS-CoV-2, previously known as 2019-nCoV), also known here as coronavirus 2019 (COVID-19), which belongs to *Coronaviridae.* The compounds of formula (I) according to the present invention can also treat said infection caused by SARS-CoV-2 virus.

Still according to this particular embodiment, the compounds of formula (I) of the present invention may be useful in the treatment and/or prevention of cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointerstinal cancer and breast cancer, such as Triple-negative breast cancer (TNBC). These cancers may be associated with high expression and activity of DYRK1A and/or CLK1, and optionally additionally with dual inhibitors of CLKs/DYRKS.

Still according to this particular embodiment, the compounds of formula (I) of thre present invention may be useful in the treatment and/or prevention of osteoarthritis. Osteoarthritis may be associated with high expression and activity of DYRK1A and/or CLK2.

Still according to this particular embodiment, the compounds of formula (I) of thre present invention may be useful in the treatment and/or prevention of infections caused by unicellular parasites, such as Leishmaniasis and *Trypanosoma brucei.* Said parasitic infections may be associated with expression and activity of DYRKs.

Still according to this particular embodiment, the compounds of formula (I) of thre present invention may be useful in the regulation of the body temperature. Said body temperature regulation may be associated with expression and activity of CLKs.

According to another particular embodiment, the compounds of formula (I) of the present invention may be useful in the treatment and/or in the prevention of Phelan-McDermid syndrome; autism; further viral infections, such as caused by Hepatitis C virus, Chikungunya virus, Dengue virus, Influenza virus and Severe acute respiratory syndrome (SARS) coronavirus, Cytomegalovirus and Human papillomavirus; further cancers, such as tissue cancer, including liposarcoma, Hedgehog/GLI-dependent cancer, liver cancer, including Hepatocellular carcinoma, neuroinflammation, anemia, infections caused by unicellular parasites, such as Leishmaniasis and *Trypanosoma brucei.* Said diseases are more particularly associated with the abnormalities in other DYRKs (DYR1B, 2, 3, 4) and the closely related further cdc2-like kinases (CLKs) (CLK 2, 3, 4).

The following examples are provided as illustrations and in no way limit the scope of this invention.

The following examples illustrate in detail the preparation of some compounds according to the invention. The structures of the products obtained have been confirmed by NMR analyses and mass spectroscopy.

### Example 1: General protocol 1 - Synthesis of (5Z)-5-heteroaryl-2-thioxo-imidazolidin-4-ones

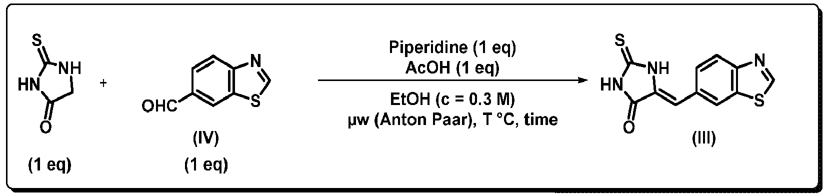

**GP1:** a stirred solution of 2-thiohydantoin (1 eq), the appropriate heteroarylcarbaldehyde (1 eq), piperidine (1 eq) and AcOH (1 eq) in EtOH (C = 0.3 M) was heated in a sealed tube in a microwave oven (Anton Paar) for the appropriate time, at the indicated temperature. Upon completion (followed by consumption of the aldehyde on TLC), the reaction medium was cooled down and added dropwise onto water. The precipitated solid was stirred for 30 min and filtered off on a fritted glass funnel, thoroughly dried, and could be used in the next step without further purification. When necessary, a final trituration in EtOH may help remove trace impurities without significant yield loss.

### Example 1.1: Synthesis of (5Z)-5-(1,3-benzothiazol-6-ylmethylene)-2-thioxo-imidazolidin-4-one (1.1)

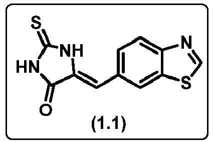

Compound (1.1) was synthesized according to **GP1:** reaction was carried out on a 4.2 mmol scale of 2-thiohydantoin, benzothiazole-6-carbaldehyde, AcOH and piperidine. Reaction temperature: 110 °C, time: 90 min. The yellow solid was triturated in EtOH after filtration. Yellow solid, 89% (978 mg). ¹H NMR (400 MHz, DMSO-*d*₆) δ_{H} 12.44 (bs, 1H, NH, D20 exchanged), 12.25 (bs, 1H, NH, D20 exchanged), 9.47 (s, 1H), 8.61 (d, *J* = 1.8 Hz, 1H), 8.09 (d, *J* = 8.5 Hz, 1H), 7.84 (dd, *J* = 8.6, 1.8 Hz, 1H), 6.64 (s, 1H). ¹³C NMR (101 MHz, DMSO-*d*₆) 5c 179.3, 165.7, 158.0, 153.1, 134.4, 129.8, 128.9, 128.1, 123.7, 123.1, 110.9. MS (ESI+): [M+H]+ 262.1.

### Example 2: General protocol 2 - S-Alkylation of (5Z)-5-heteroaryl-2-thioxo-imidazolidin-4-ones

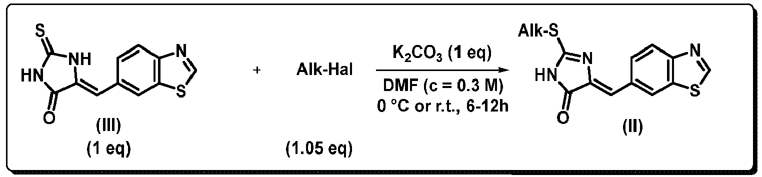

In the above scheme, Hal represents a halogen atom, in particular selected from an iodine and a bromine atom and Alk is a (C₁-C₅) alkyl.

**GP2**: The appropriate alkyliodide (1.05 eq) was added dropwise to a stirred solution of (5Z)-5-heteroaryl-2-thioxo-imidazolidin-4-one (1 eq) and K₂CO₃ (1 eq) in DMF (C = 0.3 M) at the appropriate temperature. The resulting mixture was stirred at the indicated temperature, for the appropriate time. Upon completion (TLC), the mixture was poured into water. The precipitated solid was stirred for 30 min and filtered off on a fritted glass funnel, thoroughly dried, and could be used in the next step without further purification. Trace impurities (resulting from *N*3-alkylation) may be removed by FC: elution: cyclohexane/AcOEt 7/3 to 3/7 or trituration.

### Example 2.1: Synthesis of (4Z)-4-(1,3-benzothiazol-6-ylmethylene)-2-ethylsulfanyl-1H-imidazol-5-one (2.1)

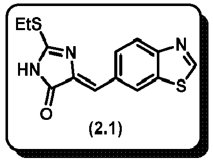

Compound (2.1) was synthesized according to **GP2:** reaction was carried out with (5*Z*)-5-(1,3-benzothiazol-6-ylmethylene)-2-thioxo-imidazolidin-4-one (7.69 mmol) and EtI, at room temperature, for 12h. Yellow solid, 89% (978 mg). ¹H NMR (400 MHz, DMSO-*d*₆) δ_{H} 11.85 (bs, 1H, NH, D20 exchanged), 9.46 (s, 1H), 8.90 (s, 1H), 8.45 (d, *J* = 8.6 Hz, 1H), 8.12 (d, *J* = 8.6 Hz, 1H), 6.88 (s, 1H), 3.70 - 3.17 (m, 2H), 1.44 (t, *J* = 7.3 Hz, 3H). ¹³C NMR (101 MHz, DMSO-*d*₆) δ_{C} 170.5, 165.0, 157.8, 153.2, 139.5, 134.1, 132.0, 129.2, 125.4, 123.0, 119.9, 24.4, 14.5. MS (ESI+): [M+H]+ 289.9.

### Example 2.2: Synthesis of (4Z)-4-(1,3-benzothiazol-6-ylmethylene)-2-methylsulfanyl-1H-imidazol-5-one (2.2)

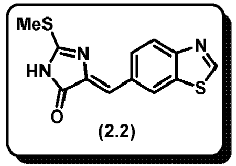

Compound (2.2) was synthesized according to **GP2:** reaction was carried out with (5*Z*)-5-(1,3-benzothiazol-6-ylmethylene)-2-thioxo-imidazolidin-4-one (3.83 mmol) and MeI, at 0 °C, for 6h. Yellow solid, 83% (879 mg). ¹H NMR (400 MHz, DMSO-*d*₆) δ_{H} 11.89 (bs, 1H, NH, D20 exchanged), 9.46 (s, 1H), 8.92 (d, *J* = 1.6 Hz, 1H), 8.45 (d, *J* = 8.6 Hz, 1H), 8.12 (d, *J* = 8.6 Hz, 1H), 6.88 (s, 1H), 2.72 (s, 3H). ¹³C NMR (101 MHz, DMSO-*d*₆) δ 171.1, 166.1, 158.3, 153.7, 140.0, 134.6, 132.4, 129.8, 125.9, 123.5, 120.4, 12.8. MS (ESI+): [M+H]+ 275.9.

### Example 3: General protocol 3 - Addition of aliphatic and aromatic amines on (4Z)-4-(1,3-benzothiazol-6-ylmethylene)-2-alkylsulfanyl-1H-imidazol-5-one

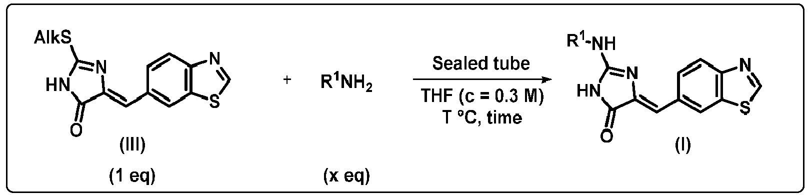

The appropriate amine^{(a)} (x eq) was added to a stirred solution of (4Z)-4-(1,3-benzothiazol-6-ylmethylene)-2-ethylsulfanyl-1*H*-imidazol-5-one^{(b)} (1 eq) in the appropriate solvent mixture (C = 0.3 M) in a sealed tube (heating block or µw). The mixture was thoroughly purged with vacuum/argon cycles and heated (µw or heating block) at the appropriate temperature for the indicated time. Upon completion (followed by consumption of the isothiourea on TLC), the mixture was brought back to room temperature.
- **GP3-A: direct precipitation of the desired product:** The reaction medium was stirred 1h at 0 °C. The precipitated solid was filtered off on a fritted-glass funnel. High purity may be achieved after filtration by washing, reprecipitation, trituration, or recrystallization.
- **GP3-B: the product failed to precipitate:** the reaction mixture was concentrated *in vacuo*, adsorbed on silica, and purified by FC. High purity may be achieved after filtration by reprecipitation, trituration, or recrystallization.
- **GP3-C: the product failed to precipitate:** the reaction mixture was concentrated *in vacuo.* The resulting crude was triturated in EtOH (at r.t. or reflux), filtered off on a fritted-glass funnel. High purity may be achieved after filtration by purification, reprecipitation, trituration, or recrystallization.
   (a) When amine hydrochlorides were used, they were quenched *in situ* with of TEA or DIPEA.
   (b) May require activation with 15 eq of AcOH, depending on the amine.

### Selected examples from sub-group A1:

### Example 3.1: Synthesis of (4Z)-4-(1,3-benzothiazol-6-ylmethylene)-2-(cyclohexylamino)-1H-imidazol-5-one (6)

Reaction was carried out according to GP3-A, in THF (0.3M), on a 2.73 mmol scale of (2.1), with 4 eq of cyclohexylamine at 110 °C (sealed tube, heating block), for 12h. The product directly precipitated in the reaction medium: it was isolated after filtration, washing with cold THF, then pentane. Isolated yield: 34%.

### Example 3.2: Synthesis of (4Z)-4-(1,3-benzothiazol-6-ylmethylene)-2-(cycloheptylmethylamino)-1H-imidazol-5-one (7)

Reaction was carried out according to GP3-B, in THF (0.3M), on a 1.01 mmol scale of (2.1), with 4 eq of cycloheptylmethylamine at 110 °C (sealed tube, heating block), for 12h. Purification by FC (elution: DCM/MeOH: 99/1 to 93/7). The final product required a reprecipitation from DCM/pentane at 0 °C. Isolated yield: 23%.

### Example 3.3: Synthesis of (4Z)-4-(1,3-benzothiazol-6-ylmethylene)-2-(cycloheptylamino)-1H-imidazol-5-one (8)

Reaction was carried out according to GP3-A, in THF (0.3M), on a 4.84 mmol scale of (2.1), with 4 eq of cycloheptylamine at 110 °C (sealed tube, heating block), for 12h. The product directly precipitated in the reaction medium: it was isolated after filtration, washing with cold THF, then pentane. Isolated yield: 49%.

### Example 3.4: Synthesis of (4Z)-4-(1,3-benzothiazol-6-ylmethylene)-2-(cyclooctylamino)-1H-imidazol-5-one (9)

Reaction was carried out according to GP3-A, in THF (0.3M), on a 2.73 mmol scale of (2.1) and 4 eq of cyclooctylamine at 110 °C (sealed tube, heating block), for 12h. The product directly precipitated in the reaction medium it was isolated after filtration, washing with cold THF, then pentane. Isolated yield: 58%.

### Example 3.5: Synthesis of (4Z)-2-(1-adamantylamino)-4-(1,3-benzothiazol-6-ylmethylene)-1H-imidazol-5-one (16)

Reaction was carried out according to GP3-B, in THF (0.3M), on a 2.54 mmol scale of (2.2), with 3 eq of 1-adamantylamine and 15 eq of AcOH, at 160 °C (sealed tube, heating block), for 24h. Purification by FC (elution: DCM/MeOH: 99/1 to 93/7). The final product required two successive triturations in refluxing EtOH. Isolated yield: 61%.

### Example 3.6: Synthesis of (4Z)-2-(2-adamantylamino)-4-(1,3-benzothiazol-6-ylmethylene)-1H-imidazol-5-one (17)

Reaction was carried out according to GP3-B, in THF (0.3M), on a 746 µmol scale of (2.2), with 4 eq of 2-adamantylamine and 15 eq of AcOH, at 170 °C (sealed tube, heating block), for 12h. Purification by FC (elution: DCM/MeOH: 99/1 to 93/7). The final product required trituration in EtOH at 0 °C. Isolated yield: 44%.

### Example 3.7: Synthesis of (4Z)-4-(1,3-benzothiazol-6-ylmethylene)-2-[(trans-5-hydroxy-2-adamantyl)amino]-1H-imidazol-5-one (19)

Reaction was carried out according to GP3-B, in THF (0.3M), on a 746 µmol scale of (2.2), with 4 eq of *trans*-4-aminoadamantan-1-ol and 15 eq of AcOH, at 170 °C (sealed tube, heating block), for 12h. Purification by FC (elution: DCM/MeOH: 99/1 to 93/7). The final product required trituration in EtOH at 0 °C. Isolated yield: 27%.

### Example 3.8: Synthesis of (4Z)-4-(1,3-benzothiazol-6-ylmethylene)-2-[(3-hydroxy-1-adamantyl)amino]-1H-imidazol-5-one (20)

Reaction was carried out according to GP3-B, in THF (0.3M), on a 746 µmol scale of (2.2), with 4 eq of 3-amino-1-adamantanol and 15 eq of AcOH, at 160 °C (sealed tube, heating block), for 12h. Purification by FC (elution: DCM/MeOH: 99/1 to 93/7). The final product required trituration in EtOH at 0 °C. Isolated yield: 59%.

### Example 3.9: Synthesis of (4Z)-4-(1,3-benzothiazol-6-ylmethylene)-2-[[(1R,2R,3R,5S)-2,6,6-trimethylnorpinan-3-yl]amino]-1H-imidazol-5-one (22)

Reaction was carried out according to GP3-B, in THF (0.3M), on a 746 µmol scale of (2.1), with 4 eq of (1*R*,2*R*,3*R*,5*S*)-3-pinanamine, at 120 °C (sealed tube, heating block), for 12h. Purification by FC (elution: DCM/MeOH: 99/1 to 93/7). The final product required a reprecipitation from DCM/pentane at 0 °C. Isolated yield: 60%.

### Example 3.10: Synthesis of (±)-(4Z)-4-(1,3-benzothiazol-6-ylmethylene)-2-(spiro[2.5]octan-2-ylamino)-1H-imidazol-5-one (25)

Reaction was carried out according to GP3-B, in THF (0.3M), on a 773 µmol scale of (2.1), with 2 eq of (±)-spiro[2.5]octan-2-amine hydrochloride and 2 eq of TEA, at 110 °C (sealed tube, heating block), for 12h. Purification by FC (elution: DCM/MeOH: 99/1 to 93/7). The final product required a reprecipitation from DCM/pentane at 0 °C. Isolated yield: 61%.

### Example 3.11: Synthesis of (4Z)-4-(1,3-benzothiazol-6-ylmethylene)-2-[[(2R)-1,7,7-trimethylnorbornan-2-yl]amino]-1H-imidazol-5-one (27)

Reaction was carried out according to GP3-B, in THF (0.3M) on a 746 µmol scale of (2.1), with 3 eq of (*R*)-(+)-bornylamine, at 150 °C (sealed tube, heating block), for 8h. Purification by FC (elution: DCM/MeOH: 99/1 to 93/7). The final product required a reprecipitation from DCM/pentane at 0 °C. Isolated yield: 34%.

### Example 3.12: Synthesis of (4Z)-4-(1,3-benzothiazol-6-ylmethylene)-2-[[(1R)-1-(hydroxymethyl)-3-methyl-butyl]amino]-1H-imidazol-5-one (34)

Reaction was carried out according to GP3-B, in THF (0.3M) on a 746 µmol scale of (2.1), with 4 eq of D-leucinol, at 110 °C (sealed tube, heating block), for 12h. Purification by FC (elution: DCM/MeOH: 99/1 to 93/7). The final product required a reprecipitation from DCM/pentane at 0 °C. Isolated yield: 39%.

### Example 3.13: Synthesis of (4Z)-4-(1,3-benzothiazol-6-ylmethylene)-2-[[(1R)-1-(methoxymethyl)-3-methyl-butyl]amino]-1H-imidazol-5-one (35)

Reaction was carried out according to GP3-B, in THF (0.3M) on a 2.91 mmol scale of (2.2), with 2.5 eq of (2*R*)-1-methoxy-4-methyl-pentan-2-amine, at 120 °C (sealed tube, heating block), for 12h. Purification by FC (elution: DCM/MeOH: 99/1 to 93/7). The final product required a reprecipitation from DCM/pentane at 0 °C. Isolated yield: 74%.

### Example 3.14: Synthesis of (4Z)-4-(1,3-Benzothiazol-6-ylmethylene)-2-[[(1S)-1-(hydroxymethyl)-3-methyl-butyl]amino]-1H-imidazol-5-one (36)

Reaction was carried out according to GP3-B, in THF (0.3M) on a 746 µmol scale of (2.1), with 4 eq of L-leucinol, at 110 °C (sealed tube, heating block), for 12h. Purification by FC (elution: DCM/MeOH: 99/1 to 93/7). The final product required a reprecipitation from DCM/pentane at 0 °C. Isolated yield: 39%.

### Example 3.15: Synthesis of (4Z)-4-(1,3-benzothiazol-6-ylmethylene)-2-[[1-(fluoromethyl)-3-methyl-butyl]amino]-1H-imidazol-5-one (40)

Reaction was carried out according to GP3-B, in THF (0.3M) on a 908 µmol scale of (2.2), with 1.2 eq of (±)-1-fluoro-4-methyl-pentan-2-amine, at 150 °C (sealed tube, heating block), for 96h. Purification by FC (elution: DCM/MeOH: 99/1 to 93/7) then PTLC. Isolated yield: 8%.

### Example 3.16: Synthesis of (±)-(4Z)-4-(1,3-benzothiazol-6-ylmethylene)-2-[[trans-2-methoxycyclopentyl]amino]-1H-imidazol-5-one (48)

Reaction was carried out according to GP3-B, in THF (0.3M) on a 272 µmol scale of (2.2), with 3 eq of (±)-*trans*-2-methoxycyclopentanamine hydrochloride and 4 eq of DIPEA, at 120 °C (sealed tube, heating block), for 7h. Purification by FC (elution: DCM/MeOH : 99/1 to 93/7). The final product required a trituration in refluxing EtOH. Isolated yield: 81%.

### Example 3.17: Synthesis of (±)-(4Z)-4-(1,3-benzothiazol-6-ylmethylene)-2-[[cis-3-hydroxycyclohexyl]amino]-1H-imidazol-5-one (55)

Reaction was carried out according to GP3-B, in THF (0.3M) on a 272 µmol scale of (2.2), with 3 eq of (±)-*cis*-3-aminocyclohexanol hydrochloride and 4 eq of DIPEA, at 120 °C (sealed tube, heating block) for 24h. Purification by FC (elution: DCM/MeOH : 99/1 to 93/7). Isolated yield: 82%.

### Example 3.18: Synthesis of (±)-(4Z)-4-(1,3-benzothiazol-6-ylmethylene)-2-[[trans-3-hydroxycyclohexyl]amino]-1H-imidazol-5-one (56)

Reaction was carried out according to GP3-B, in THF (0.3M) on a 272 µmol scale of (2.2), with 3 eq of (±)-*trans*-3-aminocyclohexanol hydrochloride and 4 eq of DIPEA, at 120 °C (sealed tube, heating block) for 24h. Purification by FC (elution: DCM/MeOH : 99/1 to 93/7) then PTLC. Isolated yield: 82%.

### Example 3.19: Synthesis of (±)-(4Z)-4-(1,3-benzothiazol-6-ylmethylene)-2-[[trans-2-methoxycyclohexyl]amino]-1H-imidazol-5-one (59)

Reaction was carried out according to GP3-B, in THF (0.3M) on a 272 µmol scale of (2.2), with 3 eq of (±)-*trans*-2-methoxycyclohexanamine hydrochloride and 4 eq of DIPEA, at 120 °C (sealed tube, heating block) for 28h. Purification by FC (elution: DCM/MeOH : 99/1 to 93/7). Isolated yield: 82%.

### Example 3.20: Synthesis of (±)-(4Z)-4-(1,3-benzothiazol-6-ylmethylene)-2-[[cis-2-hydroxycycloheptyl]amino]-1H-imidazol-5-one (61)

Reaction was carried out according to GP3-B, in THF (0.3M) on a 272 µmol scale of (2.2), with 3 eq of (±)-*cis*-2-aminocycloheptanol hydrochloride and 4 eq of DIPEA, at 120 °C (sealed tube, heating block), for 28h. Purification by FC (elution: DCM/MeOH : 99/1 to 93/7). The final product required a trituration in refluxing EtOH. Isolated yield: 56%.

### Example 3.21: Synthesis of (±)-(4Z)-4-(1,3-benzothiazol-6-ylmethylene)-2-[[trans-2-hydroxycycloheptyl]amino]-1H-imidazol-5-one (62)

Reaction was carried out according to GP3-B, in THF (0.3M) on a 272 µmol scale of (2.2), with 3 eq of (±)-*trans*-2-aminocycloheptanol, at 120 °C (sealed tube, heating block), for 6h. Purification by FC (elution: DCM/MeOH: 99/1 to 93/7). The final product required a trituration in refluxing EtOH. Isolated yield: 31%.

### Example 3.22: Synthesis of (4Z)-4-(1,3-benzothiazol-6-ylmethylene)-2-[[(1R,2R)-2-hydroxycycloheptyl]amino]-1H-imidazol-5-one (63)

Reaction was carried out according to GP3-B, in THF (0.3M) on a 746 µmol scale of (2.1), with 3 eq of (1*R*,2*R*)-2-aminocycloheptanol, at 110 °C (sealed tube, heating block) for 12h. Purification by FC (elution: DCM/MeOH: 99/1 to 93/7). The final product required a reprecipitation from DCM/pentane at 0 °C. Isolated yield: 49%.

### Example 3.23: Synthesis of (±)-(4Z)-4-(1,3-benzothiazol-6-ylmethylene)-2-[[cis-3-hydroxycycloheptyl]amino]-1H-imidazol-5-one (65)

Reaction was carried out according to GP3-B, in THF (0.3M) on a 218 µmol scale of (2.2), with 3 eq of (±)-*cis*-3-aminocycloheptanol hydrochloride and 4 eq of DIPEA, at 150 °C (sealed tube, µw) for 6h. Purification by FC (elution: DCM/MeOH: 99/1 to 93/7). Isolated yield: 46%.

### Example 3.24: Synthesis of (±)-(4Z)-4-(1,3-benzothiazol-6-ylmethylene)-2-[[trans-3-hydroxycycloheptyl]amino]-1H-imidazol-5-one (66)

Reaction was carried out according to GP3-B, in THF (0.3M) on a 272 µmol scale of (2.2), with 3 eq of (±)-*trans*-3-aminocycloheptanol hydrochloride and 4 eq of DIPEA, at 120 °C (sealed tube, heating block) for 31h. Purification by FC (elution: DCM/MeOH: 99/1 to 93/7) then PTLC. Isolated yield: 67%.

### Example 3.25: Synthesis of (±)-(4Z)-4-(1,3-benzothiazol-6-ylmethylene)-2-[[trans-2-methoxycycloheptyl]amino]-1H-imidazol-5-one (68)

Reaction was carried out according to GP3-B, in THF (0.3M) on a 272 µmol scale of (2.2), with 3 eq of (±)-*trans*-2-methoxycycloheptanamine, at 120 °C (sealed tube, heating block) for 24h. Purification by FC (elution: DCM/MeOH: 99/1 to 93/7). Isolated yield: 79%.

### Selected examples from sub-group A2:

### Example 3.26: Synthesis of (4Z)-4-(1,3-benzothiazol-6-ylmethylene)-2-(benzylamino)-1H-imidazol-5-one (74)

Reaction was carried out according to GP3-A, in THF (0.3M), on a 1.38 mmol scale of (2.1), with 4 eq of benzylamine at 110 °C (sealed tube, heating block) for 12h. The product directly precipitated in the reaction medium: it was isolated after filtration, washing with cold THF, then pentane. Isolated yield: 80%.

### Example 3.27: Synthesis of (4Z)-4-(1,3-benzothiazol-6-ylmethylene)-2-[[2-(trifluoromethyl)phenyl]methylamino]-1H-imidazol-5-one (78)

Reaction was carried out according to GP3-A, in THF (0.3M), on a 272 µmol scale of (2.2), with 3 eq of [2-(trifluoromethyl)phenyl]methanamine at 120 °C (sealed tube, heating block) for 24h. The product directly precipitated in the reaction medium: it was isolated after filtration, washing with cold THF. The final product required a trituration in refluxing EtOH. Isolated yield: 72%.

### Example 3.28: Synthesis of (±)-(4Z)-4-(1,3-benzothiazol-6-ylmethylene)-2-[[trans-2-hydroxyindan-1-yl]amino]-1H-imidazol-5-one (81)

Reaction carried out according to GP3-A, in THF (0.3M), on a 272 µmol scale of (2.2), with 3 eq of (±)-*trans*-1-aminoindan-2-ol at 120 °C (sealed tube, heating block) for 30h. The product directly precipitated in the reaction medium: it was isolated after filtration, washing with cold THF. The final product required a trituration in EtOH at r.t. Isolated yield: 56%.

### Example 3.29: Synthesis of (4Z)-4-(1,3-benzothiazol-6-ylmethylene)-2-[[(1S,2S)-2-hydroxyindan-1-yl]amino]-1H-imidazol-5-one (83)

Reaction carried out according to GP3-A, in THF (0.3M), on a 272 µmol scale of (2.2), with 3 eq of (1*S*,2*S*)-1-aminoindan-2-ol at 120 °C (sealed tube, heating block) for 40h. The product directly precipitated in the reaction medium: it was isolated after filtration, washing with cold THF. The final product required a trituration in EtOH at r.t. Isolated yield: 62%.

### Example 3.30: Synthesis of (±)-(4Z)-2-[(2-amino-1-phenyl-ethyl)amino]-4-(1,3-benzothiazol-6-ylmethylene)-1H-imidazol-5-one dihydrochloride (89)

Reaction carried out according to GP3-B, in THF (0.3M), on a 363 µmol scale of (2.2), with 3 eq of (±)-*tert*-butyl *N*-(2-amino-2-phenyl-ethyl)carbamate at 120 °C (sealed tube, heating block) for 48h. Purification by FC (elution: DCM/MeOH: 99/1 to 93/7) followed by deprotection with HCl (4M in dioxane). Isolated yield: 63%.

### Example 3.31: Synthesis of (±)-(4Z)-4-(1,3-benzothiazol-6-ylmethylene)-2-[(2-hydroxy-1-phenyl-ethyl)amino]-1H-imidazol-5-one (95)

Reaction carried out according to GP3-B, in THF (0.3M), on a 272 µmol scale of (2.2), with 3 eq of (±)-2-phenylglycinol at 150 °C (sealed tube, heating block) for 8h. Purification by FC (elution: DCM/MeOH: 99/1 to 93/7). The final product required a trituration in refluxing EtOH. Isolated yield: 37%.

### Example 3.32: Synthesis of (±)-(4Z)-4-(1,3-benzothiazol-6-ylmethylene)-2-[(2-methoxy-1-phenyl-ethyl)amino]-1H-imidazol-5-one (98)

Reaction carried out according to GP3-B, in THF (0.3M), on a 272 µmol scale of (2.2), with 3 eq of (±)-2-methoxy-1-phenyl-ethanamine at 120 °C (sealed tube, heating block) for 48h. Purification by FC (elution: DCM/MeOH: 99/1 to 93/7). The final product required a trituration in refluxing EtOH. Isolated yield: 41%.

### Example 3.33: Synthesis of (±)-(4Z)-4-(1,3-benzothiazol-6-ylmethylene)-2-[(2-hydroxy-1-phenyl-ethyl)amino]-1H-imidazol-5-one (99)

Reaction carried out according to GP3-B, in THF (0.3M), on a 272 µmol scale of (2.2), with 3 eq of (±)-2-amino-1-phenyl-ethanol hydrochloride and 4 eq of DIPEA, at 120 °C (sealed tube, heating block) for 2.5h. Purification by FC (elution: DCM/MeOH: 99/1 to 93/7). The final product required a trituration in refluxing EtOH. Isolated yield: 41%.

### Selected examples from sub-group A3:

### Example 3.34: Synthesis of (4Z)-4-(1,3-benzothiazol-6-ylmethylene)-2-[(5-methylpyrazin-2-yl)methylamino]-1H-imidazol-5-one (103)

Reaction carried out according to GP3-B, in THF (0.3M), on a 182 µmol scale of (2.2), with 3 eq of (5-methylpyrazin-2-yl)methanamine, at 80 °C (sealed tube, heating block) for 16h. Purification by FC (elution: DCM/MeOH: 99/1 to 93/7). Isolated yield: 47%.

### Example 3.35: Synthesis of (4Z)-4-(1,3-benzothiazol-6-ylmethylene)-2-[(4-methylthiazol-2-yl)methylamino]-1H-imidazol-5-one (108)

Reaction carried out according to GP3-B, in THF (0.3M), on a 182 µmol scale of (2.2), with 3 eq of (4-methylthiazol-2-yl)methanamine, at 120 °C (sealed tube, heating block) for 24h. Purification by FC (elution: DCM/MeOH: 99/1 to 93/7). The final product required a trituration in refluxing EtOH. Isolated yield: 40%.

### Selected examples from sub-group A4:

### Example 3.36: Synthesis of (4Z)-4-(1,3-benzothiazol-6-ylmethylene)-2-(tetrahydropyran-4-ylmethylamino)-1H-imidazol-5-one (113)

Reaction carried out according to GP3-B, in THF (0.3M), on a 182 µmol scale of (2.2), with 3 eq of tetrahydropyran-4-ylmethanamine, at 120 °C (sealed tube, heating block) for 2h. Purification by FC (elution: DCM/MeOH: 99/1 to 93/7). Isolated yield: 51%.

### Selected examples from sub-group A5:

### Example 3.37: Synthesis of (4Z)-4-(1,3-benzothiazol-6-ylmethylene)-2-[4-(4-methylpiperazin-1-yl)anilino]-1H-imidazol-5-one (119)

Reaction carried out according to GP3-A, in THF (0.3M), on a 182 µmol scale of (2.1), with 5 eq of 4-(4-methylpiperazin-1-yl)aniline, at 150 °C (sealed tube, µw) for 3h. The product directly precipitated in the reaction medium: it was isolated after filtration. The final product required two successive triturations in refluxing EtOH. Isolated yield: 65%.

### Example 3.38: Synthesis of (4Z)-4-(1,3-benzothiazol-6-ylmethylene)-2-[(1-methylindazol-7-yl)amino]-1H-imidazol-5-one (125)

Reaction carried out according to GP3-A, in THF (0.3M), on a 182 µmol scale of (2.2), with 5 eq of 1-methylindazol-7-amine and 15 eq AcOH, at 130 °C (sealed tube, heating block) for 5h. The product directly precipitated in the reaction medium: it was isolated after filtration. The final product required a trituration in EtOH at r.t. Isolated yield: 52%.

### Selected examples from sub-group A6:

### Example 3.39: Synthesis of (4Z)-4-(1,3-benzothiazol-6-ylmethylene)-2-(2-pyridylamino)-1H-imidazol-5-one (127)

Reaction carried out according to GP3-A, in THF (0.3M), on a 1.05 mmol scale of (2.2), with 5 eq of pyridine-2-amine and 15 eq AcOH, at 150 °C (sealed tube, µw) for 2h. The product directly precipitated in the reaction medium: it was isolated after filtration. The final product required a trituration in refluxing EtOH. Isolated yield: 41%.

### Example 3.40: Synthesis of (4Z)-4-(1,3-benzothiazol-6-ylmethylene)-2-[(1-methylpyrazol-3-yl)amino]-1H-imidazol-5-one (128)

Reaction carried out according to GP3-A, in THF (0.3M), on a 182 µmol scale of (2.2), with 5 eq of 1-methylpyrazol-3-amine, at 150 °C (sealed tube, µw) for 3h. The product directly precipitated in the reaction medium: it was isolated after filtration. The final product required a trituration in EtOH at r.t. Isolated yield: 71%.

### Selected examples from sub-group A7:

### Example 3.41: Synthesis of (4Z)-4-(1,3-benzothiazol-6-ylmethylene)-2-[[(3S)-tetrahydrofuran-3-yl]amino]-1H-imidazol-5-one (146)

Reaction carried out according to GP3-B, in THF (0.3M), on a 746 µmol scale of (2.2), with 3 eq of (3*S*)-tetrahydrofuran-3-amine, at 120 °C (sealed tube, heating block) for 12h. The product directly precipitated in the reaction medium: it was isolated after filtration. Purification by FC (elution: DCM/MeOH: 99/1 to 93/7). The final product required a reprecipitation from DCM/pentane at 0 °C. Isolated yield: 51%.

### Example 3.42: Synthesis of (4Z)-4-(1,3-benzothiazol-6-ylmethylene)-2-[[(3R)-tetrahydropyran-3-yl]amino]-1H-imidazol-5-one (147)

Reaction carried out according to GP3-B, in THF (0.3M), on a 1.04 mmol scale of (2.1), with 4 eq of (3*R*)-tetrahydropyran-3-amine hydrochloride and 6 eq of TEA, at 110 °C (sealed tube, heating block) for 12h. Purification by FC (elution: DCM/MeOH: 99/1 to 93/7). The final product required a reprecipitation from DCM/pentane at 0 °C. Isolated yield: 60%.

### Example 3.43: Synthesis of (4Z)-4-(1,3-benzothiazol-6-ylmethylene)-2-[[(3S)-tetrahydropyran-3-yl]aminol-1H-imidazol-5-one (148)

Reaction carried out according to GP3-B, in THF (0.3M), on a 746 µmol scale of (2.2), with 3 eq of (3*S*)-tetrahydropyran-3-amine hydrochloride and 4 eq of DIPEA, at 130 °C (sealed tube, heating block) for 12h. Purification by FC (elution: DCM/MeOH: 99/1 to 93/7). The final product required a reprecipitation from DCM/pentane at 0 °C. Isolated yield: 62%.

### Example 3.44: Synthesis of (4Z)-4-(1,3-benzothiazol-6-ylmethylene)-2-[[(3R,4R)-4-hydroxytetrahydropyran-3-yl]amino]-1H-imidazol-5-one (150)

Reaction carried out according to GP3-B, in THF (0.3M), on a 746 µmol scale of (2.2), with 3 eq of (3*R*,4*R*)-3-aminotetrahydropyran-4-ol, at 130 °C (sealed tube, heating block) for 12h. Purification by FC (elution: DCM/MeOH: 99/1 to 93/7). The final product required a reprecipitation from DCM/pentane at 0 °C. Isolated yield: 35%.

### Example 3.45: Synthesis of (±)-(4Z)-4-(1,3-benzothiazol-6-ylmethylene)-2-(oxepan-3-ylamino)-1H-imidazol-5-one (151)

Reaction carried out according to GP3-B, in THF (0.3M), on a 272 µmol scale of (2.2), with 1.2 eq of (±)-oxepan-3-amine, at 130 °C (sealed tube, heating block) for 12h. Purification by FC (elution: DCM/MeOH: 99/1 to 93/7). Isolated yield: 33%.

### Example 4: Biological activity

### MATERIAL AND METHODS

### PROTEIN KINASE ASSAYS

### 1. Overview

Assays were performed by ProQinase GmbH (Engesserstr. 4, D-79108 Freiburg, Germany, www.proqinase.com).The IC₅₀ profile of all compounds was determined using 12 protein kinases (CDK2/cyclin E, CK1ε, CLK1, 2, 3, 4, DYRK1A, 1B, 2, 3, 4, GSK3β). IC₅₀ values were measured by testing 10 concentrations (10 µM to 30 nM) of each compound in singlicate.

### 2. Test Compounds

The compounds were provided as 1µM stock solutions in 100% DMSO. Prior to testing, the 1 µM stock solutions were subjected to a serial, semi-logarithmic dilution using 100 % DMSO as a solvent. This resulted in 10 distinct concentrations, with a dilution endpoint of 3 x 10 nM/100 % DMSO, with 100 % DMSO as controls. In the process, 90 µl H₂O were added to each well of each compound dilution plate. To minimize potential precipitation, the H₂O was added to each plate only a few minutes before the transfer of the compound solutions into the assay plates. The plate was shaken thoroughly, resulting in a compound dilution plate/ 10 % DMSO.

For the assays (see below), 5 µL solution from each well of the compound dilution plates/10 % DMSO were transferred into the assay plates. The final volume of the assay was 50 µL. All compounds were tested at 10 final assay concentrations in the range from 10 µM to 30 nM. The final DMSO concentration in the reaction cocktails was 1 % in all cases.

### 3. Recombinant Protein Kinases

All protein kinases provided by ProQinase were expressed in Sf9 insect cells or in *E. coli* as recombinant GST-fusion proteins or His-tagged proteins, either as full-length or enzymatically active fragments. All kinases were produced from human cDNAs and purified by either GSH-affinity chromatography or immobilized metal. Affinity tags were removed from a number of kinases during purification. The purity of the protein kinases was examined by SDS-PAGE/Coomassie staining, the identity was checked by mass spectroscopy.

### 4. Protein Kinase Assay

A radiometric protein kinase assay (33PanQinase® Activity Assay) was used for measuring the kinase activity of the 12 protein kinases. All kinase assays were performed in 96-well FlashPlatesTM from PerkinElmer (Boston, MA, USA) in a 50 µL reaction volume. The reaction cocktail was pipetted in four steps in the following order:
- 25 µL of assay buffer (standard buffer/[γ-³³P]-ATP),
- 10 µL of ATP solution (in H₂O),
- 5 µL of test compound (in 10 % DMSO),
- 10 µL of enzyme/substrate mixture.

The assay for all protein kinases contained 70 mM HEPES-NaOH pH 7.5, 3 mM MgCl₂, 3 mM MnCl₂, 3 µM Na-orthovanadate, 1.2 mM DTT, 50 µg/ml PEG20000, ATP (variable concentrations, corresponding to the apparent ATP-Km of the respective kinase), [γ-³³P]-ATP (approx. 6.5 x 10-05 cpm per well), protein kinase (variable amounts), and substrate (variable amounts). The reaction cocktails were incubated at 30°C for 60 minutes. The reaction was stopped with 50 µL of 2% (v/v) H₃PO₄, plates were aspirated and washed two times with 200 µL 0.9% (w/v) NaCl. Incorporation of ³³Pi was determined with a microplate scintillation counter (Microbeta, Wallac). The IC₅₀ values for all compounds were calculated from the dose response curves

### 5. Quality controls

As a parameter for assay quality, the Z'-factor (Zhang et al., J. Biomol. Screen. 2: 67-73, 1999) for the low and high controls of each assay plate (n = 8) was used. ProQinase's criterion for repetition of an assay plate is a Z'-factor below 0.4 (Iversen et al., J. Biomol. Screen. 3: 247-252, 2006).

Their activity has been classified according to two criteria, kinase inhibition potency and kinase selectivity.

The **kinase inhibition potency classification** was done according to the following ranges of IC₅₀ values:
Some compounds have an IC₅₀ activity varying from above 0.050 µM. These compounds correspond to the above-reported class **E**. Some compounds have an IC₅₀ activity varying from 0.025 to 0.050 µM. These compounds correspond to the above-reported class **D**. Some compounds of the invention have an IC₅₀ activity varying from 0.010 to 0.025 µM. These compounds correspond to the above-reported class **C**. Further some particular compounds have an IC₅₀ activity varying from 0.005 to 0.010 µM. These compounds correspond to the above-reported class **B**. Even preferred are compounds of the invention that have an IC₅₀ activity of less than 0.005 µM. These compounds correspond to the above-reported class **A**. This classification was applied to CLK1 and DYRK1A. Said letters A to E were used to quote the activity/efficacy of the compounds of the invention in the following Table 4, 4A and 4B.

The **kinase selectivity classification** was based on comparing the IC₅₀ values of DYRK1A with that of CLK1 or with that of DYRK1B. The classification was done according to the following ranges of values: **I**: ratio of IC₅₀ on CLK1 or DYRK1B over IC₅₀ on DYRK1A ≥ 10 fold (most DYRK1A-selective compounds); **II**: ratio between 2 and 10 fold; **III**: ratio between 0.5 and 2 fold; **IV**: ratio between 0.1 and 0.5 fold; **V**: ratio > 0.1 fold (most CLK1- or DYRK1B-selective compounds). Said numbers I to V were used to quote the relative selectivity of the compounds of the invention in the following Tables.

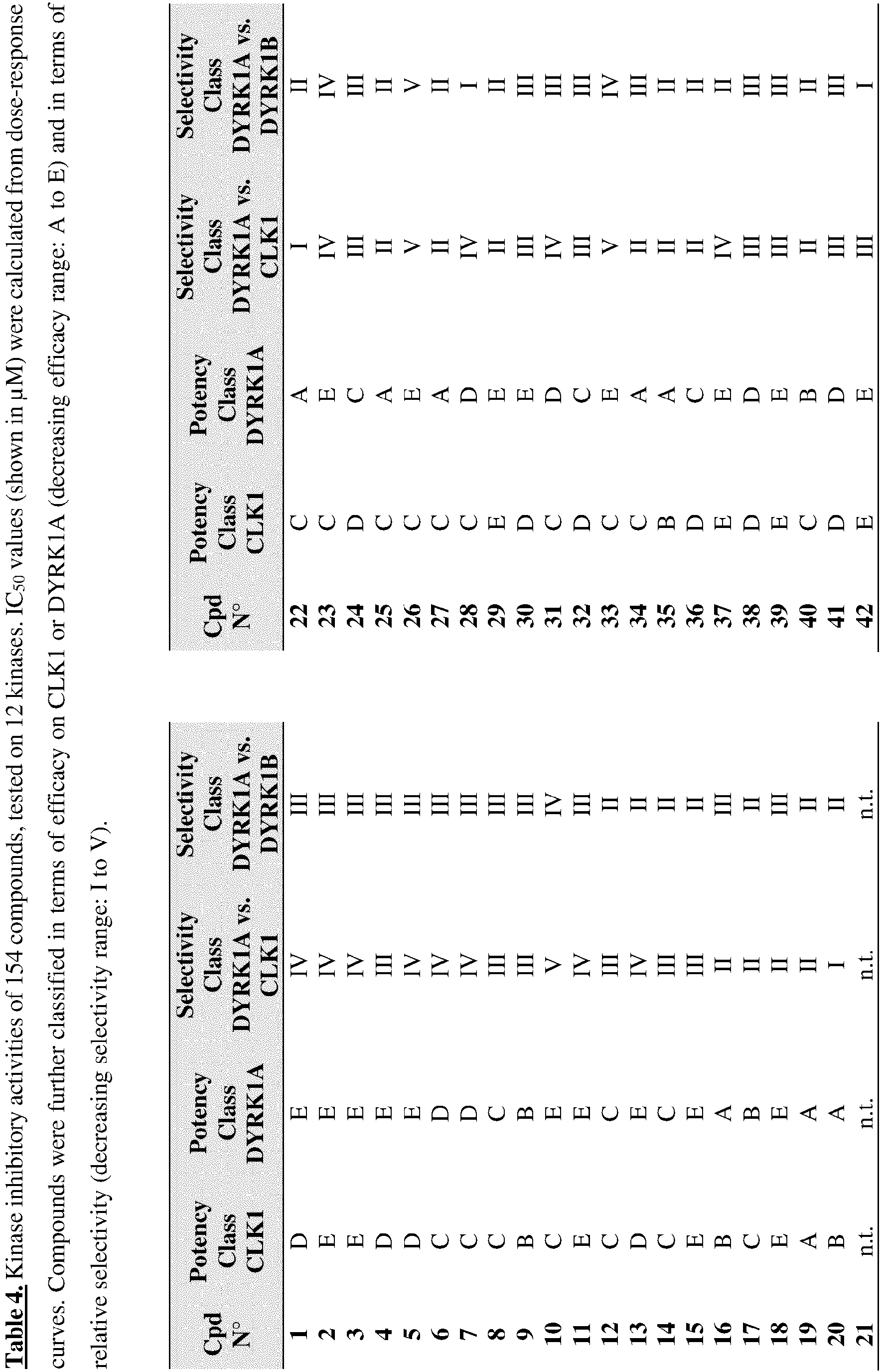

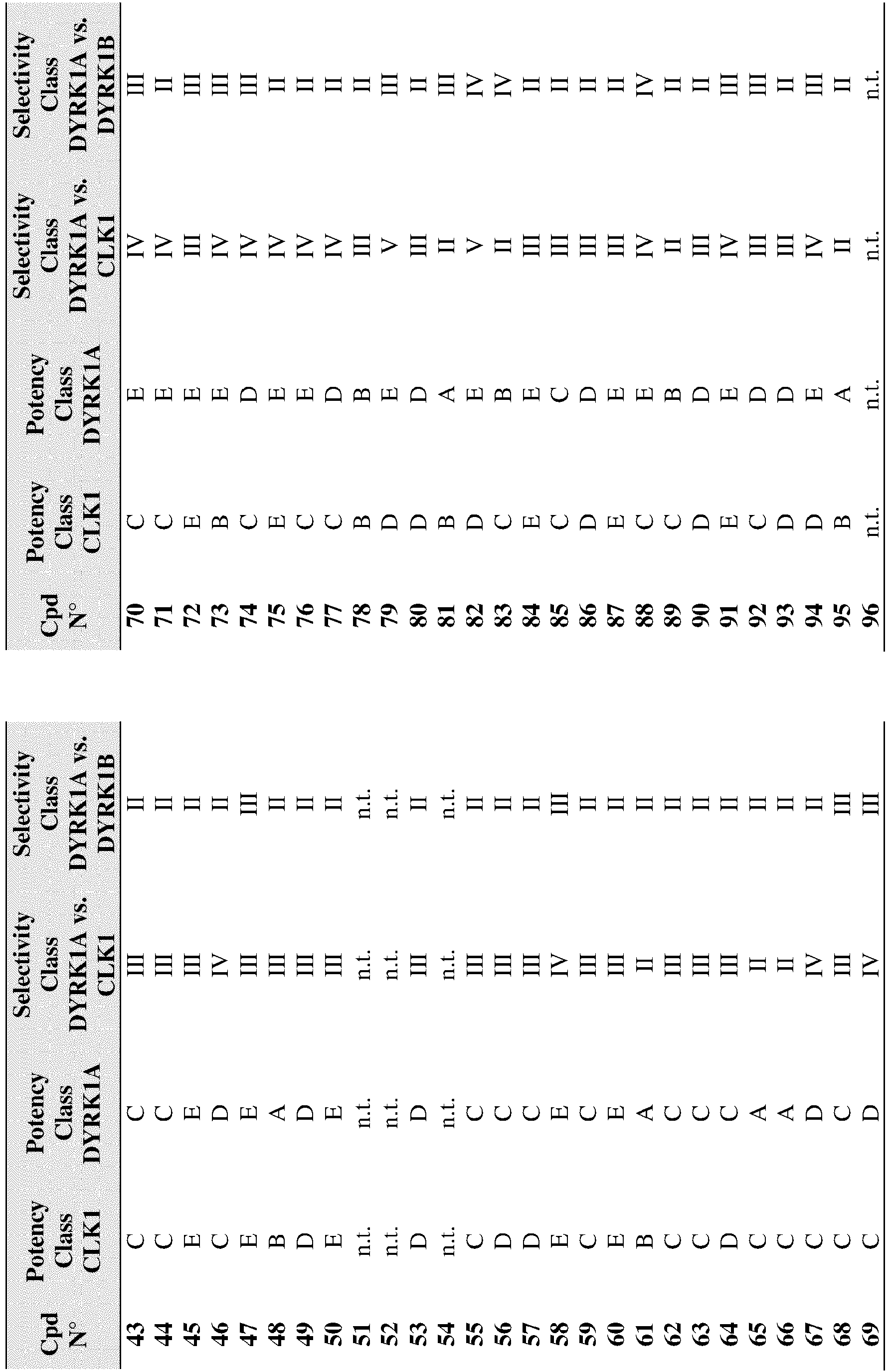

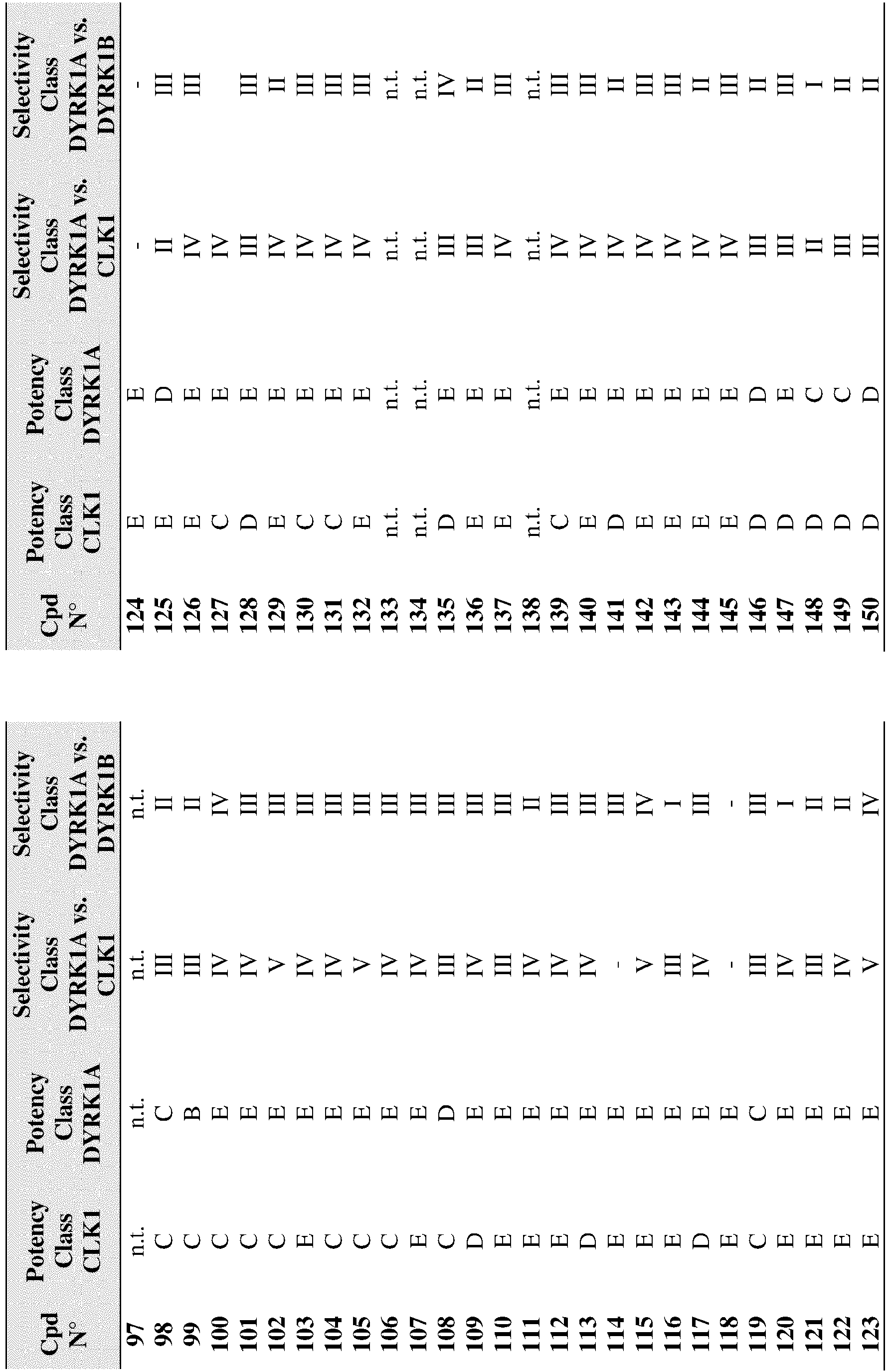

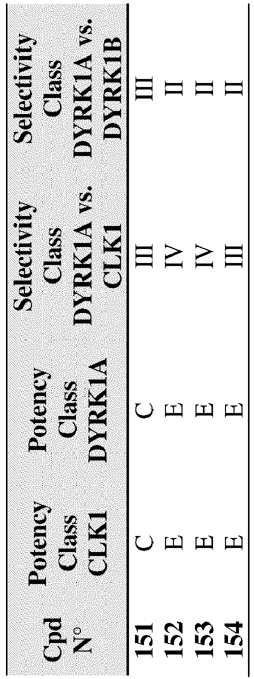

**Table 4A. Most potent CLK1 inhibitors (IC₅₀ ≤ 10 nM). Kinase inhibition potency classes: IC₅₀ values: A ≤ 0.005 µM; B ≤ 0.010 µM; C ≤ 0.025 µM; D ≤ 0.050 µM; E > 0.050 µM. Kinase selectivity classes based on DYRK1A: class I (>10 fold selectivity), class II (2-10 fold selectivity), class III (0.5-2 fold selectivity = equipotency). Some compounds display a better selectivity for CLK1 or DYRK1B compared to DYRK1A. These compounds correspond to the above-reported class IV (2-10-fold selectivity) or class V (> 10-fold selectivity).**

| **Cpd N°** | **Potency Class CLK1** | **Potency Class DYRK1A** | **Selectivity Class DYRK1A vs. CLK1** |
|---|---|---|---|
| **19** | A | A | II |
| **73** | B | E | IV |
| **78** | B | B | III |
| **20** | B | A | I |
| **61** | B | A | II |
| **95** | B | A | II |
| **16** | B | A | II |
| **35** | B | A | II |
| **48** | B | A | III |
| **81** | B | A | II |
| **9** | B | B | III |

The most potent CLK2 inhibitors, with an IC₅₀ lower or equal to 10 nM are compounds (9), (16), (20), (22), (24), (25), (81), (83), (89) and (95).

The most potent CLK3 inhibitors, with an IC₅₀ lower or equal to 100 nM are compounds (48), (89) and (90).

The most potent CLK4 inhibitors, with an IC₅₀ lower or equal to 10 nM are compounds (7), (8), (9), (10), (12), (16), (17), (19), (20), (23), (25), (26), (28), (32), (33), (34), (35), (40), (41), (43), (44), (46), (48), (55), (56), (57), (59), (61), (62), (63), (65), (66), (67), (68), (69), (70), (73), (77), (78), (81), (83), (88), (89), (90), (95), (98), (99), (100), (101), (102), (104), (105), (106), (117), (119), (127), (131), (139), (148), (149) and (151).

**Table 4B. Most potent DYRK1A inhibitors (IC₅₀ ≤ 10 nM). Kinase Inhibition Efficacy Classes and Kinase Selectivity Classes as sdescribed in Table 4A.**

| **Cpd N°** | **Potency Class CLK1** | **Potency Class DYRK1A** | **Selectivity Class DYRK1A vs. CLK1** | **Selectivity Class DYRK1A vs. DYRK1B** |
|---|---|---|---|---|
| **20** | B | A | I | II |
| **16** | B | A | II | III |
| **19** | A | A | II | II |
| **27** | C | A | II | II |
| **22** | C | A | I | II |
| **81** | B | A | II | III |
| **61** | B | A | II | II |
| **35** | B | A | II | II |
| **95** | B | A | II | II |
| **65** | C | A | II | II |
| **66** | C | A | II | II |
| **34** | C | A | II | III |
| **25** | C | A | II | II |
| **48** | B | A | III | II |
| **83** | C | B | II | IV |
| **40** | C | B | II | II |
| **9** | B | B | III | III |
| **89** | C | B | II | II |
| **17** | C | B | II | II |
| **78** | B | B | III | II |
| **99** | C | B | III | II |

The most potent DYRK1B inhibitors, with an IC₅₀ lower or equal to 10 nM are compounds (9), (16), (20), (22), (27), (34), (35), (61), (81), (83) and (95).

The most potent DYRK2 inhibitors, with an IC₅₀ lower or equal to 100 nM are compounds (16), (20), (48), (89), (90), (99), and (119).

The most potent DYRK3 inhibitors, with an IC₅₀ lower or equal to 100 nM are compounds (16), (20), (89), (90) and (119).

The most potent DYRK4 inhibitors, with an IC₅₀ lower or equal to 100 nM are compounds (20) and (48).

**Table 4C. Most DYRK1A selective inhibitors compared to CLK1. Kinase Selectivity Classes as described in Table 4A.**

| **Cpd N°** | **Selectivity Class DYRK1A vs. CLK1** |
|---|---|
| **20** | I |
| **22** | I |
| **16** | II |
| **17** | II |
| **61** | II |
| **27** | II |
| **35** | II |
| **95** | II |
| **89** | II |
| **19** | II |
| **81** | II |
| **148** | II |
| **65** | II |
| **34** | II |
| **40** | II |
| **25** | II |
| **66** | II |
| **83** | II |
| **18** | II |
| **29** | II |
| **125** | II |
| **36** | II |

**Table 4D. Most CLK1 selective inhibitors compared to DYRK1A. Kinase Selectivity Classes as described in Table 4A.**

| **Cpd N°** | **Selectivity Class DYRK1A vs. CLK1** |
|---|---|
| **79** | V |
| **115** | V |
| **123** | V |
| **82** | V |
| **10** | V |
| **33** | V |
| **102** | V |
| **26** | V |
| **105** | V |

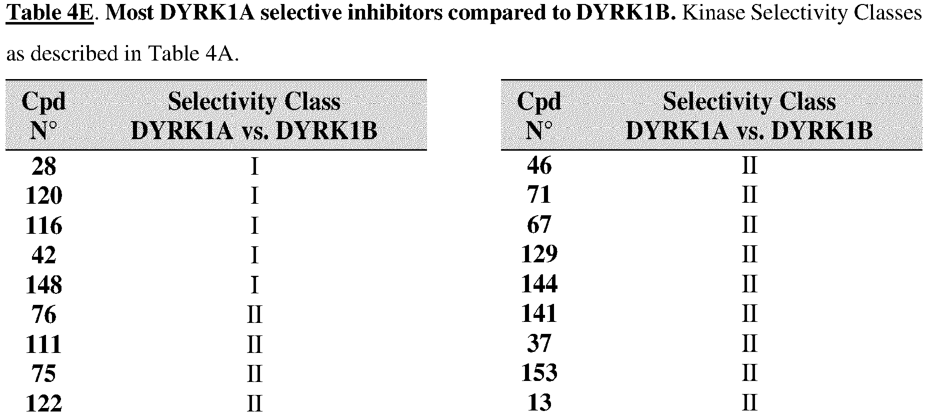

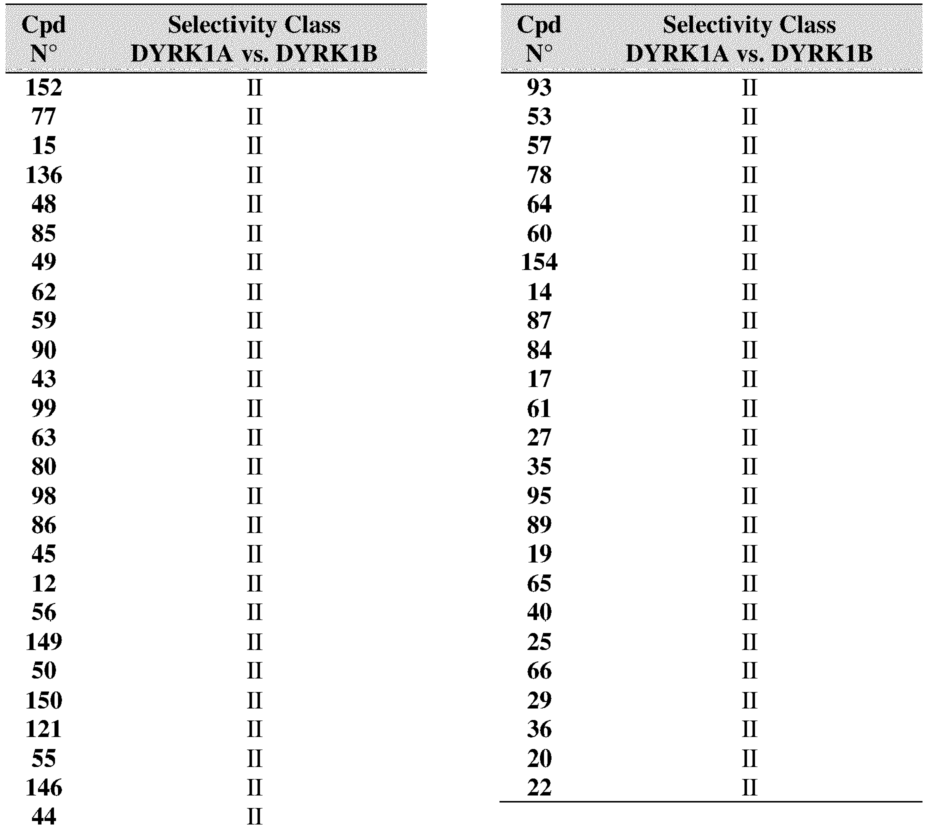

**Table 4F. Most DYRK1B selective inhibitors compared to DYRK1A. Kinase Selectivity Classes as described in Table 4A.**

| **Cpd N°** | **Selectivity Class DYRK1A vs. DYRK1B** |
|---|---|
| **26** | V |
| **115** | IV |
| **123** | IV |
| **82** | IV |
| **10** | IV |
| **33** | IV |
| **88** | IV |
| **23** | IV |
| **100** | IV |
| **135** | IV |
| **83** | IV |

### RESULTS

Most compounds of the present invention present an IC₅₀ activity of less than 2 µM on CLKs or DYRKs. All the compounds were inhibitors of DYRK1A and CLK1 (Table 4).

Compounds were preferably inhibitory on CLK1 (Table 4A), CLK4, DYRK1A (Table 4B) and DYRK1B.

Some are most selective for DYRK1A vs. CLK1 (Table 4C), DYRK1A vs. DYRK1B (Table 4E), DYRK1B vs. DYRK1A (Table 4F) or CLK1 vs. DYRK1A (Table 4D). Some compounds display a better selectivity for DYRK1A compared to CLK1 or DYRK1B. These compounds correspond to the above-reported class **I** (> 10-fold selectivity) or class **II** (2 to 10-fold selectivity). Some compounds are equipotent. These compounds correspond to the above-reported class **III** (0.5 to 2-fold selectivity). Some compounds display a better selectivity for CLK1 or DYRK1B compared to DYRK1A. These compounds correspond to the above-reported class **IV** (2 to 10-fold selectivity) or class **V** (>10-fold selectivity).

### CONCLUSION

Based on the previous results, it can be concluded that the compounds of formula (I) are suitable chemical compounds in the prevention and/or treatment of cognitive deficits associated with Down syndrome (Trisomy 21); Alzheimer's disease and related diseases; dementia; tauopathies; other neurodegenerative diseases (Parkinson's disease; Pick disease, including Niemann-Pick Type C Disease); CDKL5 Deficiency Disorder; diabetes; regulation of folate and methionine metabolism; osteoarthritis, in particular knee osteoarthritis; Duchenne muscular dystrophy; several cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointerstinal cancer and breast cancer, such as Triple-negative breast cancer (TNBC) and viral infections, such as caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and in the regulation of body temperature.

It may further be concluded that some compounds of formula (I) are further suitable for treating and/or preventing Phelan-McDermid syndrome; autism; further viral infections, such as caused by Hepatitis C virus, Chikungunya virus, Dengue virus, Influenza virus and Severe acute respiratory syndrome (SARS) coronavirus, Cytomegalovirus and Human papillomavirus; further cancers, such as tissue cancer, including liposarcoma, Hedgehog/GLI-dependent cancer, liver cancer, including Hepatocellular carcinoma, neuroinflammation, anemia, infections caused by unicellular parasites, such as Leishmaniasis and *Trypanosoma brucei.*

Leucettinibs display much increased efficacy (down to sub-micromolar and single-digit micromolar IC₅₀ values) compared to reference compounds. They also display a large range of selectivity towards DYRK1A or CLK1. And some products being equipotent on CLKs and DYRKs may also find applications as dual-specificity inhibitors.

The present invention further relates to a pharmaceutical composition comprising at least one compound of formula (I) as defined above or any of its pharmaceutically acceptable salts or at least any of compounds (1) to (154) as defined above or any of its pharmaceutically acceptable salts and also at least one pharmaceutically acceptable excipient.

Pharmaceutical compositions of the invention can contain one or more compound(s) of the invention in any form described herein.

Still a further object of the present invention consists of the use of at least one compound of formula (I) as defined above, and compounds (1) to (154) as defined above, or one of their pharmaceutically acceptable salts according to the present invention for preparing a drug for preventing and/or treating cognitive deficits associated with Down syndrome (Trisomy 21); Alzheimer's disease and related diseases; dementia; tauopathies; and other neurodegenerative diseases (Parkinson's disease; Pick disease, including Niemann-Pick Type C Disease); CDKL5 Deficiency Disorder; McDermid syndrome; autism; diabetes; regulation of folate and methionine metabolism; osteoarthritis, in particular knee osteoarthritis; Duchenne muscular dystrophy; several cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointerstinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such as Leishmaniasis and *Trypanosoma brucei* and for regulating body temperature.

Still a further object of the present invention consists of the use of at least one compound of formula (I) as defined above, and compounds (1) to (154) as defined above, or one of their pharmaceutically acceptable salts according to the present invention for preparing a drug for preventing and/or treating cognitive deficits associated with Down syndrome (Trisomy 21); Alzheimer's disease and related diseases; dementia; tauopathies; other neurodegenerative diseases (Parkinson's disease; Pick disease, including Niemann-Pick Type C Disease); CDKL5 Deficiency Disorder; diabetes; regulation of folate and methionine metabolism; osteoarthritis, in particular knee osteoarthritis; Duchenne muscular dystrophy; several cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointerstinal cancer and breast cancer, such as Triple-negative breast cancer (TNBC) and viral infections, such as caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and for regulating body temperature.

Still a further object of the present invention consists of the use of at least one compound of formula (I), as defined above, and compounds (1) to (154) as defined above, or one of their pharmaceutically acceptable salts according to the present invention for preparing a drug for combatting diabetes, viral infections, in partiuclar as mentioned above, osteoarthritis, cancers, in particular as mentioned above, infections caused by unicellular parasites and to regulate body temperature.

According to a particular embodiment, the treatment is continuous or noncontinuous.

A "continuous treatment" means a long-term treatment which can be implemented with various administration frequencies, such as once every day, every three days, once a week, or once every two weeks or once every month.

According to one embodiment, the compound of formula (I), or any of its pharmaceutically acceptable salts, is administered at a dose varying from 0.1 to 1000 mg. in particular varying from 1 to 500 mg, or for example varying from 5 to 100 mg.

Another object of the invention relates to a therapeutic method for the treatment and/or for the prevention of cognitive deficits associated with Down syndrome (Trisomy 21); Alzheimer's disease and related diseases; dementia; tauopathies; and other neurodegenerative diseases (Parkinson's disease; Pick disease, including Niemann-Pick Type C Disease); CDKL5 Deficiency Disorder; McDermid syndrome; autism; diabetes; regulation of folate and methionine metabolism; osteoarthritis, in particular knee osteoarthritis; Duchenne muscular dystrophy; several cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointerstinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such as Leishmaniasis and *Trypanosoma brucei* and for the regulation of the body temperature, in a patient in need thereof, comprising at least a step of administering a therapeutically effective amount of a compound of formula (I) or of compounds (1) to (154), as defined above. or one of their acceptable salts.

In a specific embodiment, the invention provides a use of a compound of formula (I) according to the invention or a pharmaceutically acceptable salt thereof or a pharmaceutically active derivative thereof or a method according to the invention wherein the compound of formula (I) is to be administered in combination with a co-agent useful in anyone of the hereabove mentioned diseases.

The compounds can be administered through any mode of administration such as, for example, Intramuscular, intravenous. intranasal or oral route, etc.

The inventive composition can further include one or more additives such as diluents, Excipients, stabilizers and preservatives. Such additives are well known to those skilled in the art and are described notably in "Ullmann's Encyclopedia of Industrial Chemistry, 6th Ed." (various editors, 1989-1998, Marcel Dekker) and in "Pharmaceutical Dosage Forms and Drug Delivery Systems" (ANSEL et al, 1994, WILLIAMS & WILKINS).

The aforementioned excipients are selected according to the dosage form and the desired mode of administration.

Compositions of this invention may be administered in any manner, including, but not limited to, orally, parenterally, sublingually, transdermally, vaginally, rectally, transmucosally, topically, intranasally via inhalation, via buccal or intranasal administration, or combinations thereof. Parenteral administration includes, but is not limited to, intravenous, intra-arterial, intra-peritoneal, subcutaneous, intramuscular, intra-thecal, and intra-articular. The compositions of this invention may also be administered in the form of an implant, which allows slow release of the compositions as well as a slow controlled i.v. infusion.

For example, a compound of formula (I) can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions.

In a particular embodiment, a compound of formula (I) according to the invention is administered orally.

Oral route of administration is in particular preferred in the prophylaxis or treatment aspect of the invention.

## Claims

1. A compound of formula (I) 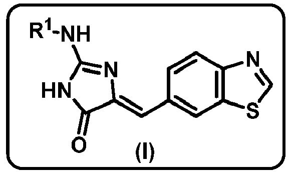 wherein R¹ represents:
(i). a (C₁-C₆)alkyl group substituted by one or two groups selected from a -COOR^{a} group, a hydroxy group, a halogen, a (C₁-C₃)alkoxy group and a benzyloxy group,
(ii). a spiro(C₅-C₁₁)bicyclic ring,
(iii). a fused phenyl group, selected from phenyl groups fused with a (C₅-C₆)cycloalkyl or (C₅-C₆)heterocycloalkyl, which (C₅-C₆)cycloalkyl and (C₅-C₆)heterocycloalkyl ring optionally comprise an insaturation and is optionally substituted by a (C₁-C₄) alkyl group, a hydroxy group, a halogen atom, a (C₁-C₃)alkoxy group or a -COR^{a} group,
(iv). a phenyl group, substituted by one or two groups selected from a (C₁-C₈)alkyl, a (C₁-C₃)fluoroalkyl, a fluoro(C₁-C₄)alkoxy group, a halogen atom, and a (C₄-C₇)heterocycloalkyl group, said (C₄-C₇)heterocycloalkyl group being itself optionally substituted by a (C₁-C₄) alkyl group, or
(v). a R'-L- group, wherein L is either a single bond or a (C₁-C₃)alkanediyl group, optionnally susbstituted by a group selected from a hydroxy group and a (C₁-C₃)alkoxy group, and
R' represents:
(v.1) a (C₃-C₈)cycloalkyl group, optionally substituted by one, two or three groups selected from a (C₁-C₄)alkyl group, a hydroxy group, a halogen atom and a (C₁-C₃) alkoxy group,
(v.2) a bridged (C₆-C₁₀)cycloalkyl group, optionally substituted by one to three groups selected from a (C₁-C₄)alkyl group, a (C₁-C₄)alkoxy group, a halogen atom and a hydroxy group,
(v.3) a (C₃-C₈)heterocycloalkyl group, optionally substituted by one or two groups selected from a -COOR^{a} group, a hydroxy group, a halogen atom, a (C₁-C₄)alkyl group and an oxo group, or
(v.4) a (C₃-C₈)heteroaryl group, optionally substituted by one to three groups selected from a halogen atom, a (C₁-C₄)alkyl group, a (C₁-C₄)alkoxy group and a *N*-methylpiperazinyl group, or
(vi). a R'-L- group wherein L is a (C₁-C₃)alkanediyl group, optionally substituted by a group selected from a -NR^{b}R^{c} group, a (C₁-C₃)alkoxy group, a hydroxy group, a -COOR^{a} group and a halogen atom, and
R' is a phenyl group, optionally substituted by one to three groups selected from the group consisting of (C₁-C₆)alkyl group, a fluoro(C₁-C₄)alkyl group and a fluoro(C₁-C₄)alkoxy group, a halogen atom and a hydroxy group,
R^{a} represents a (C₁-C₄)alkyl group or a hydrogen atom, and
R^{b} and R^{c}independently represent a (C₁-C₆)alkyl group or a hydrogen atom, or any of its pharmaceutically acceptable salt.

2. A compound of formula (I) according to claim 1, wherein R¹ represents:
(i). a (C₂-C₆)alkyl group substituted by one or two groups selected from a -COOR^{a} group, a hydroxy group, a halogen, a (C₁-C₃) alcoxy group and a benzyloxy group,
(ii). a spiro(C₇-C₉)bicyclic ring,
(iii). a fused phenyl group, selected from phenyl groups fused with a cyclopentyl or a heterocyclopentyl, which cyclopentyl and heterocyclopentyl group optionally comprise an insaturation and is optionally substituted by a (C₁-C₄) alkyl group, a hydroxy group, a halogen atom, a (C₁-C₃)alkoxy group or a -COR^{a} group
(iv). a phenyl group, substituted by one or two groups selected from (C₁-C₈)alkyl, a (C₁-C₃)fluoroalkyl, a fluoro(C₁-C₄)alkoxy group a halogen atom, and a (C₄-C₇)heterocycloalkyl group, said (C₄-C₇)heterocycloalkyl group being itself optionally substituted by a (C₁-C₄) alkyl group, or
(v). a R'-L- group wherein
• L is either a single bond or a (C₁-C₃)alkanediyl group, optionnally susbstituted by a group selected from a hydroxy group and a (C₁-C₃)alkoxy group, and
• R' represents:
(v.1) a (C₃-C₈)cycloalkyl group, optionally substituted by one, two or three groups selected from a (C₁-C₄)alkyl group, a hydroxy group and a (C₁-C₃) alkoxy group,
(v.2) a bridged (C₇-C₁₀)cycloalkyl group, optionally substituted by one to three groups selected from a (C₁-C₄)alkyl group, a (C₁-C₄)alkoxy group, a hydroxy group and a halogen atom,
(v.3) a (C₄-C₇)heterocycloalkyl group, optionally substituted by one or two groups selected from a -COOR^{a} group, a hydroxy group, a halogen atom, a (C₁-C₄)alkyl group and a oxo group, or
(v.4) a heteroaryl group, optionally substituted by one to three groups selected from a halogen atom, (C₁-C₄)alkyl group, a (C₁-C₄)alkoxy group, a *N*-methylpiperazinyl group,
(vi). a R'-L- group wherein
• L is a (C₁-C₃)alkanediyl group, optionally substituted by a group selected from a -NR^{b}R^{c} group, a (C₁-C₃)alkoxy group, a hydroxy group, a -COOR^{a} group and a halogen atom, and
• R' is a phenyl group, optionally substituted by one to three groups selected from the group consisting of (C₁-C₆)alkyl group, a fluoro(C₁-C₄)alkyl group and a fluoro(C₁-C₄)alkoxy group, a hydroxy group and a halogen atom,
R^{a} representing a (C₁-C₄)alkyl group or a hydrogen atom, and
R^{b} and R^{c} independently represent a (C₁-C₆)alkyl group or a hydrogen atom, or any of its pharmaceutically acceptable salt.

3. A compound of formula (I) according to any of claims 1 or 2, wherein R¹ represents:
(i). a (C₂-C₆)alkyl group substituted by one or two groups selected from a -COOCH3 group, a hydroxy group, a fluorine atom, a methoxy group and a benzyloxy group,
(ii). a spiro(C₇-C₈)bicyclic ring, in particular a spiro[3.3]heptyl, a spiro[2.5]octanyl or a 7-azaspiro[3.5]nonyl,
(iii). a fused phenyl group, chosen from phenyl groups fused with a cyclopentyl or a heterocyclopentyl, which cyclopentyl and heterocyclopentyl group optionally comprise an insaturation and is optionally substituted by a methyl, a hydroxy group, a methoxy group and a -COCH₃ group,
(iv). a phenyl group, substituted by one or two groups selected from a methyl, a hexyl, a trifluoromethyl, a difluoromethoxy group, a halogen atom, in particular a fluor atom, a morpholino group and a *N-*methylpiperazinyl group, or
(v). a R'-L- group wherein L is either a single bond or a (C₁-C₃)alkanediyl group, optionnally susbstituted by a group chosen from a hydroxy group and a (C₁-C₃)alkoxy group,
and R' is selected from the group consisting of:
(v.1). a (C₃-C₈)cycloalkyl group in particular a cyclopropyl, a cyclobutyl, a cyclopentyl, a cyclohexyl, a cycloheptyl or a cyclooctyl, optionally substituted by one, two or three groups selected from a methyl, an isopropyl, a hydroxy group and a methoxy group,
(v.2). a bridged (C₇-C₁₀)cycloalkyl group, in particular an adamantyl or a bicyclo[3.1.1]heptyl, optionally substituted by one to three groups selected from methyl, methoxy and hydroxy group,
(v.3). a (C₅-C₈)heterocycloalkyl group, in particular a tetrahydropyranyl, a piperidinyl, an oxetanyl, a tetrahydrofuranyl or an oxepanyl, optionally substituted by a group selected from a -COOR^{d} group, a hydroxy group, a methyl group, wherein R^{d} represents either an ethyl or an isopropyl group,
(v.4). a heteroaryl group, in particular a pyrimidinyl, a pyridinyl, a thiazolyl, a imidazolyl, a pyrazolyl, a thiadiazolyl, a pyridazinyl, a pyrazinyl, a furyl, optionally substituted by one to three groups selected from methyl group, a methoxy group and a *N-*methylpiperazinyl group, or
(vi). a R'-L- group wherein L is a (C₁-C₃)alkanediyl group, optionally substituted by a group selected from the group consisting of a -NR^{b}R^{c} group, a (C₁-C₃)alkoxy group, a hydroxy group, a -COOR^{a} group and a halogen atom, in particular a fluor atom, and
R' is a phenyl group, optionally substituted by one or two groups selected from the group consisting of methyl group, a trifluoromethyl group and a trifluromethoxy group,
R^{a} representing a (C₁-C₃)alkyl group, and
R^{b} and R^{c} are independently chosen from a methyl group or a hydrogen atom, or any of its pharmaceutically acceptable salts.

4. A compound of formula (I) according to any of the preceding claims, wherein L is selected from a group consisting of a -CH₂- group, a -CH(CH3)- group, a -CH(CH₂OH)-CH₂- group, a -CH(CH₂OH)- group, a -CH(CH₂OCH₃)- group, a -CH(OH)-CH₂- group, a -CH₂-CH(CH₂OCH₃)- group, a -CH(OCH₃)-CH₂- group, a -CH₂-CH(COOCH₃)- group, a -CH(CH₂F)- group, a -CH(CH₂NH₂)- group, a -CH(CH₂NHCH₃)- group, a -CH(CH₂N(CH₃)₂)- group, a -CH₂-CH(CH₂OH)- group, a -CH(OCH₃)-CH₂- group, a -CH₂-CH(OCH₃)- group, a -CH₂-CH(OH)-CH₂- group, a -CH₂-CH(OCH₃)-CH₂ group, a -(CH2)3- group and a -(CH2)2- group, or any of its pharmaceutically acceptable salts.

5. A compound of formula (I) according to any of the preceding claims, wherein:
(v.1). when R' is a (C₃-C₈)cycloalkyl group, L is selected from the group consisting of a -CH₂- group, a -CH(CH₃)- group, a -CH(CH₂OH)-CH₂- group, a -CH(CH₂OH)- group, a -CH(CH₂OCH₃)-group and a -CH(OH)-CH₂- group and a -CH(OCH₃)-CH₂- group,
(v.2). when R' is a bridged (C₇-C₁₀)cycloalkyl group, L is a -CH₂-group or a -CH(CH3)- group,
(v.3). when R' is a (C₅-C₈)heterocycloalkyl group including spiro(C₃-C₈)heterocycloalkyls, L is a -CH₂- group,
(v.4). when R' is a phenyl, L is selected from the group consisting of a -CH₂- group, a -CH₂-CH(COOCH₃)- group, a -CH(CH₂F)- group, a -CH(CH₂NH₂)- group, a -CH(CH₂NHCH₃)- group, a -CH(CH₂N(CH₃)₂)-group, a -CH₂-CH(CH₂OH)- group, a -CH(CH₂OH)- group, a -CH(CH₂OCH₃)- group, a -CH(OH)-CH₂- group, a -CH₂-CH(CH₂OCH₃)-group, a -CH₂-CH(OH)-CH₂- group and a -CH₂-CH(OCH₃)-CH₂ group,
(v.5). when R' is a heteroaryl group, L is selected from the group comprising a -CH₂- group, a -(CH₂)₃- group and a -(CH₂)₂- group.

6. A compound of formula (I) according to any of the preceding claims, wherein R¹ represents:
- a (C₁-C₆)alkyl group substituted by one or two groups selected from a -COOR^{a} group, a hydroxy group, a fluorine atom, a (C₁-C₃)alkoxy group and a benzyloxy group,
- a spiro(C₅-C₁₁)bicyclic ring, or
- a R'-L- group, wherein
∘ L is either a single bond or a (C₁-C₃)alkanediyl group, optionnally susbstituted by a group chosen from a hydroxy group and a (C₁-C₃)alkoxy group, and
∘ R' is selected from the group consisting of:
▪ a (C₃-C₈)cycloalkyl group, optionally substituted by one, two or three groups selected from a halogen atom, a (C₁-C₄)alkyl group, a hydroxy group and a (C₁-C₃) alkoxy group, and
▪ a bridged (C₆-C₁₀)cycloalkyl group, optionally substituted by one to three groups selected from a (C₁-C₄)alkyl group, a (C₁-C₄)alkoxy group, a halogen atom and a hydroxy group.
R^{a} representing a (C₁-C₄)alkyl group,
or any of its pharmaceutically acceptable salt.

7. A compound of formula (I) according to any of claims 1 to 5, wherein R¹ represents:
- a fused phenyl group, chosen from phenyl groups fused with a (C₅-C₆)cycloalkyl or (C₅-C₆)heterocycloalkyl, which (C₅-C₆)cycloalkyl and (C₅-C₆)heterocycloalkyl group optionally comprise an insaturation and is optionally substituted by a (C₁-C₄) alkyl group, a hydroxy group, a halogen atom, a (C₁-C₃)alkoxy group and a -COR^{a} group,
- a phenyl group, substituted by one or two groups selected from (C₁-C₈)alkyl, a (C₁-C₃)fluoroalkyl, a fluoro(C₁-C₄)alkoxy group a halogen atom, and a (C₄-C₇)heterocycloalkyl group said (C₄-C₇)heterocycloalkyl group being itself optionally substituted by a (C₁-C₄) alkyl group, or
- a R'-L- group, wherein
∘ L is a (C₁-C₃)alkanediyl group, optionnally susbstituted by a group chosen from a hydroxy group, a (C₁-C₃)alkoxy group, a -NR^{b}R^{c} group, a -COOR^{a} group and a halogen atom, and
∘ R' is a phenyl group, optionally substituted by one to three groups selected from the group consisting of (C₁-C₆)alkyl group, a fluoro(C₁-C₄)alkyl group and a fluoro(C₁-C₄)alkoxy group, a halogen atom and a hydroxy group,
wherein R^{a} is a (C₁-C₄)alkyl or a hydrogen atom and R^{b} and R^{c} are independently chosen from (C₁-C₆)alkyl and a hydrogen atom or any pharmaceutically acceptable salt thereof.

8. A compound of formula (I) according to any of claims 1 to 5, wherein R¹ represents a R'-L- group wherein
- R' is a (C₃-C₈)heteroaryl group, optionally substituted by one to three groups selected from a halogen atom, a (C₁-C₄)alkyl group, a (C₁-C₄)alkoxy group and a *N*-methylpiperazinyl group, and
- L is a (C₁-C₃)alkanediyl or a single bond,
or any pharmaceutically acceptable salt thereof.

9. A compound of formula (I) according to any of claims 1 to 5, wherein R¹ represents a R'-L- group wherein
- R' is a (C₃-C₅)heteroalkyl group, optionally substituted by one to two groups selected from a hydroxyl group, a (C₁-C₄)alkyl group and a -COOR^{a} group wherein R^{a} is as defined in claim 1, and
- L is a methylene or a single bond,
or any pharmaceutically acceptable salt thereof.

10. A compound of formula (I) according to anyone of claims 1 to 5 selected from:
(1). (4Z)-4-(1,3-Benzothiazol-6-ylmethylene)-2-(cyclopropylmethylamino)-1*H*-imidazol-5-one.
(2). (4Z)-4-(1,3-Benzothiazol-6-ylmethylene)-2-(cyclopropylamino)-1*H-*imidazol-5-one.
(3). (4Z)-4-(1,3-Benzothiazol-6-ylmethylene)-2-(cyclobutylamino)-1*H-*imidazol-5-one.
(4). (4Z)-4-(1,3-Bcnzothiazol-6-ylmethylene)-2-(cyclopentylamino)-1*H-*imidazol-5-one.
(5). (4Z)-4-(1,3-Benzothiazol-6-ylmethylene)-2-(cyclohexylmethylamino)-1*H*-imidazol-5-one.
(6). (4Z)-4-(1,3-Benzothiazol-6-ylmethylene)-2-(cyclohexylamino)-1*H-*imidazol-5-one.
(7). (4Z)-4-(1,3-Benzothiazol-6-ylmethylene)-2-(cycloheptylmethylamino)-1*H*-imidazol-5-one.
(8). (4Z)-4-(1,3-Benzothiazol-6-ylmethylene)-2-(cycloheptylamino)-1*H-*imidazol-5-one.
(9). (4Z)-4-(1,3-Benzothiazol-6-ylmethylene)-2-(cyclooctylamino)-1*H-*imidazol-5-one.
(10). (4Z)-4-(1,3-Benzothiazol-6-ylmethylene)-2-[(3-hydroxy-2,2-dimethylpropyl)amino]-1*H-*imidazol-5-one.
(11). (4Z)-4-(1,3-Benzothiazol-6-ylmethylene)-2-(2-benzyloxyethylamino)-1*H*-imidazol-5-one.
(12). (±)-(4Z)-4-(1,3-Benzothiazol-6-ylmethylene)-2-[[*cis*-2-methylcyclohexyl]amino]-1*H*-imidazol-5-one.
(13). (4Z)-4-(1,3-Benzothiazol-6-ylmethylene)-2-[[(1*R*)-1-cyclohexylethyl]amino]-1*H*-imidazol-5-one.
(14). (4Z)-2-(1-Adamantylmethylamino)-4-(1,3-benzothiazol-6-ylmethylene)-1*H-*imidazol-5-one.
(15). (±)-(4Z)-2-[1-(1-Adamantyl)ethylamino]-4-(1,3-benzothiazol-6-ylmethylene)-1*H-*imidazol-5-one.
(16). (4Z)-2-(1-Adamantylamino)-4-(1,3-benzothiazol-6-ylmethylene)-1*H-*imidazol-5-one.
(17). (4Z)-2-(2-Adamantylamino)-4-(1,3-benzothiazol-6-ylmethylene)-1*H-*imidazol-5-one.
(18). (4Z)-4-(1,3-Benzothiazol-6-ylmethylene)-2-[[3,5-dimethyl-1-adamantyl]amino]-1*H*-imidazol-5-one.
(19). (4Z)-4-(1,3-Benzothiazol-6-ylmethylene)-2-[(trans-5-hydroxy-2-adamantyl)amino]-1*H*-imidazol-5-one.
(20). (4Z)-4-(1,3-Benzothiazol-6-ylmethylene)-2-[(3-hydroxy-1-adamantyl)amino]-1*H-*imidazol-5-one.
(21). (4Z)-4-(1,3-Benzothiazol-6-ylmethylene)-2-[(3-methoxy-1-adamantyl)amino]-1*H*-imidazol-5-one.
(22). (4Z)-4-(1,3-Benzothiazol-6-ylmethylene)-2-[[(1*R*,2*R*,3*R*,5*S*)-2,6,6-trimethylnorpinan-3-yl]amino]-1*H*-imidazol-5-one.
(23). (4Z)-4-(1,3-Benzothiazol-6-ylmethylene)-[[(1*S*,2*S*,3*S*,5*R*)-2,6,6-trimethylnorpinan-3-yl]amino]-1*H*-imidazol-5-one.
(24). (4Z)-4-(1,3-Benzothiazol-6-ylmethylene)-2-[[(1*R*,2*R*,5*R*)-6,6-dimethylnorpinan-2-yl]methylamino]-1*H*-imidazol-5-one.
(25). (±)-(4Z)-4-(1,3-Benzothiazol-6-ylmethylene)-2-(spiro[2.5]octan-2-ylamino)-1*H*-imidazol-5-one.
(26). (4Z)-4-(1,3-Benzothiazol-6-ylmethylene)-2-(spiro[3.3]heptan-2-ylamino)-1*H*-imidazol-5-one.
(27). (4Z)-4-(1,3-Benzothiazol-6-ylmethylene)-2-[[(2*R*)-1,7,7-trimethylnorbornan-2-yl]amino]-1*H-*imidazol-5-one.
(28). (±)-(4Z)-4-(1,3-Benzothiazol-6-ylmethylene)-2-(norbornan-2-ylamino)-1*H*-imidazol-5-one.
(29). (4Z)-4-(1,3-Benzothiazol-6-ylmethylene)-2-[[(1*R*,2*S*,5*R*)-2-isopropyl-5-methyl-cyclohexyl]amino]-1*H*-imidazol-5-one.
(30). (4Z)-4-(1,3-Benzothiazol-6-ylmethylene)-2-[[(1*R*)-1-(cyclohexylmethyl)-2-hydroxy-ethyl]amino]-1*H*-imidazol-5-one.
(31). (4Z)-4-(1,3-Benzothiazol-6-ylmethylene)-2-[[(1*R*)-1-(cyclopentylmethyl)-2-hydroxy-ethyl]amino]-1*H*-imidazol-5-one.
(32). (4Z)-4-(1,3-Benzothiazol-6-ylmethylene)-2-[[(1*R*)-1-(cyclobutylmethyl)-2-hydroxy-ethyl]amino]-1*H*-imidazol-5-one.
(33). (4Z)-4-(1,3-Benzothiazol-6-ylmethylene)-2-[[(1*R*)-1-(cyclopropylmethyl)-2-hydroxy-ethyl]amino]-1*H*-imidazol-5-one.
(34). (4Z)-4-(1,3-Benzothiazol-6-ylmethylene)-2-[[(1*R*)-1-(hydroxymethyl)-3-methyl-butyl]amino]-1*H*-imidazol-5-one.
(35). (4Z)-4-(1,3-Benzothiazol-6-ylmethylene)-2-[[(1*R*)-1-(methoxymethyl)-3-methyl-butyl]amino]-1*H*-imidazol-5-one.
(36). (4Z)-4-(1,3-Benzothiazol-6-ylmethylene)-2-[[(1*S*)-1-(hydroxymethyl)-3-methyl-butyl]amino]-1*H*-imidazol-5-one.
(37). (4Z)-4-(1,3-Benzothiazol-6-ylmethylene)-2-[[(1*S*)-1-(methoxymethyl)-3-methyl-butyl]amino]-1*H*-imidazol-5-one.
(38). (4Z)-4-(1,3-Benzothiazol-6-ylmethylene)-2-[[(1*R*)-1-(hydroxymethyl)propyl]amino]-1*H*-imidazol-5-one.
(39). (4Z)-4-(1,3-Benzothiazol-6-ylmethylene)-2-[[(1*S*)-1-(hydroxymethyl)propyl]amino]-1*H*-imidazol-5-one.
(40).(±)-(4Z)-4-(1,3-Benzothiazol-6-ylmethylene)-2-[[1-(fluoromethyl)-3-methyl-butyl]amino]-1*H*-imidazol-5-one.
(41). (±)-(4Z)-4-(1,3-Benzothiazol-6-ylmethylene)-2-[(1-cyclohexyl-2-hydroxy-ethyl)amino]-1*H*-imidazol-5-one.
(42). (±)-(4Z)-4-(1,3-Benzothiazol-6-ylmethylene)-2-[(1-cyclohexyl-2-methoxy-ethyl)amino]-1*H*-imidazol-5-one.
(43). (±)-(4Z)-4-(1,3-Benzothiazol-6-ylmethylene)-2-[(2-cyclohexyl-2-hydroxy-ethyl)amino]-1*H*-imidazol-5-one.
(44). (±)-(4Z)-4-(1,3-Benzothiazol-6-ylmethylene)-2-[(2-cyclohexyl-2-methoxy-ethyl)amino]-1*H*-imidazol-5-one.
(45). (±)-(4Z)-4-(1,3-Benzothiazol-6-ylmethylene)-2-[[*cis*-2-hydroxycyclopentyl]amino]-1*H*-imidazol-5-one.
(46). (±)-(4Z)-4-(1,3-Benzothiazol-6-ylmethylene)-2-[[*trans*-2-hydroxycyclopentyl]amino]-1*H*-imidazol-5-one.
(47). (±)-(4Z)-4-(1,3-Benzothiazol-6-ylmethylene)-2-[[*cis*-2-methoxycyclopentyl]amino]-1*H*-imidazol-5-one.
(48). (±)-(4Z)-4-(1,3-Benzothiazol-6-ylmethylene)-2-[[*trans*-2-methoxycyclopentyl]amino]-1*H*-imidazol-5-one.
(49). (±)-(4Z)-4-(1,3-Benzothiazol-6-ylmethylene)-2-[[*cis*-2-hydroxycyclohexyl]amino]-1*H*-imidazol-5-one.
(50). (±)-(4Z)-4-(1,3-Benzothiazol-6-ylmethylene)-2-[[trans-2-hydroxycyclohexyl]amino]-1*H*-imidazol-5-one.
(51). (4Z)-4-(1,3-Benzothiazol-6-ylmethylene)-2-[[(1*R*,2*S*)-2-hydroxycyclohexyl]amino]-1*H*-imidazol-5-one.
(52). (4Z)-4-(1,3-Benzothiazol-6-ylmethylene)-2-[[(1*S*,2*R*)-2-hydroxycyclohexyl]amino]-1*H*-imidazol-5-one.
(53). (4Z)-4-(1,3-Benzothiazol-6-ylmethylene)-2-[[(1*R*,2*R*)-2-hydroxycyclohexyl]amino]-1*H*-imidazol-5-one.
(54). (4Z)-4-(1,3-Benzothiazol-6-ylmethylene)-2-[[(1*S*,2*S*)-2-hydroxycyclohexyl]amino]-1*H*-imidazol-5-one.
(55). (±)-(4Z)-4-(1,3-Benzothiazol-6-ylmethylene)-2-[[*cis*-3-hydroxycyclohexyl]amino]-1*H*-imidazol-5-one.
(56). (±)-(4Z)-4-(1,3-Benzothiazol-6-ylmethylene)-2-[[*trans*-3-hydroxycyclohexyl]amino]-1*H*-imidazol-5-one.
(57). (4Z)-4-(1,3-Benzothiazol-6-ylmethylene)-2-[(*trans*-4-hydroxycyclohexyl)amino]-1*H*-imidazol-5-one.
(58). (±)-(4Z)-4-(1,3-Benzothiazol-6-ylmethylene)-2-[[*cis*-2-methoxycyclohexyl]amino]-1*H*-imidazol-5-one.
(59). (±)-(4Z)-4-(1,3-Benzothiazol-6-ylmethylene)-2-[[*trans*-2-methoxycyclohexyl]amino]-1*H*-imidazol-5-one.
(60). (4Z)-4-(1,3-Benzothiazol-6-ylmethylene)-2-[(*trans*-4-methoxycyclohexyl)amino]-1*H*-imidazol-5-one.
(61). (±)-(4Z)-4-(1,3-Benzothiazol-6-ylmethylene)-2-[[*cis*-2-hydroxycycloheptyl]amino]-1*H*-imidazol-5-one.
(62). (±)-(4Z)-4-(1,3-Benzothiazol-6-ylmethylene)-2-[[trans-2-hydroxycycloheptyl]amino]-1*H*-imidazol-5-one.
(63). (4Z)-4-(1,3-Benzothiazol-6-ylmethylene)-2-[[(1*R*,2*R*)-2-hydroxycycloheptyl]amino]-1*H*-imidazol-5-one.
(64). (4Z)-4-(1,3-Benzothiazol-6-ylmethylene)-2-[[(1*S*,2*S*)-2-hydroxycycloheptyl]amino]-1*H*-imidazol-5-one.
(65). (±)-(4Z)-4-(1,3-Benzothiazol-6-ylmethylene)-2-[[*cis*-3-hydroxycycloheptyl]amino]-1*H*-imidazol-5-one.
(66). (±)-(4Z)-4-(1,3-Benzothiazol-6-ylmethylene)-2-[[*trans*-3-hydroxycycloheptyl]amino]-1*H*-imidazol-5-one.
(67). (±)-(4Z)-4-(1,3-Benzothiazol-6-ylmethylene)-2-[[*cis*-2-methoxycycloheptyl]amino]-1*H*-imidazol-5-one.
(68). (±)-(4Z)-4-(1,3-Benzothiazol-6-ylmethylene)-2-[[*trans*-2-methoxycycloheptyl]amino]-1*H*-imidazol-5-one.
(69). Methyl (2*S*)-2-[[(4Z)-4-(1,3-benzothiazol-6-ylmethylene)-5-oxo-1*H-*imidazol-2-yl]amino]-3-methyl-butanoate.
(70). Methyl (2*S*)-2-[[(4Z)-4-(1,3-benzothiazol-6-ylmethylene)-5-oxo-1*H-*imidazol-2-yl]amino]propanoate.
(71). Methyl (2*S*)-2-[[(4*Z*)-4-(1,3-benzothiazol-6-ylmethylene)-5-oxo-1*H-*imidazol-2-yl]amino]-4-methyl-pentanoate.
(72). Methyl (2*R*)-2-[[(4Z)-4-(1,3-benzothiazol-6-ylmethylene)-5-oxo-1*H-*imidazol-2-yl]amino]-4-methyl-pentanoate.
(73). Methyl (2*S*)-2-[[(4Z)-4-(1,3-benzothiazol-6-ylmethylene)-5-oxo-1*H-*imidazol-2-yl]amino]-3-hydroxy-butanoate.
(74). (4Z)-4-(1,3-Benzothiazol-6-ylmethylene)-2-(benzylamino)-1*H-*imidazol-5-one.
(75). (4Z)-4-(1,3-Benzothiazol-6-ylmethylene)-2-(indan-2-ylamino)-1*H-*imidazol-5-one.
(76). (4Z)-4-(1,3-Benzothiazol-6-ylmethylene)-2-[(3,4-dimethylphenyl)methylamino]-1*H*-imidazol-5-one.
(77). (4Z)-4-(1,3-Benzothiazol-6-ylmethylene)-2-[(2,4-dimethylphenyl)methylamino]-1*H*-imidazol-5-one.
(78). (4Z)-4-(1,3-Benzothiazol-6-ylmethylene)-2-[[2-(tnfluoromethyl)phenyl]methylamino]-1*H*-imidazol-5-one.
(79). (4Z)-4-(1,3-Benzothiazol-6-ylmethylene)-2-[[2-(trifluoromethoxy)phenyl]methylamino]-1*H*-imidazol-5-one.
(80). (±)-(4Z)-4-(1,3-Benzothiazol-6-ylmethylene)-2-[[*cis*-2-hydroxyindan-1-yl]amino]-1*H*-imidazol-5-one.
(81). (±)-(4Z)-4-(1,3-Benzothiazol-6-ylmethylene)-2-[[*trans*-2-hydroxyindan-1-yl]amino]-1*H*-imidazol-5-one.
(82). (4Z)-4-(1,3-Benzothiazol-6-ylmethylene)-2-[[(1R,2R)-2-hydroxyindan-1-yl]amino]-1*H*-imidazol-5-one.
(83). (4Z)-4-(1,3-Benzothiazol-6-ylmethylene)-2-[[(1*S*,2*S*)-2-hydroxyindan-1-yl]amino]-1*H*-imidazol-5-one.
(84). (±)-(4Z)-4-(1,3-Benzothiazol-6-ylmethylene)-2-[[*cis*-2-methoxyindan-1-yl]amino]-1*H*-imidazol-5-one.
(85). (±)-(4Z)-4-(1,3-Benzothiazol-6-ylmethylene)-2-[[*trans*-2-methoxyindan-1-yl]amino]-1*H*-imidazol-5-one.
(86). Methyl (2*S*)-2-[[(4Z)-4-(1,3-benzothiazol-6-ylmethylene)-5-oxo-1*H-*imidazol-2-yl]amino]-3-phenyl-propanoate.
(87). Methyl (2*R*)-2-[[(4Z)-4-(1,3-benzothiazol-6-ylmethylene)-5-oxo-1*H-*imidazol-2-yl]amino]-3-phenyl-propanoate.
(88). (±)-(4Z)-4-(1,3-Benzothiazol-6-ylmethylene)-2-[(2-fluoro-1-phenylethyl)amino]-1*H*-imidazol-5-one.
(89). (±)-(4Z)-2-[(2-Amino-1-phenyl-ethyl)amino]-4-(1,3-benzothiazol-6-ylmethylene)-1*H*-imidazol-5-one dihydrochloride.
(90). (±)-(4Z)-4-(1,3-Benzothiazol-6-ylmethylene)-2-[[2-(methylamino)-1-phenyl-ethyl]amino]-1*H*-imidazol-5-one dihydrochloride.
(91). (±)-(4Z)-4-(1,3-Benzothiazol-6-ylmethylene)-2-[[2-(dimethylamino)-1-phenyl-ethyl]amino]-1*H*-imidazol-5-one.
(92). (±)-(4Z)-4-(1,3-Benzothiazol-6-ylmethylene)-2-[(1-benzyl-2-hydroxyethyl)amino]-1*H*-imidazol-5-one.
(93). (4Z)-4-(1,3-Benzothiazol-6-ylmethylene)-2-[[(1*R*)-1-benzyl-2-hydroxyethyl]amino]-1*H*-imidazol-5-one.
(94). (±)-(4Z)-4-(1,3-Benzothiazol-6-ylmethylene)-2-[(1-benzyl-2-methoxyethyl)amino]-1*H*-imidazol-5-one.
(95). (±)-(4Z)-4-(1,3-Benzothiazol-6-ylmethylene)-2-[(2-hydroxy-1-phenylethyl)amino]-1*H*-imidazol-5-one.
(96). (4Z)-4-(1,3-Benzothiazol-6-ylmethylene)-2-[[(1*R*)-2-hydroxy-1-phenylethyl]amino]-1*H*-imidazol-5-one.
(97). (4Z)-4-(1,3-Benzothiazol-6-ylmethylene)-2-[[(1*S*)-2-hydroxy-1-phenylethyl]amino]-1*H*-imidazol-5-one.
(98). (±)-(4Z)-4-(1,3-Benzothiazol-6-ylmethylene)-2-[(2-methoxy-1-phenylethyl)amino]-1*H*-imidazol-5-one.
(99). (±)-(4Z)-4-(1,3-Benzothiazol-6-ylmethylene)-2-[(2-hydroxy-2-phenylethyl)amino]-1*H*-imidazol-5-one.
(100). (±)-(4Z)-4-(1,3-Benzothiazol-6-ylmethylene)-2-[(2-methoxy-2-phenylethyl)amino]-1*H*-imidazol-5-one.
(101). (±)-(4Z)-4-(1,3-Benzothiazol-6-ylmethylene)-2-[(2-hydroxy-3-phenylpropyl)amino]-1*H*-imidazol-5-one.
(102). (±)-(4Z)-4-(1,3-Benzothiazol-6-ylmethylene)-2-[(2-methoxy-3-phenylpropyl)amino]-1*H*-imidazol-5-one.
(103). (4Z)-4-(1,3-Benzothiazol-6-ylmethylene)-2-[(5-methylpyrazin-2-yl)methylamino]-1*H*-imidazol-5-one.
(104). (4Z)-4-(1,3-Benzothiazol-6-ylmethylene)-2-(2-pyridylmethylamino)-1*H*-imidazol-5-one.
(105). (4Z)-4-(1,3-Benzothiazol-6-ylmethylene)-2-(3-pyridylmethylamino)-1*H*-imidazol-5-one.
(106). (4Z)-4-(1,3-Benzothiazol-6-ylmethylene)-2-(4-pyridylmethylamino)-1*H*-imidazol-5-one.
(107). (4Z)-4-(1,3-Benzothiazol-6-ylmethylene)-2-[(5-methyl-2-furyl)methylamino]-1*H*-imidazol-5-one.
(108). (4Z)-4-(1,3-Benzothiazol-6-ylmethylene)-2-[(4-methylthiazol-2-yl)methylamino]-1*H*-imidazol-5-one.
(109). (4Z)-4-(1,3-Benzothiazol-6-ylmethylene)-2-(3-imidazol-1-ylpropylamino)-1*H*-imidazol-5-one.
(110). (4Z)-4-(1,3-Benzothiazol-6-ylmethylene)-2-[2-(2-pyridyl)ethylamino]-1*H*-imidazol-5-one.
(111). (4Z)-2-(1,3-Benzothiazol-2-ylmethylamino)-4-(1,3-benzothiazol-6-ylmethylene)-1*H*-imidazol-5-one.
(112). (4Z)-4-(1,3-Benzothiazol-6-ylmethylene)-2-[(1-methyl-4-piperidyl)methylamino]-1*H*-imidazol-5-one.
(113). (4Z)-4-(1,3-Benzothiazol-6-ylmethylene)-2-(tetrahydropyran-4-ylmethylamino)-1*H-*imidazol-5-one.
(114). *Tert*-butyl 4-[[[(4Z)-4-(1,3-benzothiazol-6-ylmethylene)-5-oxo-1*H-*imidazol-2-yl]amino]methyl]piperidine-1-carboxylate.
(115). (4Z)-4-(1,3-Benzothiazol-6-ylmethylene)-2-[(7-methyl-7-azaspiro[3.5]nonan-2-yl)amino]-1*H*-imidazol-5-one.
(116). (4Z)-4-(1,3-Benzothiazol-6-ylmethylene)-2-(3-fluoro-4-methyl-anilino)-1*H-*imidazol-5-one.
(117). (4Z)-4-(1,3-Benzothiazol-6-ylmethylene)-2-(4-fluoroanilino)-1*H-*imidazol-5-one.
(118). (4Z)-4-(1,3-Benzothiazol-6-ylmethylene)-2-(4-hexylanilino)-1*H-*imidazol-5-one.
(119). (4Z)-4-(1,3-Benzothiazol-6-ylmethylene)-2-[4-(4-methylpiperazin-1-yl)anilino]-1*H*-imidazol-5-one.
(120). (4Z)-4-(1,3-Benzothiazol-6-ylmethylene)-2-[3-(difluoromethoxy)anilino]-1*H*-imidazol-5-one.
(121). (4Z)-2-[(1-Acetylindolin-6-yl)amino]-4-(1,3-benzothiazol-6-ylmethylene)-1*H-*imidazol-5-one.
(122). (4Z)-4-(1,3-Benzothiazol-6-ylmethylene)-2-[3-(trifluoromethyl)anilino]-1*H*-imidazol-5-one.
(123). (4Z)-4-(1,3-Benzothiazol-6-ylmethylene)-2-(indan-5-ylamino)-1*H-*imidazol-5-one.
(124). (4Z)-4-(1,3-Benzothiazol-6-ylmethylene)-2-(4-morpholinoanilino)-1*H-*imidazol-5-one.
(125). (4Z)-4-(1,3-Benzothiazol-6-ylmethylene)-2-[(1-methylindazol-7-yl)amino]-1*H*-imidazol-5-one.
(126). (4Z)-4-(1,3-Benzothiazol-6-ylmethylene)-2-(pyrimidin-2-ylamino)-1*H-*imidazol-5-one.
(127). (4Z)-4-(1,3-Benzothiazol-6-ylmethylene)-2-(2-pyridylamino)-1*H-*imidazol-5-one.
(128). (4Z)-4-(1,3-Benzothiazol-6-ylmethylene)-2-[(1-methylpyrazol-3-yl)amino]-1*H*-imidazol-5-one.
(129). (4Z)-4-(1,3-Benzothiazol-6-ylmethylene)-2-[(2-methoxy-6-methyl-3-pyridyl)amino]-1*H-*imidazol-5-one.
(130). (4Z)-4-(1,3-Benzothiazol-6-ylmethylene)-2-(pyrimidin-5-ylamino)-1*H-*imidazol-5-one.
(131). (4Z)-4-(1,3-Benzothiazol-6-ylmethylene)-2-(3-pyridylamino)-1*H-*imidazol-5-one.
(132). (4Z)-4-(1,3-Benzothiazol-6-ylmethylene)-2-(1,3,4-thiadiazol-2-ylamino)-1*H*-imidazol-5-one.
(133). (4Z)-4-(1,3-Benzothiazol-6-ylmethylene)-2-[[5-(4-methylpiperazin-1-yl)-2-pyridyllaminol-1*H*-imidazol-5-one.
(134). (4Z)-4-(1,3-Benzothiazol-6-ylmethylene)-2-[[6-(4-methylpiperazin-1-yl)-3-pyndyl]amino]-1*H*-imidazol-5-one.
(135). (4Z)-4-(1,3-Benzothiazol-6-ylmethylene)-2-[[2-(4-methy1piperazin-1-yl)pyrimidin-5-yl]amino]-1*H*-imidazol-5-one.
(136). (4Z)-4-(1,3 -Benzothiazol-6-ylmethylene)-2-[[5-(4-methylpiperazin-1-yl)pyrimidin-2-yl]amino]-1*H*-imidazol-5-one.
(137). (4Z)-4-(1,3-Benzothiazol-6-ylmethylene)-2-[[5-(4-methylpiperazin-1-yl)pyrazin-2-yl]amino]-1*H*-imidazol-5-one.
(138). (4Z)-4-(1,3-Benzothiazol-6-ylmethylene)-2-[[6-(4-methylpiperazin-1-yl)pyridazin-3-yl]amino]-1*H*-imidazol-5-one.
(139). (4Z)-4-(1,3-Benzothiazol-6-ylmethylene)-2-(tetrahydropyran-4-ylamino)-1*H*-imidazol-5-one.
(140). *Tert*-butyl 4-[[(4Z)-4-(1,3-benzothiazol-6-ylmethylene)-5-oxo-1*H-*imidazol-2-yl]amino]piperidine-1-carboxylate.
(141). Ethyl 4-[[(4Z)-4-(1,3-benzothiazol-6-ylmethylene)-5-oxo-1*H*-imidazol-2-yl]amino]piperidine-1-carboxylate.
(142). (4Z)-4-(1,3-benzothiazol-6-ylmethylene)-2-[(1-methyl-4-piperidyl)amino]-1*H*-imidazol-5-one.
(143). (±)-(4Z)-4-(1,3-Benzothiazol-6-ylmethylene)-2-[(1-methyl-3-piperidyl)amino]-1*H*-imidazol-5-one.
(144). (4Z)-4-(1,3-Benzothiazol-6-ylmethylene)-2-(oxetan-3-ylamino)-1*H-*imidazol-5-one.
(145). (4Z)-4-(1,3-benzothiazol-6-ylmethylene)-2-[[(3*R*)-tetrahydrofuran-3-yl]amino]-1*H*-imidazol-5-one.
(146). (4Z)-4-(1,3-Benzothiazol-6-ylmethylene)-2-[[(3*S*)-tetrahydrofuran-3-yl]amino]-1*H*-imidazol-5-one.
(147). (4Z)-4-(1,3-Benzothiazol-6-ylmethylene)-2-[[(3*R*)-tetrahydropyran-3-yl]amino]-1*H*-imidazol-5-one.
(148). (4Z)-4-(1,3-Benzothiazol-6-ylmethylene)-2-[[(3*S*)-tetrahydropyran-3-yl]amino]-1*H*-imidazol-5-one.
(149). (±)-(4Z)-4-(1,3-Benzothiazol-6-ylmethylene)-2-[(6,6-dimethyltetrahydropyran-3-yl)amino]-1*H*-imidazol-5-one.
(150). (4Z)-4-(1,3-Benzothiazol-6-ylmethylene)-2-[[(3*R*,4*R*)-4-hydroxytetrahydropyran-3-yl]amino]-1*H*-imidazol-5-one.
(151). (±)-(4Z)-4-(1,3-Benzothiazol-6-ylmethylene)-2-(oxepan-3-ylamino)-1*H*-imidazol-5-one.
(152). (±)-3-[[(4Z)-4-(1,3-Benzothiazol-6-ylmethylene)-5-oxo-1*H*-imidazol-2-yl]amino]piperidin-2-one.
(153). (3*S*)-3-[[(4Z)-4-(1,3-Benzothiazol-6-ylmethylene)-5-oxo-1*H*-imidazol-2-yl]amino]piperidin-2-one.
(154). (5*S*)-5-[[(4*Z*)-4-(1,3-Benzothiazol-6-ylmethylene)-5-oxo-1*H*-imidazol-2-yl]amino]piperidin-2-one.
or anyone of their pharmaceutically acceptable salts.

11. A pharmaceutical composition comprising at least one compound as defined in anyone of claims 1 to 9 or or any of its pharmaceutically acceptable salt or as defined in claim 10.

12. Synthesis process for manufacturing a compounds of formula (I) as defined in anyone of claims 1 to 9 or or any of its pharmaceutically acceptable salt or as defined in claim 10 or any of its pharmaceutically acceptable salts, comprising at least a step of coupling a compound of formula (II) below 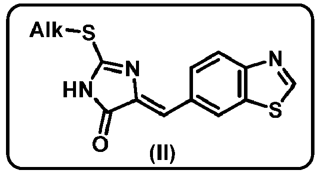 wherein Alk is a (C₁-C₅)alkyl,
with an amine of formula R¹ NH₂ wherein R¹ is as defined in anyone of claims 1 to 9.

13. A synthetic intermediate of formula (II) below 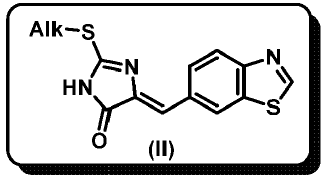 wherein Alk is a (C₁-C₅)alkyl, in particular Alk is selected from the group consisting of an ethyl and a methyl.

14. A compound of formula (I) as defined in anyone of claim 1 to 9 or any of its pharmaceutically acceptable salts, or at least any of compounds (1) to (154) as defined in claim 10 or any of its pharmaceutically acceptable salts, for use as a medicament.

15. A compound of formula (I) according to anyone of claims 1 to 9 or any of its pharmaceutically acceptable salts, or at least any of compounds (1) to (154) as defined in claim 10 or any of its pharmaceutically acceptable salts for use in the treatment and/or in the prevention of cognitive deficits associated with Down syndrome (Trisomy 21); Alzheimer's disease and related diseases; dementia; tauopathies; and other neurodegenerative diseases (Parkinson's disease; Pick disease, including Niemann-Pick Type C Disease); CDKL5 Deficiency Disorder; McDermid syndrome; autism; diabetes; regulation of folate and methionine metabolism; osteoarthritis, in particular knee osteoarthritis; Duchenne muscular dystrophy; several cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointerstinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such as Leishmaniasis and *Trypanosoma brucei* and for regulating body temperature.

16. A compound of formula (I) for use according to anyone of claims 1 to 9 or any of its pharmaceutically acceptable salts, or at least any of compounds (1) to (154) as defined in claim 10 or any of its pharmaceutically acceptable salts for use in the treatment and/or prevention of Down syndrome, Alzheimer's disease, dementia, tauopathies, Parkinson's disease, Niemann-Pick Type C Disease, CDKL5 Deficiency Disorder and Phelan-McDermid syndrome and their associated cognitive and motor conditions.
